# FASCICULE DE BREVET EUROPEEN

(11) **EP 1 483 289 B1**
(45) Date de publication et mention de la délivrance du brevet: **09.07.2014**
(21) Numéro de dépôt: 03727609.4
(22) Date de dépôt: 21.03.2003
(51) Int. Cl.: C07K 14/47, C12N 15/12, A61K 38/17, C12N 5/10, C12Q 1/68, C07K 16/18, C12N 15/11, A61K 39/395, A61P 9/00

(54) **GENES REGULATEURS DE L'ANGIOGENESE, PREPARATIONS PHARMACEUTIQUES LES CONTENANT ET LEURS APPLICATIONS**
GENE DIE ANGIOGENESE REGULIEREN, PHARMAZEUTISCHE ZUSAMMENSETZUNGEN DIE SIE ENTHALTEN UND DEREN VERWENDUNGEN
ANGIOGENESIS REGULATOR GENES, PHARMACEUTICAL PREPARATIONS CONTAINING SAME AND USES THEREOF

(30) Priorité: 22.03.2002 FR 0203655
(43) Date de publication de la demande: 08.12.2004
(73) Titulaire: GENE SIGNAL INTERNATIONAL SA, 1015 Lausanne VD (CH)
(72) Inventeur: AL-MAHMOOD, Salman, 75014 Paris (FR); COLIN, Sylvie, 75014 Paris (FR); SCHNEIDER, Christophe, 51100 Reims (FR)
(74) Mandataire: Icosa
(86) Numéro de dépôt international: PCT/FR2003/000912
(87) Numéro de publication internationale: WO 2003/080105

(56) Documents cités:
- WO-A-01/21831
- WO-A-02/34783
- WO-A-98/21333
- WO-A-99/00498
- WO-A-03/074073
- US-A- 5 747 660
- DATABASE GSN [en ligne] Geneseq; 16 septembre 2002 (2002-09-16) Database accession no. ABV26916 XP002219162 -& WO 01 60860 A (MILLENIUM PREDICTIVE MEDICINE) 23 août 2001 (2001-08-23)
- CHARRIN STEPHANIE ET AL: "The major CD9 and CD81 molecular partner: Identification and characterization of the complexes." JOURNAL OF BIOLOGICAL CHEMISTRY, vol. 276, no. 17, 27 avril 2001 (2001-04-27), pages 14329-14337, XP002219161 ISSN: 0021-9258
- DATABASE EMBL [en ligne] EBI; 23 août 1999 (1999-08-23) Database accession no. AI953818 XP002219163
- DATABASE EMBL [en ligne] EBI; 6 février 2002 (2002-02-06) "Homo sapiens, similar to prostaglandin F2 receptor negative regulator, clone IMAGE:4448037" Database accession no. BC022235 XP002219164
- DATABASE EMBL [en ligne] EBI; 14 mars 2000 (2000-03-14) "Homo sapiens mRNA for KIAA1436 protein" Database accession no. AB037857 XP002219166 cité dans la demande
- DATABASE EMBL [en ligne] EBI; 18 mars 1996 (1996-03-18) "Cloning vector pCI-neo" Database accession no. U47120 XP002219165
- DATABASE GSN GENESEQ; 16 septembre 2002 (2002-09-16) Database accession no. ABV25139 XP002269996 & WO 01 60860 A (MILLENNIUM PREDICTIVE MEDICINE) 23 août 2001 (2001-08-23)
- DATABASE EMBL EBI; 5 septembre 2000 (2000-09-05) "Homo sapiens homeobox NKX3.1 mRNA, complete cds." Database accession no. AF247704 XP002269997
- DATABASE GSN GENESEQ; 16 septembre 2002 (2002-09-16) Database accession no. ABV23250 XP002269998 & WO 01 60860 A (MILLENNIUM PREDICTIVE MEDICINE) 23 août 2001 (2001-08-23)
- DATABASE EMBL EBI; 27 juin 1997 (1997-06-27) Database accession no. AA486235 XP002269999
- DATABASE EMBL EBI; 10 juillet 1998 (1998-07-10) "Homo sapiens zinc-finger helicase (hZFH) mRNA, complete cds." Database accession no. U91543 XP002270000 & AUBRY FLORENCE ET AL: "Identification of a human 17p-located cDNA encoding a protein of the Snf2-like helicase family" EUROPEAN JOURNAL OF BIOCHEMISTRY, vol. 254, no. 3, juin 1998 (1998-06), pages 558-564, ISSN: 0014-2956
- DATABASE GSN [en ligne] GENESEQ; 25 février 2003 (2003-02-25) Database accession no. ABX63370 XP002270001 -& US 2002/137081 A1 (BANDMAN) 26 septembre 2002 (2002-09-26)
- DATABASE EMBL EBI; 25 octobre 2001 (2001-10-25) Database accession no. BC001571 XP002270002
- DATABASE EMBL EBI; 24 janvier 2001 (2001-01-24) Database accession no. BF980038 XP002270003

### LISTAGE DE SEQUENCES

<110> GENE SIGNAL Salman Al-Mahmood Colin, Sylvie Schneider, Christophe Al-Mahmood, Salman
<120> GENES REGULATEURS DE L'ANGIOGENESE, PREPARATIONS PHARMACEUTIQUES LES CONTENANT ET LEURS APPLICATIONS.
<130> 25599 GENE SIGNAL
<140> PCT/FR03/XXXXX
   <141> 2003-03-21
<150> FR02/03655
   <151> 2002-03-22
<160> 34
<170> PatentIn version 3.1
<210> 1
   <211> 6160
   <212> DNA
   <213> Homo sapiens
<220>
   <221> misc_feature
   <223> séquence appelée GS-N1
<300>
   <308> GenBank/AB037857
   <309> 2000-03-14
   <313> (1)..(6160)
<400> 1
<210> 2
   <211> 3266
   <212> DNA
   <213> Homo sapiens
<220>
   <221> misc_feature
   <223> séquence appelée GS-N2
<300>
   <308> GenBank/AF247704
   <309> 2000-09-02
   <313> (1)..(3266)
<400> 2
   gcggtgcggg ccgggcgggt gcattcaggc caaggcgggg ccgccgggat gctcagggtt     60
   ccggagccgc ggcccgggga ggcgaaagcg gagggggccg cgccgccgac cccgtccaag    120
   ccgctcacgt ccttcctcat ccaggacatc ctgcgggacg gcgcgcagcg gcaaggcggc    180
   cgcacgagca gccagagaca gcgcgacccg gagccggagc cagagccaga gccagaggga    240
   ggacgcagcc gcgccggggc gcagaacgac cagctgagca ccgggccccg cgccgcgccg    300
   gaggaggccg agacgctggc agagaccgag ccagaaaggc acttggggtc ttatctgttg     360
   gactctgaaa acacttcagg cgcccttcca aggcttcccc aaacccctaa gcagccgcag     420
   aagcgctccc gagctgcctt ctcccacact caggtgatcg agttggagag gaagttcagc    480
   catcagaagt acctgtcagc ccctgaacgg gcccacctgg ccaagaacct caagctcacg    540
   gagacccaag tgaagatatg gttccagaac agacgctata agactaagcg aaagcagctc    600
   tcctcggagc tgggagactt ggagaagcac tcctctttgc cggccctgaa agaggaggcc    660
   ttctcccggg cctccctggt ctccgtgtat aacagctatc cttactaccc atacctgtac    720
   tgcgtgggca gctggagccc agctttttgg taatgccagc tcaggtgaca accattatga    780
   tcaaaaactg ccttccccag ggtgtctcat atgaaaagca caaggggcca aggtcaggga    840
   gcaagaggtg tgcacaccaa aactattgga gaattgcgtg gaaatcttca gattcttcac    900
   tggtgagaca atgaaacaac agagacagtg aaagttttaa tacctaagtc attcccccag    960
   tgcatactgt agcgtcaagt ttttgcttct ggctacctgt ttgaagggga gagagggaaa   1020
   atcaagtggt attttccagc actttgtatg attttggatg agctgtacac ccaaggattc   1080
   tgttctgcaa ctccatcctc ctgtgtcact gaatatcaac tctgaaagag caaacctaac   1140
   aggagaaagg acaaccagga tgaggatgtc accaactgaa ttaaacttaa gtccagaagc   1200
   ctcctgttgg ccttggaata tggccaaggc tctctctgtc cctgtaaaag agaggggcaa   1260
   atagtctcca aagagaacgc cctcatgctc agcacatatt tgcatggaag ggggagatgg   1320
   gtgggaggag atgaaaatat cagcttttct tattcctttt tattcctttt aaaatggtat   1380
   gccaacttaa gtatttacag ggtggcccaa atagaacaag atgcactcgc tgtgatttta   1440
   agacaagctg tataaacaga actccactgc aagagggagg gccgggccag gagaatctcc   1500
   gcttgtccaa gacaggggcc taaggagggt ctccacactg ctgctagggg ctgttgcatt   1560
   tttttattag tagaaagtgg aaaggcctct tctcaacttt tttcccttgg gctggagaat   1620
   ttagaatcag aagtttcctg gagttttcag gctatcatat atactgtatc ctgaaaggca   1680
   acataattct tccttccctc cttttaaaat tttgtgttcc tttttgcagc aattactcac   1740
   taaagggctt cattttagtc cagattttta gtctggctgc acctaactta tgcctcgctt   1800
   atttagcccg agatccggtc tttttttttt tttttttttc cgtctcccca aagctttatc   1860
   tgtcttgact ttttaaaaaa gtttgggggc agattctgaa ttggctaaaa gacatgcatt   1920
   tttaaaacta gcaactctta tttctttcct ttaaaaatac atagcattaa atcccaaatc   1980
   ctatttaaag acctgacagc ttgagaaggg tcactactgc atttatagga ccttctggtg   2040
   gttctgctgt tacgtttgaa gtctgacaat ccttgagaat ctttgcatgc agaggaggta   2100
   agaggtattg gattttcaca gagggaagaa cacagcgcag aatgaaggcg caggcttact   2160
   gagctgtcca gtggagggct catgggtggg acatggaaaa gaaggcagcc taggccctgg   2220
   ggagcccagt ccactgagca agcaagggac tgagtgagcc ttttgcagga aaaggctaag   2280
   aaaaaggaaa accattctaa aacacaacaa gaaactgtcc aaatgctttg ggaactgtgt   2340
   ttattgccta taatggggtc cccaaaatgg gtaacctaga cttcagagag aatgagcaga   2400
   gagcaaagga gaaatctggg ctgtccttcc attttcattc tgttatctca ggtgagctgg   2460
   tagaggggag acattagaaa aaaatgaaac aacaaaacaa ttactaatga ggtacgctga   2520
   ggcctgggag tctcttgagc tccacgactt caaaattaaa atgagccatg agtcaaacca   2580
   ctgcaatcca gcctgggcaa cgagcaagac ccagtctcta ctgttggtgg caaaattgcc   2640
   aacataagtt aatagaaagt tggccaattt caccccattt tctgtggttt gggctccaca   2700
   ttgcaatgtt caatgccacg tgctgctgac accgaccgga gtactagcca gcacaaaagg   2760
   cagggtagcc tgaattgctt tctgctcttt acatttcttt taaaataagc atttagtgct   2820
   cagtccctac tgagtactct ttctctcccc tcctctgaat ttaattcttt caacttgcaa   2880
   tttgcaaggg ttacacattt cactgtgatg tatattgtgt tgcaaaaaaa aaaaagtgtc   2940
   tttgtttaaa attacttggt ttgtgaatcc atcttgcttt ccccattgga actagtcatt   3000
   aacccatctc tgaactggta gaaaaacatc tgaagagcta gtctatcagc atctgacagg   3060
   tgaattggat ggttctcaga accatttcac ccagacagcc tgtttctatc ctgtttaata   3120
   aattagtttg ggttctctac atgcataaca aaccctgctc caatctgtca cataaaagtc   3180
   tgtgacttga agtttagtca gcacccccac caaactttat ttttctatgt gttttttgca   3240
   acatatgagt gttttgaaaa taaagt                                          3266
<210> 3
   <211> 6771
   <212> DNA
   <213> Homo sapiens
<220>
   <221> misc_feature
   <223> séquence appelée GS-N3
<300>
   <308> GenBank/U91543
   <309> 1998-08-04
   <313> (1)..(6771)
<400> 3
   atatggaggt gaagggtgag atcgggaaac aaagggtatg gccccctagt tcccaaaggg     60
   agcagggaga tgggaataga attgaaggta ggttttaggc tacttgggag gaggaatatt    120
   taggtaattg tggagacttt ctcctgtgtg atgaaggcgg cagacactgt gatcctgtgg    180
   gcaagaagta aaaatgacca gctgaggatt tcttttcctc caggactgtg ttggggtgac    240
   aggatgcctg ataaggatga cattcggctg ctgccgtcag cattgggtgt gaagaagaga    300
   aaacgaggac ccaagaagca gaaggagaac aagccaggaa aaccccgaaa acgcaagaag    360
   cgtgacagtg aggaggaatt tggttctgag cgagatgagt accgggagaa gtcagagagt    420
   gggggcagtg aatatggaac cggaccgggt cggaaacgaa gaaggaagca ccgagaaaaa    480
   aaggagaaga agacaaagcg gcggaaaaag gggagggagg atggggggca aaagcaagtg     540
   gaacagaagt catcagcaac tctgcttctg acctggggcc tggaggatgt ggagcatgtg    600
   ttctctgagg aggattacca cacgctcacc aactacaaag ccttcagcca gttcatgagg    660
   cccctaattg ctaagaagaa tcctaagatc ccaatgtcta agatgatgac catccttggg    720
   gccaaatgga gagagttcag tgccaacaac cccttcaagg ggtcagcagc tgctgtggcg    780
   gcggcagcgg cagcagcagc agcagctgta gctgagcagg tgtcagctgc tgtctcgtcg    840
   gccaccccca tagcaccctc cggacccccc gcccttccac caccccctgc tgctgatatc     900
   cagcccccac ccatccgaag agccaaaacc aaagagggca aaggtccagg ccataagagg    960
   cggagtaaga gcccccgagt gcctgatgga cgcaagaagc ttcggggaaa gaaaatggca   1020
   ccactcaaaa taaaactagg gcttctgggt ggcaagagga agaaaggagg ctcgtatgtt   1080
   tttcagagcg acgaaggtcc tgaaccagag gctgaggaat cagacctgga cagtggcagt   1140
   gtccacagtg cctcaggccg gcctgatggc cctgtccgca ccaagaaact aaagagaggc   1200
   cggccaggaa ggaagaagaa gaaggtcctg ggctgtcctg cagtggccgg ggaggaggag   1260
   gttgatggct acgagacgga tcaccaggat tactgtgagg tgtgccagca gggtggggaa   1320
   attattctgt gtgacacctg ccctcgtgcc taccacctcg tctgccttga tcctgagctt   1380
   gaccgggctc cagagggcaa atggagctgc cctcactgtg agaaggaggg ggtccagtgg   1440
   gaggccaagg aggaagaaga agaatacgaa gaggagggag aggaagaagg ggagaaggag   1500
   gaggaggatg atcacatgga gtactgccgc gtatgcaagg acggcgggga gctcctgtgc   1560
   tgtgacgcgt gcatctcctc ctaccacatt cattgtctaa accctcccct gcctgacatt   1620
   cccaatggtg aatggctgtg tccccgatgc acatgccccg tgctgaaggg tcgagtgcag   1680
   aagatcctac attggcggtg gggggagcca cctgtagcag tgccagcccc tcaacaggca   1740
   gatggaaatc cagatgtccc acccccccgt cctcttcaag gcagatcaga gcgagagttc   1800
   tttgtcaagt gggtaggact atcctactgg cactgctcct gggccaagga gcttcagctg   1860
   gaaatcttcc atttggttat gtatcgaaac taccagcgga agaatgacat ggatgagccc   1920
   ccacccctgg actatggctc cggcgaggat gatgggaaga gcgacaagcg taaagtgaaa   1980
   gacccgcact atgctgagat ggaggagaag tactatcgtt ttggcatcaa gccagagtgg   2040
   atgaccgtcc accgcatcat caaccacagt gtggataaaa aggggaatta ccactatcta   2100
   gtaaaatgga gggacttacc atatgaccag tccacgtggg aggaagatga aatgaatatc   2160
   cctgaatacg aagaacataa gcaaagctac tggagacacc gagaactaat tatgggggaa   2220
   gaccctgccc agccccgcaa gtataagaag aagaagaagg agctacaggg tgatgggcct   2280
   cccagttctc ccactaatga tcctaccgtg aaatatgaga ctcagccacg gtttatcaca   2340
   gccactggag gcaccctgca catgtatcag ttggaagggc tgaactggct acgcttctcc   2400
   tgggcccagg gcactgacac cattctagct gatgagatgg ggctaggcaa gaccatacaa   2460
   accatcgtct tcctctactc actctacaag gagggccaca caaaaggtcc cttcctggtg   2520
   agtgccccac tctctaccat cattaactgg gagcgggagt tccagatgtg ggcacccaaa   2580
   ttctatgtgg tgacatacac gggtgacaag gacagccggg ccatcattcg tgagaatgaa   2640
   ttctcctttg aggacaatgc catcaaaggg ggcaagaaag cttttaagat gaagagggag   2700
   gcacaggtga agttccatgt tctcctgaca tcgtatgagc tgatcaccat tgatcaggca   2760
   gcacttggtt ccatccgctg ggcctgtctt gtggtagatg aggcccatcg actcaagaac   2820
   aaccagtcca agtttttcag ggttctcaat ggttacaaga tagatcataa gttgctgctg   2880
   acaggaaccc cattgcagaa taatctggag gagctcttcc atctcctgaa cttcctcacc   2940
   ccagagagat ttaacaactt ggagggcttc ctggaggagt ttgctgacat atccaaagag   3000
   gaccagatca agaaactgca tgatttgctg gggccacaca tgctgcggag actcaaggca    3060
   gatgtcttta agaacatgcc agccaagaca gagctcatcg ttcgggtgga gctaagcccc   3120
   atgcagaaga aatactacaa atacatcctg actcgaaatt ttgaggcctt gaattcacga   3180
   ggtggtggga accaggtgtc gctgcttaat atcatgatgg atcttaagaa gtgctgcaac   3240
   catccatacc tttttcccgt ggctgctatg gagtccccca aactccccag tggggcttat   3300
   gagggtgggg cacttattaa gtcgtctggg aagctcatgc tgctccagaa gatgctgcga   3360
   aagctgaagg agcaaggaca ccgagtgctc atcttctcgc agatgaccaa aatgttagac   3420
   ttgcttgagg acttcttaga ctatgaaggc tacaagtatg agcgcatcga tggtggtatc   3480
   acgggtgccc tgaggcagga ggccatcgat cggtttaatg ctcctggggc ccaacaattc   3540
   tgcttcctcc tgtccacccg agctgggggc ctgggcatca atctggccac tgctgacact   3600
   gtcatcatct ttgattctga ctggaacccc cataatgaca tccaggcctt tagccgggct   3660
   catcggattg gccaggccaa caaagtgatg atttaccggt ttgtgactcg cgcgtcagtg   3720
   gaagagcgaa tcacacaagt ggccaagaga aagatgatgc tgacacacct ggttgtgcgg   3780
   cctgggctgg gctccaaggc aggctccatg tccaagcagg agcttgacga cattctcaaa   3840
   tttggcactg aagagctatt caaggatgaa aacgaggggg agaacaagga ggaggacagc   3900
   agtgtgattc attatgacaa tgaggccatc gctcggctgt tggaccggaa ccaggatgca   3960
   actgaggaca ctgacgtgca gaacatgaat gagtatctca gctccttcaa ggtggcacag   4020
   tacgtcgtgc gggaagaaga caagattgag gaaattgagc gagagatcat caagcaggag   4080
   gagaatgtgg accctgacta ctgggagaag ctgctgaggc atcactatga gcaacagcag   4140
   gaagacctag cccggaatct aggcaagggc aagcgggttc gcaagcaagt taactacaat   4200
   gatgctgctc aggaagacca agacaaccag tcagagtact cggtgggttc agaggaggag   4260
   gatgaagact tcgatgaacg tcctgaaggg cgtagacagt caaagaggca gctccggaat   4320
   gagaaagata agccactgcc tccactgctg gcccgagtcg ggggcaacat tgaggtgctg    4380
   ggcttcaaca cccgtcagcg gaaggctttc ctcaatgctg tgatgcgctg ggggatgcca    4440
   ccacaggatg ccttcaccac acagtggctg gtgcgggacc tgaggggcaa gactgagaag   4500
   gagtttaagg cctatgtgtc tttgttcatg cgccatctgt gtgagcctgg ggcagacggc   4560
   tctgaaacct ttgccgatgg ggtccctcgg gagggactga gtcgccagca ggtgttgacc   4620
   cgcattggag tcatgtctct cgtcaaaaag aaggtgcagg agtttgagca catcaatggg   4680
   cgttggtcaa tgccggaact gatgcctgac cccagcgccg attctaagcg ctcctccaga   4740
   gcctcctctc ctaccaaaac gtctcccacc actcctgagg cttctgctac caacagtccc   4800
   tgcacctcta aacctgctac tccagctcca agtgagaaag gagaaggcat aaggacacct   4860
   cttgagaagg aggaagctga aaaccaggag gaaaagccag agaagaacag cagaattggg   4920
   gagaagatgg agacagaggc tgatgccccc agcccagccc catcacttgg ggagcggctg   4980
   gagccaagga agattcctct agaggatgag gtgccagggg tgcctggaga gatggagcct   5040
   gaacctgggt accgtgggga cagagagaag tcagccacag agtcgacgcc aggagaaagg   5100
   ggggaggaga agccgttgga tggacaggaa cacagggaga ggccggaggg ggaaacaggg   5160
   gatttgggca agagagaaga tgtaaaaggt gaccgggagc ttcgaccagg gcctcgagat   5220
   gagccacggt ccaatgggcg acgagaggaa aagacagaga acccccggtt catgttcaat   5280
   atcgccgatg gtggcttcac agagcttcac acactgtggc agaatgagga acgggcagct   5340
   atttcctcgg ggaaactcaa tgagatctgg cacagaagac atgactattg gcttctggct   5400
   gggattgtcc tccatggcta tgcacggtgg caggacatcc agaatgatgc tcaatttgcc   5460
   attatcaacg agccatttaa aactgaagcc aataagggga actttctgga gatgaaaaat   5520
   aagttcctgg cccggaggtt caagctcctg gagcaggcgc tggtgattga ggagcagctg   5580
   cggcgggcgg cctacctgaa cctgtcgcag gagccggcgc accccgccat ggccctccac   5640
   gcccgcttcg ccgaggccga gtgcctggcc gagagccacc agcacctctc caaggagtcg   5700
   ctggcgggga acaagccggc caacgccgtc ctgcacaagg ttctgaacca gctggaggag   5760
   ttgctgagcg acatgaaggc ggacgtgacc cgcctgccag ccacgctgtc ccgaataccc   5820
   cccatcgcag cccgccttca gatgtccgag cgcagcatcc tcagccggct ggccagcaag   5880
   ggcacggagc ctcaccccac accggcctac ccgccgggtc cctacgctac acctccgggg   5940
   tacggggcgg ccttcagcgc cgcacccgta ggggccctgg ccgccgcagg cgccaattac   6000
   agccagatgc ctgcagggtc cttcatcaca gccgccacca acggccctcc agtgcttgtg   6060
   aagaaggaga aggaaatggt gggggcattg gtgtcagacg ggctggatcg gaaggagccc   6120
   cgagccgggg aggtgatctg tatagacgac tgactggatc ccaggcctgc ccttcaccca   6180
   ggccccgtcc ccgaggccga cccccagctc aagcgctggg gcctgctgcc agccctccac   6240
   cttccccacc ccttgggcca tcactgggct aggaacccct ttgcccctct ctgcagctcc   6300
   tctcttcaag aagggccctt tgtctttctc cactcccaca cacctttccc accaagcctt   6360
   gaagactgtg ctggtgagaa gaagtctggg tgggagatgg ctggcagggt cttccaagta   6420
   ccttcctccc acactgccaa gtatacacaa cttcccagta aatggttgtg gggaggaaag   6480
   aggtggagcc tccccagccg tttccctgca gaatcagctc tgtctcatgt ggaagtggag   6540
   aatcagcctt gcctggcctt taggaacttt tgtggggaag agagctttga agagaggagg   6600
   gggactttag agagggatga aaatgagccc tgggagggag gaagggacga ggaggggtgg  6660
   ctgcatgtta ccgtccccta cctctcccca cgtggagggt ggagcagtta tgagggagga   6720
   agtcaactgc tgttcagcct cagaataaag gtgccgttca ctggctcagt t            6771
<210> 4
   <211> 3041
   <212> DNA
   <213> Homo sapiens
<220>
   <221> misc_feature
   <223> séquence appelée GS-N4
<300>
   <308> GenBank/BC001571
   <309> 2001-10-29
   <313> (1)..(3041)
<400> 4
   gtcagggccc tgcggtgtga ctcgcgggct cagctggtcc ggccgtagca cctccgcgcc     60
   gtcgccatgt cgcggttttt caccaccggt tcggacagcg agtccgagtc gtccttgtcc    120
   ggggaggagc tcgtcaccaa acctgtcgga ggcaactatg gcaaacagcc attgttgctg    180
   agcgaggatg aagaagatac caagagagtt gtccgcagtg ccaaggacaa gaggtttgag    240
   gagctgacca accttatccg gaccatccgt aatgccatga agattcgtga tgtcaccaag    300
   tgcctggaag agtttgagct cctgggaaaa gcatatggga aggccaaaag cattgtggac    360
   aaagaaggtg tcccccggtt ctatatccgc atcctggctg acctagagga ctatcttaat    420
   gagctttggg aagataagga agggaagaag aagatgaaca agaacaatgc caaggctctg    480
   agcaccttgc gtcagaagat ccgaaaatac aaccgtgatt tcgagtccca tatcacaagc    540
   tacaagcaga accccgagca gtctgcggat gaagatgctg agaaaaatga ggaggattca    600
   gaaggctctt cagatgagga tgaggatgag gacggagtca gtgctgcaac tttcttgaag    660
   aagaaatcag aagctccttc tggggagagt cgcaagttcc tcaaaaagat ggatgatgaa    720
   gatgaggact cagaagattc cgaagatgat gaagactggg acacaggttc cacatcttcc    780
   gattccgact cagaggagga agaagggaaa caaaccgcgc tggcctcaag atttcttaaa    840
   aaggcaccca ccacagatga ggacaagaag gcagccgaga agaaacggga ggacaaagct    900
   aagaagaagc acgacaggaa atccaagcgc ctggatgagg aggaggagga caatgaaggc    960
   ggggagtggg aaagggtccg gggcggagtg ccgttggtta aggagaagcc aaaaatgttt   1020
   gccaagggaa ctgagatcac ccatgctgtt gttatcaaga aactgaatga gatcctacag   1080
   gcacgaggca agaagggaac tgatcgtgct gcccagattg agctgctgca actgctggtt   1140
   cagattgcag cggaaaacaa cctgggagag ggcgtcattg tcaagatcaa gttcaatatc   1200
   atcgcctctc tctatgacta caaccccaac ctggcaacct acatgaagcc agagatgtgg   1260
   gggaagtgcc tggactgcat caatgagctg atggatatcc tgtttgcaaa tcccaacatt   1320
   tttgttggag agaatattct ggaagagagt gagaacctgc acaacgctga ccagccactg   1380
   cgtgtccgtg gctgcatcct aactctggtg gaacgaatgg atgaagaatt taccaaaata   1440
   atgcaaaata ctgaccctca ctcccaagag tacgtggagc acttgaagga tgaggcccag   1500
   gtgtgtgcca tcatcgagcg tgtgcagcgc tacctggagg agaagggcac taccgaggag   1560
   gtctgccgca tctacctgct gcgcatcctg cacacctact acaagtttga ttacaaggcc   1620
   catcagcgac agctgacccc gcctgagggc tcctcaaagt ctgagcaaga ccaggcagaa   1680
   aatgagggcg aggactcggc tgtgttgatg gagagactgt gcaagtacat ctacgccaag   1740
   gaccgcacag accggatccg cacatgtgcc atcctctgcc acatctacca ccatgctctg   1800
   cactcgcgct ggtaccaggc ccgcgacctc atgctcatga gccacttgca ggacaacatt   1860
   cagcatgcag acccgccagt gcagatcctt tacaaccgca ccatggtgca gctgggcatc   1920
   tgtgccttcc gccaaggcct gaccaaggac gcacacaacg ccctgctgga catccagtcg   1980
   agtggccgag ccaaggagct tctgggccag ggcctgctgc tgcgcagcct gcaggagcgc   2040
   aaccaggagc aggagaaggt ggagcggcgc cgtcaggtcc ccttccacct gcacatcaac   2100
   ctggagctgc tggagtgtgt ctacctggtg tctgccatgc tcctggagat cccctacatg   2160
   gccgcccatg agagcgatgc ccgccgacgc atgatcagca agcagttcca ccaccagctg   2220
   cgcgtgggcg agcgacagcc cctgctgggt ccccctgagt ccatgcggga acatgtggtc   2280
   gctgcctcca aggccatgaa gatgggtgac tggaagacct gtcacagttt tatcatcaat   2340
   gagaagatga atgggaaagt gtgggacctt ttccccgagg ctgacaaagt ccgcaccatg   2400
   ctggttagga agatccagga agagtcactg aggacctacc tcttcaccta cagcagtgtc   2460
   tatgactcca tcagcatgga gacgctgtca gacatgtttg agctggatct gcccactgtg   2520
   cactccatca tcagcaaaat gatcattaat gaggagctga tggcctccct ggaccagcca   2580
   acacagacag tggtgatgca ccgcactgag cccactgccc agcagaacct ggctctgcag   2640
   ctggccgaga agctgggcag cctggtggag aacaacgaac gggtgtttga ccacaagcag   2700
   ggcacctacg gggctacttt ccgagaccag aaggacggct accgcaaaaa cgagggctac   2760
   atgcgccgcg gtggctaccg ccagcagcag tctcagacgg cctactgagc tctccactct   2820
   gtttcccgcc tgggccatcc aaccttgaag tcctaaacca cacctcagtc actaaaggtc   2880
   tgtttaaagt tgttctggtt gattgcttgt tgccacaaag gtgtgttgag cccattcatt   2940
   tgctgattat atttctgagc tgctaccaca tgacagacac aaggctcaga tgaagaggtg   3000
   aattaaacac gttcctgacc tcaaaaaaaa aaaaaaaaaa a                        3041
<210> 5
   <211> 1507
   <212> DNA
   <213> Homo sapiens
<220>
   <221> misc_feature
   <223> séquence appelée GS-N5
<300>
   <308> GenBAnk/BC000533
   <309> 2001-07-12
   <313> (1)..(1507)
<400> 5
   ggcacgaggg ggccctgcgg tgtgactcgc gggctcagct ggtccggccg tagcacctcc     60
   gcgccgtcgc catgtcgcgg tttttcacca ccggttcgga cagcgagtcc gagtcgtcct    120
   tgtccgggga ggagctcgtc accaaacctg tcggaggcaa ctatggcaaa cagccattgt    180
   tgctgagcga ggatgaagaa gataccaaga gagttgtccg cagtgccaag gacaagaggt    240
   ttgaggagct gaccaacctt atccggacca tccgtaatgc catgaagatt cgtgatgtca    300
   ccaagtgcct ggaagagttt gagctcctgg gaaaagcaca tgtgccatcc tctgccacat    360
   ctaccaccat gctctgcact cgcgctggta ccaggcccgc gacctcatgc tcatgagcca    420
   cttgcaggac aacattcagc atgcagaccc gccagtgcag atcctttaca accgcaccat    480
   ggtgcagctg ggcatctgtg ccttccgcca aggcctgacc aaggacgcac acaacgccct    540
   gctggacatc cagtcgagtg gccgagccaa ggagcttctg ggccagggcc tgctgctgcg    600
   cagcctgcag gagcgcaacc aggagcagga gaaggtggag cggcgccgtc aggtcccctt    660
   ccacctgcac atcaacctgg agctgctgga gtgtgtctac ctggtgtctg ccatgctcct    720
   ggagatcccc tacatggccg cccatgagag cgatgcccgc cgacgcatga tcagcaagca    780
   gttccaccac cagctgcgcg tgggcgagcg acagcccctg ctgggtcccc ctgagtccat    840
   gcgggaacat gtggtcgctg cctccaaggc catgaagatg ggtgactgga agacctgtca    900
   cagttttatc atcaatgaga agatgaatgg gaaagtgtgg gaccttttcc ccgaggctga     960
   caaagtccgc accatgctgg ttaggaagat ccaggaagag tcactgagga cctacctctt   1020
   cacctacagc agtgtctatg actccatcag catggagacg ctgtcagaca tgtttgagct   1080
   ggatctgccc actgtgcact ccatcatcag caaaatgatc attaatgagg agctgatggc   1140
   ctccctggac cagccaacac agacagtggt gatgcaccgc actgagccca ctgcccagca   1200
   gaacctggct ctgcagctgg ccgagaagct gggcagcctg gtggagaaca acgaacgggt   1260
   gtttgaccac aagcagggca cctacggggg ctacttccga gaccagaagg acggctaccg   1320
   caaaaacgag ggctacatgc gccgcggtgg ctaccgccag cagcagtctc agacggccta   1380
   ctgagctctc cactctgttt cccgcctggg ccatccaacc ttgaagtcct aaaccacacc   1440
   tcagtcacta aaggtctgtt taaagttaaa aaaaaaaaaa aaaaaaaaaa aaaaaaaaaa   1500
   aaaaaaa                                                             1507
<210> 6
   <211> 924
   <212> PRT
   <213> Homo sapiens
<220>
   <221> misc_feature
   <223> CDS partielle de la séquence GS-N1:
   <1..2777
<220>
   <221> misc_feature
   <223> séquence appelée GS-P1: Angioparterine
<400> 6
   Arg Glu Glu Ser Gly Ala Gly Ala Arg Pro Arg Arg Arg Ser Ala Asp
   1               5                   10                  15
   Ser Gly Ala Ala Gly Ala Gly Arg Gly Gly Gly Gly Glu Ala Ala Gly
               20                  25                  30
   Lys Glu Glu Glu Gly Glu Ser Arg Ser Arg Arg Ala Ser Met Gly Arg
           35                  40                  45
   Leu Ala Ser Arg Pro Leu Leu Leu Ala Leu Leu Ser Leu Ala Leu Cys
       50                  55                  60
   Arg Gly Arg Val Val Arg Val Pro Thr Ala Thr Leu Val Arg Val Val
   65                  70                  75                  80
   Gly Thr Glu Leu Val Ile Pro Cys Asn Val Ser Asp Tyr Asp Gly Pro
                   85                  90                  95
   Ser Glu Gln Asn Phe Asp Trp Ser Phe Ser Ser Leu Gly Ser Ser Phe
   100 105 110
   Val Glu Leu Ala Ser Thr Trp Glu Val Gly Phe Pro Ala Gln Leu Tyr
   115 120 125
   Gln Glu Arg Leu Gln Arg Gly Glu Ile Leu Leu Arg Arg Thr Ala Asn
   130 135 140
   Asp Ala Val Glu Leu His Ile Lys Asn Val Gln Pro Ser Asp Gln Gly
   145 150 155 160
   His Tyr Lys Cys Ser Thr Pro Ser Thr Asp Ala Thr Val Gln Gly Asn
   165 170 175
   Tyr Glu Asp Thr Val Gln Val Lys Val Leu Ala Asp Ser Leu His Val
   180 185 190
   Gly Pro Ser Ala Arg Pro Pro Pro Ser Leu Ser Leu Arg Glu Gly Glu
   195 200 205
   Pro Phe Glu Leu Arg Cys Thr Ala Ala Ser Ala Ser Pro Leu His Thr
   210 215 220
   His Leu Ala Leu Leu Trp Glu Val His Arg Gly Pro Ala Arg Arg Ser
   225 230 235 240
   Val Leu Ala Leu Thr His Glu Gly Arg Phe His Pro Gly Leu Gly Tyr
   245 250 255
   Glu Gln Arg Tyr His Ser Gly Asp Val Arg Leu Asp Thr Val Gly Ser
   260 265 270
   Asp Ala Tyr Arg Leu Ser Val Ser Arg Ala Leu Ser Ala Asp Gln Gly
   275 280 285
   Ser Tyr Arg Cys Ile Val Ser Glu Trp Ile Ala Glu Gln Gly Asn Trp
   290 295 300
   Gln Glu Ile Gln Glu Lys Ala Val Glu Val Ala Thr Val Val Ile Gln
   305 310 315 320
   Pro Ser Val Leu Arg Ala Ala Val Pro Lys Asn Val Ser Val Ala Glu
   325 330 335
   Gly Lys Glu Leu Asp Leu Thr Cys Asn Ile Thr Thr Asp Arg Ala Asp
   340 345 350
   Asp Val Arg Pro Glu Val Thr Trp Ser Phe Ser Arg Met Pro Asp Ser
   355 360 365
   Thr Leu Pro Gly Ser Arg Val Leu Ala Arg Leu Asp Arg Asp Ser Leu
   370 375 380
   Val His Ser Ser Pro His Val Ala Leu Ser His Val Asp Ala Arg Ser
   385 390 395 400
   Tyr His Leu Leu Val Arg Asp Val Ser Lys Glu Asn Ser Gly Tyr Tyr
   405 410 415
   Tyr Cys His Val Ser Leu Trp Ala Pro Gly His Asn Arg Ser Trp His
   420 425 430
   Lys Val Ala Glu Ala Val Ser Ser Pro Ala Gly Val Gly Val Thr Trp
   435 440 445
   Leu Glu Pro Asp Tyr Gln Val Tyr Leu Asn Ala Ser Lys Val Pro Gly
       450                 455                 460
   Phe Ala Asp Asp Pro Thr Glu Leu Ala Cys Arg Val Val Asp Thr Lys
   465                 470                 475                 480
   Ser Gly Glu Ala Asn Val Arg Phe Thr Val Ser Trp Tyr Tyr Arg Met
                   485                 490                 495
   Asn Arg Arg Ser Asp Asn Val Val Thr Ser Glu Leu Leu Ala Val Met
               500                 505                 510
   Asp Gly Asp Trp Thr Leu Lys Tyr Gly Glu Arg Ser Lys Gln Arg Ala
           515                 520                 525
   Gln Asp Gly Asp Phe Ile Phe Ser Lys Glu His Thr Asp Thr Phe Asn
       530                 535                 540
   Phe Arg Ile Gln Arg Thr Thr Glu Glu Asp Arg Gly Asn Tyr Tyr Cys
   545                 550                 555                 560
   Val Val Ser Ala Trp Thr Lys Gln Arg Asn Asn Ser Trp Val Lys Ser
                   565                 570                 575
   Lys Asp Val Phe Ser Lys Pro Val Asn Ile Phe Trp Ala Leu Glu Asp
               580                 585                 590
   Ser Val Leu Val Val Lys Ala Arg Gln Pro Lys Pro Phe Phe Ala Ala
           595                 600                 605
   Gly Asn Thr Phe Glu Met Thr Cys Lys Val Ser Ser Lys Asn Ile Lys
       610                 615                 620
   Ser Pro Arg Tyr Ser Val Leu Ile Met Ala Glu Lys Pro Val Gly Asp
   625                 630                 635                 640
   Leu Ser Ser Pro Asn Glu Thr Lys Tyr Ile Ile Ser Leu Asp Gln Asp
                   645                 650                 655
   Ser Val Val Lys Leu Glu Asn Trp Thr Asp Ala Ser Arg Val Asp Gly
               660                 665                 670
   Val Val Leu Glu Lys Val Gln Glu Asp Glu Phe Arg Tyr Arg Met Tyr
           675                 680                 685
   Gln Thr Gln Val Ser Asp Ala Gly Leu Tyr Arg Cys Met Val Thr Ala
       690                 695                 700
   Trp Ser Pro Val Arg Gly Ser Leu Trp Arg Glu Ala Ala Thr Ser Leu
   705                 710                 715                 720
   Ser Asn Pro Ile Glu Ile Asp Phe Gln Thr Ser Gly Pro Ile Phe Asn
                   725                 730                 735
   Ala Ser Val His Ser Asp Thr Pro Ser Val Ile Arg Gly Asp Leu Ile
               740                 745                 750
   Lys Leu Phe Cys Ile Ile Thr Val Glu Gly Ala Ala Leu Asp Pro Asp
           755                 760                 765
   Asp Met Ala Phe Asp Val Ser Trp Phe Ala Val His Ser Phe Gly Leu
       770                 775                 780
   Asp Lys Ala Pro Val Leu Leu Ser Ser Leu Asp Arg Lys Gly Ile Val
   785                 790                 795                 800
   Thr Thr Ser Arg Arg Asp Trp Lys Ser Asp Leu Ser Leu Glu Arg Val
                   805                 810                 815
   Ser Val Leu Glu Phe Leu Leu Gln Val His Gly Ser Glu Asp Gln Asp
               820                 825                 830
   Phe Gly Asn Tyr Tyr Cys Ser Val Thr Pro Trp Val Lys Ser Pro Thr
           835                 840                 845
   Gly Ser Trp Gln Lys Glu Ala Glu Ile His Ser Lys Pro Val Phe Ile
       850                 855                 860
   Thr Val Lys Met Asp Val Leu Asn Ala Phe Lys Tyr Pro Leu Leu Ile
   865                 870                 875                 880
   Gly Val Gly Leu Ser Thr Val Ile Gly Leu Leu Ser Cys Leu Ile Gly
                   885                 890                 895
   Tyr Cys Ser Ser His Trp Cys Cys Lys Lys Glu Val Gln Glu Thr Arg
               900                 905                 910
   Arg Glu Arg Arg Arg Leu Met Ser Met Glu Met Asp
           915                 920
<210> 7
   <211> 234
   <212> PRT
   <213> Homo sapiens
<220>
   <221> misc_feature
   <223> CDS de la séquence GS-N2: 49..753
<220>
   <221> misc_feature
   <223> séquence appelée GSP2: proteine homeobox NKX3.1
<400> 7
   Met Leu Arg Val Pro Glu Pro Arg Pro Gly Glu Ala Lys Ala Glu Gly
   1               5                   10                  15
   Ala Ala Pro Pro Thr Pro Ser Lys Pro Leu Thr Ser Phe Leu Ile Gln
               20                  25                  30
   Asp Ile Leu Arg Asp Gly Ala Gln Arg Gln Gly Gly Arg Thr Ser Ser
           35                  40                  45
   Gln Arg Gln Arg Asp Pro Glu Pro Glu Pro Glu Pro Glu Pro Glu Gly
       50                  55                  60
   Gly Arg Ser Arg Ala Gly Ala Gln Asn Asp Gln Leu Ser Thr Gly Pro
   65                  70                  75                  80
   Arg Ala Ala Pro Glu Glu Ala Glu Thr Leu Ala Glu Thr Glu Pro Glu
                   85                  90                  95
   Arg His Leu Gly Ser Tyr Leu Leu Asp Ser Glu Asn Thr Ser Gly Ala
               100                 105                 110
   Leu Pro Arg Leu Pro Gln Thr Pro Lys Gln Pro Gln Lys Arg Ser Arg
           115                 120                 125
   Ala Ala Phe Ser His Thr Gln Val Ile Glu Leu Glu Arg Lys Phe Ser
       130                 135                 140
   His Gln Lys Tyr Leu Ser Ala Pro Glu Arg Ala His Leu Ala Lys Asn
   145                 150                 155                 160
   Leu Lys Leu Thr Glu Thr Gln Val Lys Ile Trp Phe Gln Asn Arg Arg
                   165                 170                 175
   Tyr Lys Thr Lys Arg Lys Gln Leu Ser Ser Glu Leu Gly Asp Leu Glu
               180                 185                 190
   Lys His Ser Ser Leu Pro Ala Leu Lys Glu Glu Ala Phe Ser Arg Ala
           195                 200                 205
   Ser Leu Val Ser Val Tyr Asn Ser Tyr Pro Tyr Tyr Pro Tyr Leu Tyr
       210                 215                 220
   Cys Val Gly Ser Trp Ser Pro Ala Phe Trp
   225                 230
<210> 8
   <211> 2000
   <212> PRT
   <213> Homo sapiens
<220>
   <221> misc_feature
   <223> CDS de la séquence GS-N3: 151..6153
<220>
   <221> misc_feature
   <223> séquence appelée GS-P3: hélicase doigt de Zinc
<400> 8
<210> 9
   <211> 913
   <212> PRT
   <213> Homo sapiens
<220>
   <221> misc_feature
   <223> CDS de la séquence GS-N4:67..2808
<220>
   <221> misc_feature
   <223> séquence appelée GS-P4: facteur d'initiation à la traduction euca ryote, sous unité 8 (110kD) (EIF3S8)
<400> 9
   Met Ser Arg Phe Phe Thr Thr Gly Ser Asp Ser Glu Ser Glu Ser Ser
   1               5                   10                  15
   Leu Ser Gly Glu Glu Leu Val Thr Lys Pro Val Gly Gly Asn Tyr Gly
               20                  25                  30
   Lys Gln Pro Leu Leu Leu Ser Glu Asp Glu Glu Asp Thr Lys Arg Val
           35                  40                  45
   Val Arg Ser Ala Lys Asp Lys Arg Phe Glu Glu Leu Thr Asn Leu Ile
       50                  55                  60
   Arg Thr Ile Arg Asn Ala Met Lys Ile Arg Asp Val Thr Lys Cys Leu
   65                  70                  75                  80
   Glu Glu Phe Glu Leu Leu Gly Lys Ala Tyr Gly Lys Ala Lys Ser Ile
                   85                  90                  95
   Val Asp Lys Glu Gly Val Pro Arg Phe Tyr Ile Arg Ile Leu Ala Asp
               100                 105                 110
   Leu Glu Asp Tyr Leu Asn Glu Leu Trp Glu Asp Lys Glu Gly Lys Lys
           115                 120                 125
   Lys Met Asn Lys Asn Asn Ala Lys Ala Leu Ser Thr Leu Arg Gln Lys
       130                 135                 140
   Ile Arg Lys Tyr Asn Arg Asp Phe Glu Ser His Ile Thr Ser Tyr Lys
   145                 150                 155                 160
   Gln Asn Pro Glu Gln Ser Ala Asp Glu Asp Ala Glu Lys Asn Glu Glu
                   165                 170                 175
   Asp Ser Glu Gly Ser Ser Asp Glu Asp Glu Asp Glu Asp Gly Val Ser
               180                 185                 190
   Ala Ala Thr Phe Leu Lys Lys Lys Ser Glu Ala Pro Ser Gly Glu Ser
           195                 200                 205
   Arg Lys Phe Leu Lys Lys Met Asp Asp Glu Asp Glu Asp Ser Glu Asp
       210                 215                 220
   Ser Glu Asp Asp Glu Asp Trp Asp Thr Gly Ser Thr Ser Ser Asp Ser
   225                 230                 235                 240
   Asp Ser Glu Glu Glu Glu Gly Lys Gln Thr Ala Leu Ala Ser Arg Phe
                   245                 250                 255
   Leu Lys Lys Ala Pro Thr Thr Asp Glu Asp Lys Lys Ala Ala Glu Lys
               260                 265                 270
   Lys Arg Glu Asp Lys Ala Lys Lys Lys His Asp Arg Lys Ser Lys Arg
           275                 280                 285
   Leu Asp Glu Glu Glu Glu Asp Asn Glu Gly Gly Glu Trp Glu Arg Val
       290                 295                 300
   Arg Gly Gly Val Pro Leu Val Lys Glu Lys Pro Lys Met Phe Ala Lys
   305                 310                 315                 320
   Gly Thr Glu Ile Thr His Ala Val Val Ile Lys Lys Leu Asn Glu Ile
                   325                 330                 335
   Leu Gln Ala Arg Gly Lys Lys Gly Thr Asp Arg Ala Ala Gln Ile Glu
               340                 345                 350
   Leu Leu Gln Leu Leu Val Gln Ile Ala Ala Glu Asn Asn Leu Gly Glu
           355                 360                 365
   Gly Val Ile Val Lys Ile Lys Phe Asn Ile Ile Ala Ser Leu Tyr Asp
       370                 375                 380
   Tyr Asn Pro Asn Leu Ala Thr Tyr Met Lys Pro Glu Met Trp Gly Lys
   385                 390                 395                 400
   Cys Leu Asp Cys Ile Asn Glu Leu Met Asp Ile Leu Phe Ala Asn Pro
                   405                 410                 415
   Asn Ile Phe Val Gly Glu Asn Ile Leu Glu Glu Ser Glu Asn Leu His
               420                 425                 430
   Asn Ala Asp Gln Pro Leu Arg Val Arg Gly Cys Ile Leu Thr Leu Val
           435                 440                 445
   Glu Arg Met Asp Glu Glu Phe Thr Lys Ile Met Gln Asn Thr Asp Pro
       450                 455                 460
   His Ser Gln Glu Tyr Val Glu His Leu Lys Asp Glu Ala Gln Val Cys
   465                 470                 475                 480
   Ala Ile Ile Glu Arg Val Gln Arg Tyr Leu Glu Glu Lys Gly Thr Thr
                   485                 490                 495
   Glu Glu Val Cys Arg Ile Tyr Leu Leu Arg Ile Leu His Thr Tyr Tyr
               500                 505                 510
   Lys Phe Asp Tyr Lys Ala His Gln Arg Gln Leu Thr Pro Pro Glu Gly
           515                 520                 525
   Ser Ser Lys Ser Glu Gln Asp Gln Ala Glu Asn Glu Gly Glu Asp Ser
       530                 535                 540
   Ala Val Leu Met Glu Arg Leu Cys Lys Tyr Ile Tyr Ala Lys Asp Arg
   545                 550                 555                 560
   Thr Asp Arg Ile Arg Thr Cys Ala Ile Leu Cys His Ile Tyr His His
                   565                 570                 575
   Ala Leu His Ser Arg Trp Tyr Gln Ala Arg Asp Leu Met Leu Met Ser
               580                 585                 590
   His Leu Gln Asp Asn Ile Gln His Ala Asp Pro Pro Val Gln Ile Leu
           595                 600                 605
   Tyr Asn Arg Thr Met Val Gln Leu Gly Ile Cys Ala Phe Arg Gln Gly
       610                 615                 620
   Leu Thr Lys Asp Ala His Asn Ala Leu Leu Asp Ile Gln Ser Ser Gly
   625                 630                 635                 640
   Arg Ala Lys Glu Leu Leu Gly Gln Gly Leu Leu Leu Arg Ser Leu Gln
                   645                 650                 655
   Glu Arg Asn Gln Glu Gln Glu Lys Val Glu Arg Arg Arg Gln Val Pro
               660                 665                 670
   Phe His Leu His Ile Asn Leu Glu Leu Leu Glu Cys Val Tyr Leu Val
           675                 680                 685
   Ser Ala Met Leu Leu Glu Ile Pro Tyr Met Ala Ala His Glu Ser Asp
       690                 695                 700
   Ala Arg Arg Arg Met Ile Ser Lys Gln Phe His His Gln Leu Arg Val
   705                 710                 715                 720
   Gly Glu Arg Gln Pro Leu Leu Gly Pro Pro Glu Ser Met Arg Glu His
                   725                 730                 735
   Val Val Ala Ala Ser Lys Ala Met Lys Met Gly Asp Trp Lys Thr Cys
               740                 745                 750
   His Ser Phe Ile Ile Asn Glu Lys Met Asn Gly Lys Val Trp Asp Leu
           755                 760                 765
   Phe Pro Glu Ala Asp Lys Val Arg Thr Met Leu Val Arg Lys Ile Gln
       770                 775                 780
   Glu Glu Ser Leu Arg Thr Tyr Leu Phe Thr Tyr Ser Ser Val Tyr Asp
   785                 790                 795                 800
   Ser Ile Ser Met Glu Thr Leu Ser Asp Met Phe Glu Leu Asp Leu Pro
                   805                 810                 815
   Thr Val His Ser Ile Ile Ser Lys Met Ile Ile Asn Glu Glu Leu Met
               820                 825                 830
   Ala Ser Leu Asp Gln Pro Thr Gln Thr Val Val Met His Arg Thr Glu
           835                 840                 845
   Pro Thr Ala Gln Gln Asn Leu Ala Leu Gln Leu Ala Glu Lys Leu Gly
       850                 855                 860
   Ser Leu Val Glu Asn Asn Glu Arg Val Phe Asp His Lys Gln Gly Thr
   865                 870                 875                 880
   Tyr Gly Gly Tyr Phe Arg Asp Gln Lys Asp Gly Tyr Arg Lys Asn Glu
                   885                 890                 895
   Gly Tyr Met Arg Arg Gly Gly Tyr Arg Gln Gln Gln Ser Gln Thr Ala
               900                 905                 910
   Tyr
<210> 10
   <211> 325
   <212> PRT
   <213> Homo sapiens
<220>
   <221> misc_feature
   <223> CDS de la séquence GS-N5:407..1384
<220>
   <221> misc_feature
   <223> séquence appelée GS-P5: protéine similaire au facteur d'initiati on à la traduction eucaryote, sous unité 8 (110kD)
<400> 10
   Met Leu Met Ser His Leu Gln Asp Asn Ile Gln His Ala Asp Pro Pro
   1               5                   10                  15
   Val Gln Ile Leu Tyr Asn Arg Thr Met Val Gln Leu Gly Ile Cys Ala
               20                  25                  30
   Phe Arg Gln Gly Leu Thr Lys Asp Ala His Asn Ala Leu Leu Asp Ile
           35                  40                  45
   Gln Ser Ser Gly Arg Ala Lys Glu Leu Leu Gly Gln Gly Leu Leu Leu
       50                  55                  60
   Arg Ser Leu Gln Glu Arg Asn Gln Glu Gln Glu Lys Val Glu Arg Arg
   65                  70                  75                  80
   Arg Gln Val Pro Phe His Leu His Ile Asn Leu Glu Leu Leu Glu Cys
                   85                  90                  95
   Val Tyr Leu Val Ser Ala Met Leu Leu Glu Ile Pro Tyr Met Ala Ala
               100                 105                 110
   His Glu Ser Asp Ala Arg Arg Arg Met Ile Ser Lys Gln Phe His His
           115                 120                 125
   Gln Leu Arg Val Gly Glu Arg Gln Pro Leu Leu Gly Pro Pro Glu Ser
       130                 135                 140
   Met Arg Glu His Val Val Ala Ala Ser Lys Ala Met Lys Met Gly Asp
   145                 150                 155                 160
   Trp Lys Thr Cys His Ser Phe Ile Ile Asn Glu Lys Met Asn Gly Lys
                   165                 170                 175
   Val Trp Asp Leu Phe Pro Glu Ala Asp Lys Val Arg Thr Met Leu Val
               180                 185                 190
   Arg Lys Ile Gln Glu Glu Ser Leu Arg Thr Tyr Leu Phe Thr Tyr Ser
           195                 200                 205
   Ser Val Tyr Asp Ser Ile Ser Met Glu Thr Leu Ser Asp Met Phe Glu
       210                 215                 220
   Leu Asp Leu Pro Thr Val His Ser Ile Ile Ser Lys Met Ile Ile Asn
   225                 230                 235                 240
   Glu Glu Leu Met Ala Ser Leu Asp Gln Pro Thr Gln Thr Val Val Met
                   245                 250                 255
   His Arg Thr Glu Pro Thr Ala Gln Gln Asn Leu Ala Leu Gln Leu Ala
               260                 265                 270
   Glu Lys Leu Gly Ser Leu Val Glu Asn Asn Glu Arg Val Phe Asp His
           275                 280                 285
   Lys Gln Gly Thr Tyr Gly Gly Tyr Phe Arg Asp Gln Lys Asp Gly Tyr
       290                 295                 300
   Arg Lys Asn Glu Gly Tyr Met Arg Arg Gly Gly Tyr Arg Gln Gln Gln
   305                 310                 315                 320
   Ser Gln Thr Ala Tyr
                   325
<210> 11
   <211> 392
   <212> DNA
   <213> Homo sapiens
<220>
   <221> misc_feature
   <223> séquence nucléique antisens de GS-N1:4369... 3978
<400> 11
   ttcccctttg gaagagagag cagtgttaga tccttcaaag tcaagataaa agcagaggat     60
   tgcaattagg gcatgagtca ccagcgagcc tcagaaaacc ctactgggat gcttccgaaa    120
   ggccagtgct gtgcaggggg cgagacactt cagggagcgg gaggctccaa ggacagagct    180
   tttaaagcag cagcagaagc ctgggatgtg gaagatctct gtctactctc ctattcctga    240
   tgggagcagt cgggcaaagc acaccgctct tgactctgct ctacaggcca ttcacataac    300
   agcaaattgc tgggtttcct tcatgtccct attaggaaag caaaacatac cacaaagtat    360
   accctttctc actaaccccc tgcagtggac ct                                  392
<210> 12
   <211> 250
   <212> DNA
   <213> Homo sapiens
<220>
   <221> misc_feature
   <223> séquence nucléique antisens de GS-N2: 2859...2619
<400> 12
   ttcagaggag gggagagaaa gagtactcag tagggactga gcactaaatg cttattttaa     60
   aagaaatgta aagagcagaa agcaattcag gctaccctgc cttttgtgct ggctagcact    120
   ccggtcggtg tcagcagcac gtggcattga acattgcaat gtggagccca aaccacagaa    180
   aatggggtga aattggccaa ctttctatta acttatgttg gcaattttgc caccaacagt    240
   aagctggccc                                                           250
<210> 13
   <211> 359
   <212> DNA
   <213> Homo sapiens
<220>
   <221> misc_feature
   <223> séquence nucléique antisens de GS-N3: 3980...3622
<400> 13
   tgcacgtcag tgtcctcagt tgcatcctgg ttccggtccg acagccgagc gatggcctca     60
   ttgtcataat gaatcacact gctgtcctcc tccttgttct ccccctcgtt ttcatccttg    120
   aatagctctt cagtgccaaa tttgagaatg tcgtcaagct cctgcttgga catggagcct    180
   gccttggagc ccagcccagg ccgcacaacc aggtgtgtca gcatcatctt tctcttggcc    240
   acttgtgtga ttcgctcttc cactgacgcg cgagtcacaa accggtaaat catcactttg    300
   ttggcctggc caatccgatg agcccggcta aaggcctgga tgtcattatg ggggttcca      359
<210> 14
   <211> 167
   <212> DNA
   <213> Homo sapiens
<220>
   <221> misc_feature
   <223> séquence nucléique antisens de GS-N4: 2832...2666
<220>
   <221> misc_feature
   <223> séquence nucléique antisens de GS-N5:1408...1242
<400> 14
   caggcgggaa acagagtgga gagctcagta ggccgtctga gactgctgct ggcggtagcc     60
   accgcggcgc atgtagccct cgtttttgcg gtagccgtcc ttctggtctc ggaagtagcc    120
   cccgtaggtg ccctgcttgt ggtcaaacac ccgttcgttg ttctcca                  167
<210> 15
   <211> 5852
   <212> DNA
   <213> Homo sapiens
<220>
   <221> misc_feature
   <223> vecteur d'expression appelé GS-V1 contenant la séquence antisens de GS-N1; antisens:1108...1499
<400> 15
   tcaatattgg ccattagcca tattattcat tggttatata gcataaatca atattggcta     60
   ttggccattg catacgttgt atctatatca taatatgtac atttatattg gctcatgtcc    120
   aatatgaccg ccatgttggc attgattatt gactagttat taatagtaat caattacggg    180
   gtcattagtt catagcccat atatggagtt ccgcgttaca taacttacgg taaatggccc    240
   gcctggctga ccgcccaacg acccccgccc attgacgtca ataatgacgt atgttcccat    300
   agtaacgcca atagggactt tccattgacg tcaatgggtg gagtatttac ggtaaactgc    360
   ccacttggca gtacatcaag tgtatcatat gccaagtccg ccccctattg acgtcaatga    420
   cggtaaatgg cccgcctggc attatgccca gtacatgacc ttacgggact ttcctacttg    480
   gcagtacatc tacgtattag tcatcgctat taccatggtg atgcggtttt ggcagtacac    540
   caatgggcgt ggatagcggt ttgactcacg gggatttcca agtctccacc ccattgacgt    600
   caatgggagt ttgttttggc accaaaatca acgggacttt ccaaaatgtc gtaacaactg    660
   cgatcgcccg ccccgttgac gcaaatgggc ggtaggcgtg tacggtggga ggtctatata    720
   agcagagctc gtttagtgaa ccgtcagatc actagaagct ttattgcggt agtttatcac    780
   agttaaattg ctaacgcagt cagtgcttct gacacaacag tctcgaactt aagctgcagt    840
   gactctctta aggtagcctt gcagaagttg gtcgtgaggc actgggcagg taagtatcaa    900
   ggttacaaga caggtttaag gagaccaata gaaactgggc ttgtcgagac agagaagact    960
   cttgcgtttc tgataggcac ctattggtct tactgacatc cactttgcct ttctctccac   1020
   aggtgtccac tcccagttca attacagctc ttaaggctag agtacttaat acgactcact   1080
   ataggctagc ctcgagaatt cacgcgtttc ccctttggaa gagagagcag tgttagatcc   1140
   ttcaaagtca agataaaagc agaggattgc aattagggca tgagtcacca gcgagcctca   1200
   gaaaacccta ctgggatgct tccgaaaggc cagtgctgtg caggggcgca gacacttcag   1260
   ggagcgggag gctccaagga cagagctttt aaagcagcag cagaagcctg ggatgtggaa   1320
   gatctctgtc tactctccta ttcctgatgg gagcagtcgg gcaaagcaca ccgctcttga   1380
   ctctgctcta caggccattc acataacagc aaattgctgg gtttccttca tgtccctatt   1440
   aggaaagcaa aacataccac aaagtatacc ctttctcact aaccccctgc agtggacctg   1500
   tcgacccggg cggccgcttc cctttagtga gggttaatgc ttcgagcaga catgataaga   1560
   tacattgatg agtttggaca aaccacaact agaatgcagt gaaaaaaatg ctttatttgt   1620
   gaaatttgtg atgctattgc tttatttgta accattataa gctgcaataa acaagttaac   1680
   aacaacaatt gcattcattt tatgtttcag gttcaggggg agatgtggga ggttttttaa   1740
   agcaagtaaa acctctacaa atgtggtaaa atccgataag gatcgatccg ggctggcgta   1800
   atagcgaaga ggcccgcacc gatcgccctt cccaacagtt gcgcagcctg aatggcgaat   1860
   ggacgcgccc tgtagcggcg cattaagcgc ggcgggtgtg gtggttacgc gcagcgtgac   1920
   cgctacactt gccagcgccc tagcgcccgc tcctttcgct ttcttccctt cctttctcgc   1980
   cacgttcgcc ggctttcccc gtcaagctct aaatcggggg ctccctttag ggttccgatt   2040
   tagtgcttta cggcacctcg accccaaaaa acttgattag ggtgatggtt cacgtagtgg   2100
   gccatcgccc tgatagacgg tttttcgccc tttgacgttg gagtccacgt tctttaatag   2160
   tggactcttg ttccaaactg gaacaacact caaccctatc tcggtctatt cttttgattt   2220
   ataagggatt ttgccgattt cggcctattg gttaaaaaat gagctgattt aacaaaaatt   2280
   taacgcgaat tttaacaaaa tattaacgct tacaatttcc tgatgcggta ttttctcctt   2340
   acgcatctgt gcggtatttc acaccgcata cgcggatctg cgcagcacca tggcctgaaa   2400
   taacctctga aagaggaact tggttaggta ccttctgagg cggaaagaac cagctgtgga   2460
   atgtgtgtca gttagggtgt ggaaagtccc caggctcccc agcaggcaga agtatgcaaa   2520
   gcatgcatct caattagtca gcaaccaggt gtggaaagtc cccaggctcc ccagcaggca   2580
   gaagtatgca aagcatgcat ctcaattagt cagcaaccat agtcccgccc ctaactccgc   2640
   ccatcccgcc cctaactccg cccagttccg cccattctcc gccccatggc tgactaattt   2700
   ttttatttta tgcagaggcc gaggccgcct cggcctctga gctattccag aagtagtgag   2760
   gaggcttttt tggaggccta ggcttttgca aaaagcttga ttcttctgac acaacagtct   2820
   cgaacttaag gctagagcca ccatgattga acaagatgga ttgcacgcag gttctccggc 2880
   cgcttgggtg gagaggctat tcggctatga ctgggcacaa cagacaatcg gctgctctga 2940
   tgccgccgtg ttccggctgt cagcgcaggg gcgcccggtt ctttttgtca agaccgacct 3000
   gtccggtgcc ctgaatgaac tgcaggacga ggcagcgcgg ctatcgtggc tggccacgac 3060
   gggcgttcct tgcgcagctg tgctcgacgt tgtcactgaa gcgggaaggg actggctgct 3120
   attgggcgaa gtgccggggc aggatctcct gtcatctcac cttgctcctg ccgagaaagt 3180
   atccatcatg gctgatgcaa tgcggcggct gcatacgctt gatccggcta cctgcccatt 3240
   cgaccaccaa gcgaaacatc gcatcgagcg agcacgtact cggatggaag ccggtcttgt 3300
   cgatcaggat gatctggacg aagagcatca ggggctcgcg ccagccgaac tgttcgccag 3360
   gctcaaggcg cgcatgcccg acggcgagga tctcgtcgtg acccatggcg atgcctgctt 3420
   gccgaatatc atggtggaaa atggccgctt ttctggattc atcgactgtg gccggctggg 3480
   tgtggcggac cgctatcagg acatagcgtt ggctacccgt gatattgctg aagagcttgg 3540
   cggcgaatgg gctgaccgct tcctcgtgct ttacggtatc gccgctcccg attcgcagcg 3600
   catcgccttc tatcgccttc ttgacgagtt cttctgagcg ggactctggg gttcgaaatg 3660
   accgaccaag cgacgcccaa cctgccatca cgatggccgc aataaaatat ctttattttc 3720
   attacatctg tgtgttggtt ttttgtgtga atcgatagcg ataaggatcc gcgtatggtg 3780
   cactctcagt acaatctgct ctgatgccgc atagttaagc cagccccgac acccgccaac 3840
   acccgctgac gcgccctgac gggcttgtct gctcccggca tccgcttaca gacaagctgt 3900
   gaccgtctcc gggagctgca tgtgtcagag gttttcaccg tcatcaccga aacgcgcgag 3960
   acgaaagggc ctcgtgatac gcctattttt ataggttaat gtcatgataa taatggtttc 4020
   ttagacgtca ggtggcactt ttcggggaaa tgtgcgcgga acccctattt gtttattttt 4080
   ctaaatacat tcaaatatgt atccgctcat gagacaataa ccctgataaa tgcttcaata 4140
   atattgaaaa aggaagagta tgagtattca acatttccgt gtcgccctta ttcccttttt 4200
   tgcggcattt tgccttcctg tttttgctca cccagaaacg ctggtgaaag taaaagatgc 4260
   tgaagatcag ttgggtgcac gagtgggtta catcgaactg gatctcaaca gcggtaagat 4320
   ccttgagagt tttcgccccg aagaacgttt tccaatgatg agcactttta aagttctgct 4380
   atgtggcgcg gtattatccc gtattgacgc cgggcaagag caactcggtc gccgcataca 4440
   ctattctcag aatgacttgg ttgagtactc accagtcaca gaaaagcatc ttacggatgg 4500
   catgacagta agagaattat gcagtgctgc cataaccatg agtgataaca ctgcggccaa 4560
   cttacttctg acaacgatcg gaggaccgaa ggagctaacc gcttttttgc acaacatggg 4620
   ggatcatgta actcgccttg atcgttggga accggagctg aatgaagcca taccaaacga 4680
   cgagcgtgac accacgatgc ctgtagcaat ggcaacaacg ttgcgcaaac tattaactgg 4740
   cgaactactt actctagctt cccggcaaca attaatagac tggatggagg cggataaagt 4800
   tgcaggacca cttctgcgct cggcccttcc ggctggctgg tttattgctg ataaatctgg 4860
   agccggtgag cgtgggtctc gcggtatcat tgcagcactg gggccagatg gtaagccctc 4920
   ccgtatcgta gttatctaca cgacggggag tcaggcaact atggatgaac gaaatagaca 4980
   gatcgctgag ataggtgcct cactgattaa gcattggtaa ctgtcagacc aagtttactc 5040
   atatatactt tagattgatt taaaacttca tttttaattt aaaaggatct aggtgaagat 5100
   cctttttgat aatctcatga ccaaaatccc ttaacgtgag ttttcgttcc actgagcgtc 5160
   agaccccgta gaaaagatca aaggatcttc ttgagatcct ttttttctgc gcgtaatctg 5220
   ctgcttgcaa acaaaaaaac caccgctacc agcggtggtt tgtttgccgg atcaagagct 5280
   accaactctt tttccgaagg taactggctt cagcagagcg cagataccaa atactgttct 5340
   tctagtgtag ccgtagttag gccaccactt caagaactct gtagcaccgc ctacatacct 5400
   cgctctgcta atcctgttac cagtggctgc tgccagtggc gataagtcgt gtcttaccgg 5460
   gttggactca agacgatagt taccggataa ggcgcagcgg tcgggctgaa cggggggttc 5520
   gtgcacacag cccagcttgg agcgaacgac ctacaccgaa ctgagatacc tacagcgtga 5580
   gctatgagaa agcgccacgc ttcccgaagg gagaaaggcg gacaggtatc cggtaagcgg 5640
   cagggtcgga acaggagagc gcacgaggga gcttccaggg ggaaacgcct ggtatcttta 5700
   tagtcctgtc gggtttcgcc acctctgact tgagcgtcga tttttgtgat gctcgtcagg 5760
   ggggcggagc ctatggaaaa acgccagcaa cgcggccttt ttacggttcc tggccttttg 5820
   ctggcctttt gctcacatgg ctcgacagat ct 5852
<210> 16
   <211> 5710
   <212> DNA
   <213> Homo sapiens
<220>
   <221> misc_feature
   <222> (1).. (5710)
   <223> vecteur d'expression appelé GS-V2 contenant la séquence antisens de GS-N2; antisens:2859...2619
<400> 16
   tcaatattgg ccattagcca tattattcat tggttatata gcataaatca atattggcta     60
   ttggccattg catacgttgt atctatatca taatatgtac atttatattg gctcatgtcc    120
   aatatgaccg ccatgttggc attgattatt gactagttat taatagtaat caattacggg    180
   gtcattagtt catagcccat atatggagtt ccgcgttaca taacttacgg taaatggccc    240
   gcctggctga ccgcccaacg acccccgccc attgacgtca ataatgacgt atgttcccat 300
   agtaacgcca atagggactt tccattgacg tcaatgggtg gagtatttac ggtaaactgc 360
   ccacttggca gtacatcaag tgtatcatat gccaagtccg ccccctattg acgtcaatga 420
   cggtaaatgg cccgcctggc attatgccca gtacatgacc ttacgggact ttcctacttg 480
   gcagtacatc tacgtattag tcatcgctat taccatggtg atgcggtttt ggcagtacac 540
   caatgggcgt ggatagcggt ttgactcacg gggatttcca agtctccacc ccattgacgt 600
   caatgggagt ttgttttggc accaaaatca acgggacttt ccaaaatgtc gtaacaactg 660
   cgatcgcccg ccccgttgac gcaaatgggc ggtaggcgtg tacggtggga ggtctatata 720
   agcagagctc gtttagtgaa ccgtcagatc actagaagct ttattgcggt agtttatcac 780
   agttaaattg ctaacgcagt cagtgcttct gacacaacag tctcgaactt aagctgcagt 840
   gactctctta aggtagcctt gcagaagttg gtcgtgaggc actgggcagg taagtatcaa 900
   ggttacaaga caggtttaag gagaccaata gaaactgggc ttgtcgagac agagaagact 960
   cttgcgtttc tgataggcac ctattggtct tactgacatc cactttgcct ttctctccac 1020
   aggtgtccac tcccagttca attacagctc ttaaggctag agtacttaat acgactcact 1080
   ataggctagc ctcgagaatt cacgcgtttc agaggagggg agagaaagag tactcagtag 1140
   ggactgagca ctaaatgctt attttaaaag aaatgtaaag agcagaaagc aattcaggct 1200
   accctgcctt ttgtgctggc tagcactccg gtcggtgtca gcagcacgtg gcattgaaca 1260
   ttgcaatgtg gagcccaaac cacagaaaat ggggtgaaat tggccaactt tctattaact 1320
   tatgttggca attttgccac caacagtaag ctggcccgtc gacccgggcg gccgcttccc 1380
   tttagtgagg gttaatgctt cgagcagaca tgataagata cattgatgag tttggacaaa 1440
   ccacaactag aatgcagtga aaaaaatgct ttatttgtga aatttgtgat gctattgctt 1500
   tatttgtaac cattataagc tgcaataaac aagttaacaa caacaattgc attcatttta 1560
   tgtttcaggt tcagggggag atgtgggagg ttttttaaag caagtaaaac ctctacaaat 1620
   gtggtaaaat ccgataagga tcgatccggg ctggcgtaat agcgaagagg cccgcaccga 1680
   tcgcccttcc caacagttgc gcagcctgaa tggcgaatgg acgcgccctg tagcggcgca 1740
   ttaagcgcgg cgggtgtggt ggttacgcgc agcgtgaccg ctacacttgc cagcgcccta 1800
   gcgcccgctc ctttcgcttt cttcccttcc tttctcgcca cgttcgccgg ctttccccgt 1860
   caagctctaa atcgggggct ccctttaggg ttccgattta gtgctttacg gcacctcgac 1920
   cccaaaaaac ttgattaggg tgatggttca cgtagtgggc catcgccctg atagacggtt 1980
   tttcgccctt tgacgttgga gtccacgttc tttaatagtg gactcttgtt ccaaactgga 2040
   acaacactca accctatctc ggtctattct tttgatttat aagggatttt gccgatttcg 2100
   gcctattggt taaaaaatga gctgatttaa caaaaattta acgcgaattt taacaaaata 2160
   ttaacgctta caatttcctg atgcggtatt ttctccttac gcatctgtgc ggtatttcac 2220
   accgcatacg cggatctgcg cagcaccatg gcctgaaata acctctgaaa gaggaacttg 2280
   gttaggtacc ttctgaggcg gaaagaacca gctgtggaat gtgtgtcagt tagggtgtgg 2340
   aaagtcccca ggctccccag caggcagaag tatgcaaagc atgcatctca attagtcagc 2400
   aaccaggtgt ggaaagtccc caggctcccc agcaggcaga agtatgcaaa gcatgcatct 2460
   caattagtca gcaaccatag tcccgcccct aactccgccc atcccgcccc taactccgcc 2520
   cagttccgcc cattctccgc cccatggctg actaattttt tttatttatg cagaggccga 2580
   ggccgcctcg gcctctgagc tattccagaa gtagtgagga ggcttttttg gaggcctagg 2640
   cttttgcaaa aagcttgatt cttctgacac aacagtctcg aacttaaggc tagagccacc 2700
   atgattgaac aagatggatt gcacgcaggt tctccggccg cttgggtgga gaggctattc 2760
   ggctatgact gggcacaaca gacaatcggc tgctctgatg ccgccgtgtt ccggctgtca 2820
   gcgcaggggc gcccggttct ttttgtcaag accgacctgt ccggtgccct gaatgaactg 2880
   caggacgagg cagcgcggct atcgtggctg gccacgacgg gcgttccttg cgcagctgtg 2940
   ctcgacgttg tcactgaagc gggaagggac tggctgctat tgggcgaagt gccggggcag 3000
   gatctcctgt catctcacct tgctcctgcc gagaaagtat ccatcatggc tgatgcaatg 3060
   cggcggctgc atacgcttga tccggctacc tgcccattcg accaccaagc gaaacatcgc 3120
   atcgagcgag cacgtactcg gatggaagcc ggtcttgtcg atcaggatga tctggacgaa 3180
   gagcatcagg ggctcgcgcc agccgaactg ttcgccaggc tcaaggcgcg catgcccgac 3240
   ggcgaggatc tcgtcgtgac ccatggcgat gcctgcttgc cgaatatcat ggtggaaaat 3300
   ggccgctttt ctggattcat cgactgtggc cggctgggtg tggcggaccg ctatcaggac 3360
   atagcgttgg ctacccgtga tattgctgaa gagcttggcg gcgaatgggc tgaccgcttc 3420
   ctcgtgcttt acggtatcgc cgctcccgat tcgcagcgca tcgccttcta tcgccttctt 3480
   gacgagttct tctgagcggg actctggggt tcgaaatgac cgaccaagcg acgcccaacc 3540
   tgccatcacg atggccgcaa taaaatatct ttattttcat tacatctgtg tgttggtttt 3600
   ttgtgtgaat cgatagcgat aaggatccgc gtatggtgca ctctcagtac aatctgctct 3660
   gatgccgcat agttaagcca gccccgacac ccgccaacac ccgctgacgc gccctgacgg 3720
   gcttgtctgc tcccggcatc cgcttacaga caagctgtga ccgtctccgg gagctgcatg 3780
   tgtcagaggt tttcaccgtc atcaccgaaa cgcgcgagac gaaagggcct cgtgatacgc 3840
   ctatttttat aggttaatgt catgataata atggtttctt agacgtcagg tggcactttt 3900
   cggggaaatg tgcgcggaac ccctatttgt ttatttttct aaatacattc aaatatgtat 3960
   ccgctcatga gacaataacc ctgataaatg cttcaataat attgaaaaag gaagagtatg 4020
   agtattcaac atttccgtgt cgcccttatt cccttttttg cggcattttg ccttcctgtt 4080
   tttgctcacc cagaaacgct ggtgaaagta aaagatgctg aagatcagtt gggtgcacga 4140
   gtgggttaca tcgaactgga tctcaacagc ggtaagatcc ttgagagttt tcgccccgaa 4200
   gaacgttttc caatgatgag cacttttaaa gttctgctat gtggcgcggt attatcccgt 4260
   attgacgccg ggcaagagca actcggtcgc cgcatacact attctcagaa tgacttggtt   4320
   gagtactcac cagtcacaga aaagcatctt acggatggca tgacagtaag agaattatgc   4380
   agtgctgcca taaccatgag tgataacact gcggccaact tacttctgac aacgatcgga   4440
   ggaccgaagg agctaaccgc ttttttgcac aacatggggg atcatgtaac tcgccttgat   4500
   cgttgggaac cggagctgaa tgaagccata ccaaacgacg agcgtgacac cacgatgcct   4560
   gtagcaatgg caacaacgtt gcgcaaacta ttaactggcg aactacttac tctagcttcc   4620
   cggcaacaat taatagactg gatggaggcg gataaagttg caggaccact tctgcgctcg   4680
   gcccttccgg ctggctggtt tattgctgat aaatctggag ccggtgagcg tgggtctcgc   4740
   ggtatcattg cagcactggg gccagatggt aagccctccc gtatcgtagt tatctacacg   4800
   acggggagtc aggcaactat ggatgaacga aatagacaga tcgctgagat aggtgcctca   4860
   ctgattaagc attggtaact gtcagaccaa gtttactcat atatacttta gattgattta   4920
   aaacttcatt tttaatttaa aaggatctag gtgaagatcc tttttgataa tctcatgacc   4980
   aaaatccctt aacgtgagtt ttcgttccac tgagcgtcag accccgtaga aaagatcaaa   5040
   ggatcttctt gagatccttt ttttctgcgc gtaatctgct gcttgcaaac aaaaaaacca   5100
   ccgctaccag cggtggtttg tttgccggat caagagctac caactctttt tccgaaggta   5160
   actggcttca gcagagcgca gataccaaat actgttcttc tagtgtagcc gtagttaggc   5220
   caccacttca agaactctgt agcaccgcct acatacctcg ctctgctaat cctgttacca   5280
   gtggctgctg ccagtggcga taagtcgtgt cttaccgggt tggactcaag acgatagtta   5340
   ccggataagg cgcagcggtc gggctgaacg gggggttcgt gcacacagcc cagcttggag   5400
   cgaacgacct acaccgaact gagataccta cagcgtgagc tatgagaaag cgccacgctt   5460
   cccgaaggga gaaaggcgga caggtatccg gtaagcggca gggtcggaac aggagagcgc   5520
   acgagggagc ttccaggggg aaacgcctgg tatctttata gtcctgtcgg gtttcgccac   5580
   ctctgacttg agcgtcgatt tttgtgatgc tcgtcagggg ggcggagcct atggaaaaac   5640
   gccagcaacg cggccttttt acggttcctg gccttttgct ggccttttgc tcacatggct   5700
   cgacagatct                                                          5710
<210> 17
   <211> 5819
   <212> DNA
   <213> Homo sapiens
<220>
   <221> misc_feature
   <223> vecteur d'expression appelé GS-V3 contenant la séquence antisens de GS-N3; antisens:1108...1466
<400> 17
   tcaatattgg ccattagcca tattattcat tggttatata gcataaatca atattggcta 60
   ttggccattg catacgttgt atctatatca taatatgtac atttatattg gctcatgtcc 120
   aatatgaccg ccatgttggc attgattatt gactagttat taatagtaat caattacggg 180
   gtcattagtt catagcccat atatggagtt ccgcgttaca taacttacgg taaatggccc 240
   gcctggctga ccgcccaacg acccccgccc attgacgtca ataatgacgt atgttcccat 300
   agtaacgcca atagggactt tccattgacg tcaatgggtg gagtatttac ggtaaactgc 360
   ccacttggca gtacatcaag tgtatcatat gccaagtccg ccccctattg acgtcaatga 420
   cggtaaatgg cccgcctggc attatgccca gtacatgacc ttacgggact ttcctacttg 480
   gcagtacatc tacgtattag tcatcgctat taccatggtg atgcggtttt ggcagtacac 540
   caatgggcgt ggatagcggt ttgactcacg gggatttcca agtctccacc ccattgacgt 600
   caatgggagt ttgttttggc accaaaatca acgggacttt ccaaaatgtc gtaacaactg 660
   cgatcgcccg ccccgttgac gcaaatgggc ggtaggcgtg tacggtggga ggtctatata 720
   agcagagctc gtttagtgaa ccgtcagatc actagaagct ttattgcggt agtttatcac 780
   agttaaattg ctaacgcagt cagtgcttct gacacaacag tctcgaactt aagctgcagt 840
   gactctctta aggtagcctt gcagaagttg gtcgtgaggc actgggcagg taagtatcaa 900
   ggttacaaga caggtttaag gagaccaata gaaactgggc ttgtcgagac agagaagact 960
   cttgcgtttc tgataggcac ctattggtct tactgacatc cactttgcct ttctctccac 1020
   aggtgtccac tcccagttca attacagctc ttaaggctag agtacttaat acgactcact 1080
   ataggctagc ctcgagaatt cacgcgttgc acgtcagtgt cctcagttgc atcctggttc 1140
   cggtccgaca gccgagcgat ggcctcattg tcataatgaa tcacactgct gtcctcctcc 1200
   ttgttctccc cctcgttttc atccttgaat agctcttcag tgccaaattt gagaatgtcg 1260
   tcaagctcct gcttggacat ggagcctgcc ttggagccca gcccaggccg cacaaccagg 1320
   tgtgtcagca tcatctttct cttggccact tgtgtgattc gctcttccac tgacgcgcga 1380
   gtcacaaacc ggtaaatcat cactttgttg gcctggccaa tccgatgagc ccggctaaag 1440
   gcctggatgt cattatgggg gttccagtcg acccgggcgg ccgcttccct ttagtgaggg 1500
   ttaatgcttc gagcagacat gataagatac attgatgagt ttggacaaac cacaactaga 1560
   atgcagtgaa aaaaatgctt tatttgtgaa atttgtgatg ctattgcttt atttgtaacc 1620
   attataagct gcaataaaca agttaacaac aacaattgca ttcattttat gtttcaggtt 1680
   cagggggaga tgtgggaggt tttttaaagc aagtaaaacc tctacaaatg tggtaaaatc 1740
   cgataaggat cgatccgggc tggcgtaata gcgaagaggc ccgcaccgat cgcccttccc 1800
   aacagttgcg cagcctgaat ggcgaatgga cgcgccctgt agcggcgcat taagcgcggc 1860
   gggtgtggtg gttacgcgca gcgtgaccgc tacacttgcc agcgccctag cgcccgctcc 1920
   tttcgctttc ttcccttcct ttctcgccac gttcgccggc tttccccgtc aagctctaaa 1980
   tcgggggctc cctttagggt tccgatttag tgctttacgg cacctcgacc ccaaaaaact 2040
   tgattagggt gatggttcac gtagtgggcc atcgccctga tagacggttt ttcgcccttt 2100
   gacgttggag tccacgttct ttaatagtgg actcttgttc caaactggaa caacactcaa 2160
   ccctatctcg gtctattctt ttgatttata agggattttg ccgatttcgg cctattggtt 2220
   aaaaaatgag ctgatttaac aaaaatttaa cgcgaatttt aacaaaatat taacgcttac 2280
   aatttcctga tgcggtattt tctccttacg catctgtgcg gtatttcaca ccgcatacgc 2340
   ggatctgcgc agcaccatgg cctgaaataa cctctgaaag aggaacttgg ttaggtacct 2400
   tctgaggcgg aaagaaccag ctgtggaatg tgtgtcagtt agggtgtgga aagtccccag 2460
   gctccccagc aggcagaagt atgcaaagca tgcatctcaa ttagtcagca accaggtgtg 2520
   gaaagtcccc aggctcccca gcaggcagaa gtatgcaaag catgcatctc aattagtcag 2580
   caaccatagt cccgccccta actccgccca tcccgcccct aactccgccc agttccgccc 2640
   attctccgcc ccatggctga ctaatttttt ttatttatgc agaggccgag gccgcctcgg 2700
   cctctgagct attccagaag tagtgaggag gcttttttgg aggcctaggc ttttgcaaaa 2760
   agcttgattc ttctgacaca acagtctcga acttaaggct agagccacca tgattgaaca 2820
   agatggattg cacgcaggtt ctccggccgc ttgggtggag aggctattcg gctatgactg 2880
   ggcacaacag acaatcggct gctctgatgc cgccgtgttc cggctgtcag cgcaggggcg 2940
   cccggttctt tttgtcaaga ccgacctgtc cggtgccctg aatgaactgc aggacgaggc 3000
   agcgcggcta tcgtggctgg ccacgacggg cgttccttgc gcagctgtgc tcgacgttgt 3060
   cactgaagcg ggaagggact ggctgctatt gggcgaagtg ccggggcagg atctcctgtc 3120
   atctcacctt gctcctgccg agaaagtatc catcatggct gatgcaatgc ggcggctgca 3180
   tacgcttgat ccggctacct gcccattcga ccaccaagcg aaacatcgca tcgagcgagc 3240
   acgtactcgg atggaagccg gtcttgtcga tcaggatgat ctggacgaag agcatcaggg 3300
   gctcgcgcca gccgaactgt tcgccaggct caaggcgcgc atgcccgacg gcgaggatct 3360
   cgtcgtgacc catggcgatg cctgcttgcc gaatatcatg gtggaaaatg gccgcttttc 3420
   tggattcatc gactgtggcc ggctgggtgt ggcggaccgc tatcaggaca tagcgttggc 3480
   tacccgtgat attgctgaag agcttggcgg cgaatgggct gaccgcttcc tcgtgcttta 3540
   cggtatcgcc gctcccgatt cgcagcgcat cgccttctat cgccttcttg acgagttctt 3600
   ctgagcggga ctctggggtt cgaaatgacc gaccaagcga cgcccaacct gccatcacga 3660
   tggccgcaat aaaatatctt tattttcatt acatctgtgt gttggttttt tgtgtgaatc 3720
   gatagcgata aggatccgcg tatggtgcac tctcagtaca atctgctctg atgccgcata 3780
   gttaagccag ccccgacacc cgccaacacc cgctgacgcg ccctgacggg cttgtctgct 3840
   cccggcatcc gcttacagac aagctgtgac cgtctccggg agctgcatgt gtcagaggtt 3900
   ttcaccgtca tcaccgaaac gcgcgagacg aaagggcctc gtgatacgcc tatttttata 3960
   ggttaatgtc atgataataa tggtttctta gacgtcaggt ggcacttttc ggggaaatgt 4020
   gcgcggaacc cctatttgtt tatttttcta aatacattca aatatgtatc cgctcatgag 4080
   acaataaccc tgataaatgc ttcaataata ttgaaaaagg aagagtatga gtattcaaca 4140
   tttccgtgtc gcccttattc ccttttttgc ggcattttgc cttcctgttt ttgctcaccc 4200
   agaaacgctg gtgaaagtaa aagatgctga agatcagttg ggtgcacgag tgggttacat 4260
   cgaactggat ctcaacagcg gtaagatcct tgagagtttt cgccccgaag aacgttttcc 4320
   aatgatgagc acttttaaag ttctgctatg tggcgcggta ttatcccgta ttgacgccgg 4380
   gcaagagcaa ctcggtcgcc gcatacacta ttctcagaat gacttggttg agtactcacc 4440
   agtcacagaa aagcatctta cggatggcat gacagtaaga gaattatgca gtgctgccat 4500
   aaccatgagt gataacactg cggccaactt acttctgaca acgatcggag gaccgaagga 4560
   gctaaccgct tttttgcaca acatggggga tcatgtaact cgccttgatc gttgggaacc 4620
   ggagctgaat gaagccatac caaacgacga gcgtgacacc acgatgcctg tagcaatggc 4680
   aacaacgttg cgcaaactat taactggcga actacttact ctagcttccc ggcaacaatt 4740
   aatagactgg atggaggcgg ataaagttgc aggaccactt ctgcgctcgg cccttccggc 4800
   tggctggttt attgctgata aatctggagc cggtgagcgt gggtctcgcg gtatcattgc 4860
   agcactgggg ccagatggta agccctcccg tatcgtagtt atctacacga cggggagtca 4920
   ggcaactatg gatgaacgaa atagacagat cgctgagata ggtgcctcac tgattaagca 4980
   ttggtaactg tcagaccaag tttactcata tatactttag attgatttaa aacttcattt 5040
   ttaatttaaa aggatctagg tgaagatcct ttttgataat ctcatgacca aaatccctta 5100
   acgtgagttt tcgttccact gagcgtcaga ccccgtagaa aagatcaaag gatcttcttg 5160
   agatcctttt tttctgcgcg taatctgctg cttgcaaaca aaaaaaccac cgctaccagc 5220
   ggtggtttgt ttgccggatc aagagctacc aactcttttt ccgaaggtaa ctggcttcag 5280
   cagagcgcag ataccaaata ctgttcttct agtgtagccg tagttaggcc accacttcaa 5340
   gaactctgta gcaccgccta catacctcgc tctgctaatc ctgttaccag tggctgctgc 5400
   cagtggcgat aagtcgtgtc ttaccgggtt ggactcaaga cgatagttac cggataaggc 5460
   gcagcggtcg ggctgaacgg ggggttcgtg cacacagccc agcttggagc gaacgaccta 5520
   caccgaactg agatacctac agcgtgagct atgagaaagc gccacgcttc ccgaagggag 5580
   aaaggcggac aggtatccgg taagcggcag ggtcggaaca ggagagcgca cgagggagct 5640
   tccaggggga aacgcctggt atctttatag tcctgtcggg tttcgccacc tctgacttga 5700
   gcgtcgattt ttgtgatgct cgtcaggggg gcggagccta tggaaaaacg ccagcaacgc 5760
   ggccttttta cggttcctgg ccttttgctg gccttttgct cacatggctc gacagatct 5819
<210> 18
   <211> 5627
   <212> DNA
   <213> Homo sapiens
<220>
   <221> misc_feature
   <223> vecteur d'expression appelé GS-V4 contenant la séquence antisens de GS-N4; antisens:1108...2606
<220>
   <221> misc_feature
   <223> vecteur d'expression appelé GS-V4 contenant la séquence antisens de GS-N5; antisens:1108...2606
<400> 18
   tcaatattgg ccattagcca tattattcat tggttatata gcataaatca atattggcta     60
   ttggccattg catacgttgt atctatatca taatatgtac atttatattg gctcatgtcc    120
   aatatgaccg ccatgttggc attgattatt gactagttat taatagtaat caattacggg    180
   gtcattagtt catagcccat atatggagtt ccgcgttaca taacttacgg taaatggccc    240
   gcctggctga ccgcccaacg acccccgccc attgacgtca ataatgacgt atgttcccat    300
   agtaacgcca atagggactt tccattgacg tcaatgggtg gagtatttac ggtaaactgc    360
   ccacttggca gtacatcaag tgtatcatat gccaagtccg ccccctattg acgtcaatga    420
   cggtaaatgg cccgcctggc attatgccca gtacatgacc ttacgggact ttcctacttg    480
   gcagtacatc tacgtattag tcatcgctat taccatggtg atgcggtttt ggcagtacac    540
   caatgggcgt ggatagcggt ttgactcacg gggatttcca agtctccacc ccattgacgt    600
   caatgggagt ttgttttggc accaaaatca acgggacttt ccaaaatgtc gtaacaactg    660
   cgatcgcccg ccccgttgac gcaaatgggc ggtaggcgtg tacggtggga ggtctatata    720
   agcagagctc gtttagtgaa ccgtcagatc actagaagct ttattgcggt agtttatcac    780
   agttaaattg ctaacgcagt cagtgcttct gacacaacag tctcgaactt aagctgcagt    840
   gactctctta aggtagcctt gcagaagttg gtcgtgaggc actgggcagg taagtatcaa    900
   ggttacaaga caggtttaag gagaccaata gaaactgggc ttgtcgagac agagaagact    960
   cttgcgtttc tgataggcac ctattggtct tactgacatc cactttgcct ttctctccac   1020
   aggtgtccac tcccagttca attacagctc ttaaggctag agtacttaat acgactcact   1080
   ataggctagc ctcgagaatt cacgcgtcag gcgggaaaca gagtggagag ctcagtaggc   1140
   cgtctgagac tgctgctggc ggtagccacc gcggcgcatg tagccctcgt ttttgcggta   1200
   gccgtccttc tggtctcgga agtagccccc gtaggtgccc tgcttgtggt caaacacccg   1260
   ttcgttgttc tccagtcgac ccgggcggcc gcttcccttt agtgagggtt aatgcttcga   1320
   gcagacatga taagatacat tgatgagttt ggacaaacca caactagaat gcagtgaaaa   1380
   aaatgcttta tttgtgaaat ttgtgatgct attgctttat ttgtaaccat tataagctgc   1440
   aataaacaag ttaacaacaa caattgcatt cattttatgt ttcaggttca gggggagatg   1500
   tgggaggttt tttaaagcaa gtaaaacctc tacaaatgtg gtaaaatccg ataaggatcg   1560
   atccgggctg gcgtaatagc gaagaggccc gcaccgatcg cccttcccaa cagttgcgca   1620
   gcctgaatgg cgaatggacg cgccctgtag cggcgcatta agcgcggcgg gtgtggtggt   1680
   tacgcgcagc gtgaccgcta cacttgccag cgccctagcg cccgctcctt tcgctttctt   1740
   cccttccttt ctcgccacgt tcgccggctt tccccgtcaa gctctaaatc gggggctccc   1800
   tttagggttc cgatttagtg ctttacggca cctcgacccc aaaaaacttg attagggtga   1860
   tggttcacgt agtgggccat cgccctgata gacggttttt cgccctttga cgttggagtc   1920
   cacgttcttt aatagtggac tcttgttcca aactggaaca acactcaacc ctatctcggt   1980
   ctattctttt gatttataag ggattttgcc gatttcggcc tattggttaa aaaatgagct   2040
   gatttaacaa aaatttaacg cgaattttaa caaaatatta acgcttacaa tttcctgatg   2100
   cggtattttc tccttacgca tctgtgcggt atttcacacc gcatacgcgg atctgcgcag   2160
   caccatggcc tgaaataacc tctgaaagag gaacttggtt aggtaccttc tgaggcggaa   2220
   agaaccagct gtggaatgtg tgtcagttag ggtgtggaaa gtccccaggc tccccagcag   2280
   gcagaagtat gcaaagcatg catctcaatt agtcagcaac caggtgtgga aagtccccag   2340
   gctccccagc aggcagaagt atgcaaagca tgcatctcaa ttagtcagca accatagtcc   2400
   cgcccctaac tccgcccatc ccgcccctaa ctccgcccag ttccgcccat tctccgcccc   2460
   atggctgact aatttttttt atttatgcag aggccgaggc cgcctcggcc tctgagctat   2520
   tccagaagta gtgaggaggc ttttttggag gcctaggctt ttgcaaaaag cttgattctt   2580
   ctgacacaac agtctcgaac ttaaggctag agccaccatg attgaacaag atggattgca   2640
   cgcaggttct ccggccgctt gggtggagag gctattcggc tatgactggg cacaacagac   2700
   aatcggctgc tctgatgccg ccgtgttccg gctgtcagcg caggggcgcc cggttctttt   2760
   tgtcaagacc gacctgtccg gtgccctgaa tgaactgcag gacgaggcag cgcggctatc   2820
   gtggctggcc acgacgggcg ttccttgcgc agctgtgctc gacgttgtca ctgaagcggg   2880
   aagggactgg ctgctattgg gcgaagtgcc ggggcaggat ctcctgtcat ctcaccttgc   2940
   tcctgccgag aaagtatcca tcatggctga tgcaatgcgg cggctgcata cgcttgatcc   3000
   ggctacctgc ccattcgacc accaagcgaa acatcgcatc gagcgagcac gtactcggat   3060
   ggaagccggt cttgtcgatc aggatgatct ggacgaagag catcaggggc tcgcgccagc   3120
   cgaactgttc gccaggctca aggcgcgcat gcccgacggc gaggatctcg tcgtgaccca   3180
   tggcgatgcc tgcttgccga atatcatggt ggaaaatggc cgcttttctg gattcatcga   3240
   ctgtggccgg ctgggtgtgg cggaccgcta tcaggacata gcgttggcta cccgtgatat   3300
   tgctgaagag cttggcggcg aatgggctga ccgcttcctc gtgctttacg gtatcgccgc   3360
   tcccgattcg cagcgcatcg ccttctatcg ccttcttgac gagttcttct gagcgggact   3420
   ctggggttcg aaatgaccga ccaagcgacg cccaacctgc catcacgatg gccgcaataa   3480
   aatatcttta ttttcattac atctgtgtgt tggttttttg tgtgaatcga tagcgataag   3540
   gatccgcgta tggtgcactc tcagtacaat ctgctctgat gccgcatagt taagccagcc   3600
   ccgacacccg ccaacacccg ctgacgcgcc ctgacgggct tgtctgctcc cggcatccgc   3660
   ttacagacaa gctgtgaccg tctccgggag ctgcatgtgt cagaggtttt caccgtcatc   3720
   accgaaacgc gcgagacgaa agggcctcgt gatacgccta tttttatagg ttaatgtcat   3780
   gataataatg gtttcttaga cgtcaggtgg cacttttcgg ggaaatgtgc gcggaacccc   3840
   tatttgttta tttttctaaa tacattcaaa tatgtatccg ctcatgagac aataaccctg   3900
   ataaatgctt caataatatt gaaaaaggaa gagtatgagt attcaacatt tccgtgtcgc   3960
   ccttattccc ttttttgcgg cattttgcct tcctgttttt gctcacccag aaacgctggt   4020
   gaaagtaaaa gatgctgaag atcagttggg tgcacgagtg ggttacatcg aactggatct   4080
   caacagcggt aagatccttg agagttttcg ccccgaagaa cgttttccaa tgatgagcac   4140
   ttttaaagtt ctgctatgtg gcgcggtatt atcccgtatt gacgccgggc aagagcaact   4200
   cggtcgccgc atacactatt ctcagaatga cttggttgag tactcaccag tcacagaaaa   4260
   gcatcttacg gatggcatga cagtaagaga attatgcagt gctgccataa ccatgagtga   4320
   taacactgcg gccaacttac ttctgacaac gatcggagga ccgaaggagc taaccgcttt   4380
   tttgcacaac atgggggatc atgtaactcg ccttgatcgt tgggaaccgg agctgaatga   4440
   agccatacca aacgacgagc gtgacaccac gatgcctgta gcaatggcaa caacgttgcg   4500
   caaactatta actggcgaac tacttactct agcttcccgg caacaattaa tagactggat   4560
   ggaggcggat aaagttgcag gaccacttct gcgctcggcc cttccggctg gctggtttat   4620
   tgctgataaa tctggagccg gtgagcgtgg gtctcgcggt atcattgcag cactggggcc   4680
   agatggtaag ccctcccgta tcgtagttat ctacacgacg gggagtcagg caactatgga   4740
   tgaacgaaat agacagatcg ctgagatagg tgcctcactg attaagcatt ggtaactgtc   4800
   agaccaagtt tactcatata tactttagat tgatttaaaa cttcattttt aatttaaaag   4860
   gatctaggtg aagatccttt ttgataatct catgaccaaa atcccttaac gtgagttttc   4920
   gttccactga gcgtcagacc ccgtagaaaa gatcaaagga tcttcttgag atcctttttt   4980
   tctgcgcgta atctgctgct tgcaaacaaa aaaaccaccg ctaccagcgg tggtttgttt   5040
   gccggatcaa gagctaccaa ctctttttcc gaaggtaact ggcttcagca gagcgcagat   5100
   accaaatact gttcttctag tgtagccgta gttaggccac cacttcaaga actctgtagc   5160
   accgcctaca tacctcgctc tgctaatcct gttaccagtg gctgctgcca gtggcgataa   5220
   gtcgtgtctt accgggttgg actcaagacg atagttaccg gataaggcgc agcggtcggg   5280
   ctgaacgggg ggttcgtgca cacagcccag cttggagcga acgacctaca ccgaactgag   5340
   atacctacag cgtgagctat gagaaagcgc cacgcttccc gaagggagaa aggcggacag   5400
   gtatccggta agcggcaggg tcggaacagg agagcgcacg agggagcttc cagggggaaa  5460
   cgcctggtat ctttatagtc ctgtcgggtt tcgccacctc tgacttgagc gtcgattttt   5520
   gtgatgctcg tcaggggggc ggagcctatg gaaaaacgcc agcaacgcgg cctttttacg   5580
   gttcctggcc ttttgctggc cttttgctca catggctcga cagatct                 5627
<210> 19
   <211> 29
   <212> DNA
   <213> Homo sapiens
<220>
   <221> misc_feature
   <223> amorce sens: GV1-1
<400> 19
   cgggtcgaca ggtccactgc agggggtta 29
<210> 20
   <211> 31
   <212> DNA
   <213> Homo sapiens
<220>
   <221> misc_feature
   <223> amorce inverse: GV1-2
<400> 20
   cgcacgcgtt tcccctttgg aagagagagc a 31
<210> 21
   <211> 29
   <212> DNA
   <213> Homo sapiens
<220>
   <221> misc_feature
   <223> amorce sens: GV2-1
<400> 21
   cgggtcgacg ggccagctta ctgttggtg 29
<210> 22
   <211> 32
   <212> DNA
   <213> Homo sapiens
<220>
   <221> misc_feature
   <223> amorce inverse: GV2-2
<400> 22
   cgcacgcgtt tcagaggagg ggagagaaag ag 32
<210> 23
   <211> 31
   <212> DNA
   <213> Homo sapiens
<220>
   <221> misc_feature
   <223> amorce sens: GV3-1
<400> 23
   cgggtcgact ggaaccccca taatgacatc c 31
<210> 24
   <211> 31
   <212> DNA
   <213> Homo sapiens
<220>
   <221> misc_feature
   <223> amorce inverse: GV3-2
<400> 24
   cgcacgcgtt gcacgtcagt gtcctcagtt g 31
<210> 25
   <211> 30
   <212> DNA
   <213> Homo sapiens
<220>
   <221> misc_feature
   <223> amorce sens: GV4-1
<400> 25
   cgggtcgact ggagaacaac gaacgggtgt 30
<210> 26
   <211> 29
   <212> DNA
   <213> Homo sapiens
<220>
   <221> misc_feature
   <223> amorce inverse: GV4-2
<400> 26
   cgcacgcgtc aggcgggaaa cagagtgga 29
<210> 27
   <211> 5975
   <212> DNA
   <213> Homo sapiens
<300>
   <308> GenBank/XM_040709
   <309> 2002-02-07
   <313> (1)..(5975)
<400> 27
   gctctttgcc gagggcgtgt ggtgagagtc cccacagcga ccctggttcg agtggtgggc 60
   actgagctgg tcatcccctg caacgtcagt gactatgatg gccccagcga gcaaaacttt 120
   gactggagct tctcatcttt ggggagcagc tttgtggagc ttgcaagcac ctgggaggtg 180
   gggttcccag cccagctgta ccaggagcgg ctgcagaggg gcgagatcct gttaaggcgg 240
   actgccaacg acgccgtgga gctccacata aagaacgtcc agccttcaga ccaaggccac 300
   tacaaatgtt caacccccag cacagatgcc actgtccagg gaaactatga ggacacagtg 360
   caggttaaag tgctggccga ctccctgcac gtgggcccca gcgcgcggcc cccgccgagc 420
   ctgagcctgc gggaggggga gcccttcgag ctgcgctgca ccgccgcctc cgcctcgccg 480
   ctgcacacgc acctggcgct gctgtgggag gtgcaccgcg gcccggccag gcggagcgtc 540
   ctcgccctga cccacgaggg caggttccac ccgggcctgg ggtacgagca gcgctaccac 600
   agtggggacg tgcgcctcga caccgtgggc agcgacgcct accgcctctc agtgtcccgg 660
   gctctgtctg ccgaccaggg ctcctacagg tgtatcgtca gcgagtggat cgccgagcag 720
   ggcaactggc aggaaatcca agaaaaggcc gtggaagttg ccaccgtggt gatccagcca 780
   tcagttctgc gagcagctgt gcccaagaat gtgtctgtgg ctgaaggaaa ggaactggac 840
   ctgacctgta acatcacaac agaccgagcc gatgacgtcc ggcccgaggt gacgtggtcc 900
   ttcagcagga tgcctgacag caccctacct ggctcccgcg tgttggcgcg gcttgaccgt 960
   gattccctgg tgcacagctc gcctcatgtt gctttgagtc atgtggatgc acgctcctac 1020
   catttactgg ttcgggatgt tagcaaagaa aactctggct actattactg ccacgtgtcc 1080
   ctgtgggcac ccggacacaa caggagctgg cacaaagtgg cagaggccgt gtcttcccca 1140
   gctggtgtgg gtgtgacctg gctagaacca gactaccagg tgtacctgaa tgcttccaag 1200
   gtccccgggt ttgcggatga ccccacagag ctggcatgcc gggtggtgga cacgaagagt 1260
   ggggaggcga atgtccgatt cacggtttcg tggtactaca ggatgaaccg gcgcagcgac 1320
   aatgtggtga ccagcgagct gcttgcagtc atggacgggg actggacgct aaaatatgga 1380
   gagaggagca agcagcgggc ccaggatgga gactttattt tttctaagga acatacagac 1440
   acgttcaatt tccggatcca aaggactaca gaggaagaca gaggcaatta ttactgtgtt 1500
   gtgtctgcct ggaccaaaca gcggaacaac agctgggtga aaagcaagga tgtcttctcc 1560
   aagcctgtta acatattttg ggcattagaa gattccgtgc ttgtggtgaa ggcgaggcag 1620
   ccaaagcctt tctttgctgc cggaaataca tttgagatga cttgcaaagt atcttccaag 1680
   aatattaagt cgccacgcta ctctgttctc atcatggctg agaagcctgt cggcgacctc 1740
   tccagtccca atgaaacgaa gtacatcatc tctctggacc aggattctgt ggtgaagctg 1800
   gagaattgga cagatgcatc acgggtggat ggcgttgttt tagaaaaagt gcaggaggat 1860
   gagttccgct atcgaatgta ccagactcag gtctcagacg cagggctgta ccgctgcatg 1920
   gtgacagcct ggtctcctgt caggggcagc ctttggcgag aagcagcaac cagtctctcc 1980
   aatcctattg agatagactt ccaaacctca ggtcctatat ttaatgcttc tgtgcattca 2040
   gacacaccat cagtaattcg gggagatctg atcaaattgt tctgtatcat cactgtcgag 2100
   ggagcagcac tggatccaga tgacatggcc tttgatgtgt cctggtttgc ggtgcactct 2160
   tttggcctgg acaaggctcc tgtgctcctg tcttccctgg atcggaaggg catcgtgacc 2220
   acctcccgga gggactggaa gagcgacctc agcctggagc gcgtgagtgt gctggaattc 2280
   ttgctgcaag tgcatggctc cgaggaccag gactttggca actactactg ttccgtgact 2340
   ccatgggtga agtcaccaac aggttcctgg cagaaggagg cagagatcca ctccaagccc 2400
   gtttttataa ctgtgaagat ggatgtgctg aacgccttca agtatccctt gctgatcggc 2460
   gtcggtctgt ccacggtcat cgggctcctg tcctgtctca tcgggtactg cagctcccac 2520
   tggtgttgta agaaggaggt tcaggagaca cggcgcgagc gccgcaggct catgtcgatg 2580
   gagatggact aggctggccc gggaggggag tgacagaggg acgttctagg agcaattggg 2640
   gcaagaagag gacagtgata ttttaaaaca aagtgtgtta cactaaaaac cagtcctctc 2700
   taatctcagg tgggacttgg cgctctctct tttctgcatg tcaagttctg agcgcggaca 2760
   tgtttaccag cacacggctc ttcttcccac ggcactttct gatgtaacaa tcgagtgtgt 2820
   gttttcccaa ctgcagcttt ttaatggtta accttcatct aatttttttt ctcccactgg 2880
   tttatagatc ctctgacttg tgtgtgttta tagcttttgt ttcgcggggt tgtggtgagg 2940
   aaggggtgat ggcatgcgga gttctttatc ttcagtgaga atgtgcctgc ccgcctgaga 3000
   gccagcttcc gcgttggagg cacgtgttca gagagctgct gagcgccacc ctctacccgg 3060
   ctgacagaca acacagacct gtgccgaagg ctaatttgtg gcttttacga ccctacccca 3120
   ccccctgttt tcaggggttt agactacatt tgaaatccaa acttggagta tataacttct 3180
   tattgagccc aactgctttt tttttttttt tttttgcttc tctgcccctt ttccatttct 3240
   tttgtatttg ttttctgtga gagcactgaa atggcagccc tggaatctac aatttggctc 3300
   tccactgagc accttatctt gccaccttag ccttaagaat gaatatgaag aaaaatacac 3360
   agccacctct gtccagggca gtaagaaggg ctgcaaggaa ggggaggatg gggacaagga 3420
   aaggatcaga tacctgctcc agtagttgtg aggccactgt gtctcagggg actccaggag 3480
   gagcagaaga gggatcccac gaagttattc ttacgcagct ggggccagga gggtcagagt 3540
   ggtgccaggt gcaagttagg ctaaagaagc caccactatt cctctctctt gcccattgtg 3600
   gggggcaaag gcattggtca ccaagagtct tgcaggggga cccacagata tgccatgtcc 3660
   ttcacacgtg cttgggctcc ttaacctgaa ggcaaattgc tacttgcaag actgactgac   3720
   ttcaaggaat cagaaattac ctagaagcac catgtttttt ctatgacctt ttcagtcctt   3780
   caggtcattt taaggtccac tgcagggggt tagtgagaaa gggtatactt tgtggtatgt   3840
   tttgctttcc taatagggac atgaaggaaa cccagcaatt tgctgttatg tgaatggcct   3900
   gtagagcaga gtcaagagcg gtgtgctttg cccgactgct cccatcagga ataggagagt   3960
   agacagagat cttccacatc ccaggcttct gctgctgctt taaaagctct gtccttggag   4020
   cctcccgctc cctgaagtgt ctcgccccct gcacagcact ggcctttcgg aagcatccca   4080
   gtagggtttt ctgaggctcg ctggtgactc atgccctaat tgcaatcctc tgcttttatc   4140
   ttgactttga aggatctaac actgctctct cttccaaagg ggaaaaaaag attcatttgt   4200
   tttgagcaat aaactaatac aaaatgatgg ccattcatgt gcagctcttt gtcaccatgg   4260
   gccggatgag ttgtgctcct cctggctcac catttccccc tgctccccca cagccggttc   4320
   tgcacttatc accgagtcgc ccctggaagc agattcccat tgagttttcc ccaccaaggg   4380
   gaccatgcac atggtagaaa cattagattc tgcattgaca gtagcctttc cttggcccgg   4440
   gcctgtggtg ggaagacggg caacaagtat accccaccag ggcctgagtg actagaggaa   4500
   gaggacgagg ccttgttggc actagatttg ggtattttct gcatgtcata acatatccta   4560
   actgctattt cagaagaggc agcttgtagg tgattgtaca agtgagaatt aaagagagaa   4620
   cagatattta aacaggtgct gtattagtaa cagccagtgc cctttcagcc cttgcatcta   4680
   ttaaaaggag attcaggatt ttattggcac aggcccttct tagtaggaag aaagggtgct   4740
   tagctttgga cctgaccggg tgtgtgtaaa accatggact gagtcacagc agacactcga   4800
   tggtggtaaa tgtgatgggt gcttacacac tgtacctttt cctttcatac tgatgctgca   4860
   gttcagggct ggagttgtta aggcattgac ctccacccac ctgccccatg tccactgggc   4920
   tgcccaagct gcatgtcacc tgagggctgg caggaagggg cgagaaatcc cagggcattg   4980
   taccaaggac ctagttcctt ctagggatat aaatttccag gaatgtgtat ttttaatgtg   5040
   gtgagatgca ctcttttgtt gtaccaaata gggctcccca ccccacccct gcgacaagtg   5100
   ctcttctaga acaggttcct accagcagca ctggtgtgaa tgaaagagag acccagccgc   5160
   gtctcacaca ggtggaattg cacttcttaa caaaaaggaa ctttataaaa gtttgggatt   5220
   ttttttccta atcataaaaa tagccccaga aagagcctaa gctatgttca gatagaagcc   5280
   tcgaaattcc tgtaaattgt ttactttatg atgtttacat acacgtttca ctttgaaaaa   5340
   aaatgcaaat cgacttttta acaactgttg agatgtttca tgggacagta gaactctgac   5400
   tcaccaactg ggctaaattt taatttaaaa atgtatttat ttgagtgtct ttccccccct   5460
   caccctcacc atctgagggg ctccctgaga tcttggtaga ggaggcccct cctgcccaga   5520
   ccttcgtttg tttccccggt ggcccttgct tcttgctttg cagactgcct gcagccatga   5580
   ttttgtcact gacatctgtg agccaaagac tgagcctttt tggcaggaat aataagcaat   5640
   actacacaac ttgctacttt cagaaaactt ttttttagct tcaccgatga caacagagga   5700
   agaagggaac tgggatttgg gtaagttctc ctccactgtt tgaccaaatt ctcagtgata   5760
   aatatgtgtg cagatcccta gaagagaaaa cgctgacttt ctttttaagt gtggcacata   5820
   aggatctgca gaattttccg tagacaaaga aaggatcttg tgtatttttg tccatatcca   5880
   atgttatatg aactaattgt attgttttat actgtgacca caaatattat gcaatgcacc   5940
   atttgttttt tatttcatta aaggaagttt aattt                              5975
<210> 28
   <211> 2197
   <212> DNA
   <213> Homo sapiens
<220>
   <221> misc_feature
   <222> (1037)..(1037)
   <223> n est un résidu qui peut être choisi parmi les nucléotides a, t, c ou g
<220>
   <221> misc_feature
   <222> (1236)..(1236)
   <223> n est un résidu qui peut être choisi parmi les nucléotides a,t, c ou g.
<300>
   <308> GenBank/AB014734
   <309> 2001-01-06
   <313> (1)..(2197)
<400> 28
   gtgcattcag acacaccatc agtaattcgg ggagatctga tcaaattgtt ctgtatcatc      60
   actgtcgagg gagcagcact ggatccagat gacatggcct ttgatgtgtc ctggtttgcg     120
   gtgcactctt ttggcctgga caaggctcct gtgctcctgt cttccctgga tcggaagggc     180
   atcgtgacca cctcccggag ggactggaag agcgacctca gcctggagcg cgtgagtgtg     240
   ctggaattct tgctgcaagt gcatggctcc gaggaccagg actttggcaa ctactactgt     300
   tccgtgactc catgggtgaa gtcaccaaca ggttcctggc agaaggaggc agagatccac     360
   tccaagcccg tttttataac tgtgaagatg gatgtgctga acgccttcaa gtatcccttg     420
   ctgatcggcg tcggtctgtc cacggtcatc gggctcctgt cctgtctcat cgggtactgc     480
   agctcccact ggtgttgtaa gaaggaggtt caggagacac ggcgcgagcg ccgcaggctc     540
   atgtcgatgg agatggacta ggctggcccg ggaggggagt gacagaggga cgttctagga     600
   gcaattgggg caagaagagg acagtgatat tttaaaacaa agtgtgttac actaaaaacc     660
   agtcctctct aatctcaggt gggacttggc gctctctctt ttctgcatgt caagttctga     720
   gcgcggacat gtttaccagc acacggctct tcttcccacg gcactttctg atgtaacaat     780
   cgagtgtgtg ttttcccaac tgcagctttt taatggttaa ccttcatcta attttttttc     840
   tcccactggt ttatagatcc tctgacttgt gtgtgtttat agcttttgtt tcgcggggtt     900
   gtggtgagga aggggtgatg gcatgcggag ttctttatct tcagtgagaa tgtgcctgcc     960
   cgcctgagag ccagcttccg cgttggaggc acgtgttcag agagctgctg agcgccaccc    1020
   tctacccggc tgacagncaa cacagacctg tgccgaaggc taatttgtgg cttttacgac    1080
   cctaccccac cccctgtttt caggggttta gactacattt gaaatccaaa cttggagtat    1140
   ataacttctt attgagccca actgcttttt tttttttttt ttttttgctt ctctgcccct    1200
   tttccatttc ttttgtattt gttttctgtg agagcnctga aatggcagcc ctggaatcta    1260
   caatttggct ctccactgag caccttatct tgccacctta gccttaagaa tgaatatgaa    1320
   gaaaaataca cagccacctc tgtccagggc agtaagaagg gctgcaagga aggggaggat    1380
   ggggacaagg aaaggatcag atacctgctc cagtagttgt gaggccactg tgtctcaggg    1440
   gactccagga ggagcagaag agggatccca cgaagttatt cttacgcagc tggggccagg    1500
   agggtcagag tggtgccagg tgcaagttag gctaaagaag ccaccactat tcctctctct    1560
   tgcccattgt ggggggcaaa ggcattggtc accaagagtc ttgcaggggg acccacagat    1620
   atgccatgtc cttcacacgt gcttgggctc cttaacctga aggcaaattg ctacttgcaa    1680
   gactgactga cttcaaggaa tcagaaatta cctagaagca ccatgttttt tctatgacct    1740
   tttcagtcct tcaggtcatt ttaaggtcca ctgcaggggg ttagtgagaa agggtatact    1800
   ttgtggtatg ttttgctttc ctaataggga catgaaggaa acccagcaat ttgctgttat    1860
   gtgaatggcc tgtagagcag agtcaagagc ggtgtgcttt gcccgactgc tcccatcagg    1920
   aataggagag tagacagaga tcttccacat cccaggcttc tgctgctgct ttaaaagctc    1980
   tgtccttgga gcctcccgct ccctgaagtg tctcgccccc tgcacagcac tggcctttcg    2040
   gaagcatccc agtagggttt tctgaggctc gctggtgact catgccctaa ttgcaatcct    2100
   ctgcttttat cttgactttg aaggatctaa cactgctctc tcttccaaag gggaaaaaaa    2160
   gattcatttg ttttgagcaa taaactaata caaaatg                              2197
<210> 29
   <211> 6331
   <212> DNA
   <213> Homo sapiens
<300>
   <308> GenBank/AF006515
   <309> 1997-11-27
   <313> (1)..(6331)
<400> 29
   atctttgttt gggtctccca tactgcgtat agatgaatgg gtcaggatat ctggaacaaa      60
   atatggaggt gaagggtgag atcgggaaac aaagggtatg gccccctagt tcccaaaggg     120
   agcagggaga tgggaataga attgaaggta ggttttaggc tacttgggag gaggaatatt     180
   taggtaattg tggagacttt ctcctgtgtg atgaaggcgg cagacactgt gatcctgtgg     240
   gcaagaagta aaaatgacca gctgaggatt tcttttcctc caggactgtg ttggggtgac     300
   aggatgcctg ataaggatga cattcggctg ctgccgtcag cattgggtgt gaagaagaga     360
   aaacgaggac ccaagaagca gaaggagaac aagccaggaa aaccccgaaa acgcaagaag     420
   cgtgacagtg aggaggaatt tggttctgag cgagatgagt accgggagaa gtcagagagt     480
   gggggcagtg aatatggaac cggaccgggt cggaaacgaa gaaggaagca ccgagaaaaa     540
   aaggagaaga agacaaagcg gcggaaaaag ggggagggag atggggggca aaagcaagtg       600
   gaacagaagt catcagcaac tctgcttctg acctggggcc tggaggatgt ggagcatgtg     660
   ttctctgagg aggattacca cacgctcacc aactacaaag ccttcagcca gttcatgagg     720
   cccctaattg ctaagaagaa tcctaagatc ccaatgtcta agatgatgac catccttggg     780
   gccaaatgga gagagttcag tgccaacaac cccttcaagg ggtcagcagc tgctgtggcg     840
   gcggcagcgg cagcagcagc agcagctgta gctgagcagg tgtcagctgc tgtctcgtcg     900
   gccaccccca tagcaccctc cggacccccc gcccttccac caccccctgc tgctgatatc         960
   cagcccccac ccatccgaag agccaaaacc aaagagggca aaggtccagg ccataagagg    1020
   cggagtaaga gcccccgagt gcctgatgga cgcaagaagc ttcggggaaa gaaaatggca    1080
   ccactcaaaa taaaactagg gcttctgggt ggcaagagga agaaaggagg ctcgtatgtt    1140
   tttcagagcg acgaaggtcc tgaaccagag gctgaggaat cagacctgga cagtggcagt    1200
   gtccacagtg cctcaggccg gcctgatggc cctgtccgca ccaagaaact aaagagaggc    1260
   cggccaggaa ggaagaagaa gaaggtcctg ggctgtcctg cagtggccgg ggaggaggag    1320
   gttgatggct acgagacgga tcaccaggat tactgtgagg tgtgccagca gggtggggaa    1380
   attattctgt gtgacacctg ccctcgtgcc taccacctcg tctgccttga tcctgagctt    1440
   gaccgggctc cagagggcaa atggagctgc cctcactgtg agaaggaggg ggtccagtgg    1500
   gaggccaagg aggaagaaga agaatacgaa gaggagggag aggaagaagg ggagaaggag    1560
   gaggaggatg atcacatgga gtactgccgc gtatgcaagg acggcgggga gctcctgtgc 1620
   tgtgacgcgt gcatctcctc ctaccacatt cattgtctaa accctcccct gcctgacatt 1680
   cccaatggtg aatggctgtg tccccgatgc acatgccccg tgctgaaggg tcgagtgcag 1740
   aagatcctac attggcggtg gggggagcca cctgtagcag tgccagcccc tcaacaggca 1800
   gatggaaatc cagatgtccc acccccccgt cctcttcaag gcagatcaga gcgagagttc 1860
   tttgtcaagt gggtaggact atcctactgg cactgctcct gggccaagga gcttcagctg 1920
   gaaatcttcc atttggttat gtatcgaaac taccagcgga agaatgacat ggatgagccc 1980
   ccacccctgg actatggctc cggcgaggat gatgggaaga gcgacaagcg taaagtgaaa 2040
   gacccgcact atgctgagat ggaggagaag tactatcgtt ttggcatcaa gccagagtgg 2100
   atgaccgtcc accgcatcat caaccacagt gtggataaaa aggggaatta ccactatcta 2160
   gtaaaatgga gggacttacc atatgaccag tccacgtggg aggaagatga aatgaatatc 2220
   cctgaatacg aagaacataa gcaaagctac tggagacacc gagaactaat tatgggggaa 2280
   gaccctgccc agccccgcaa gtataagaag aagaagaagg agctacaggg tgatgggcct 2340
   cccagttctc ccactaatga tcctaccgtg aaatatgaga ctcagccacg gtttatcaca 2400
   gccactggag gcaccctgca catgtatcag ttggaagggc tgaactggct acgcttctcc 2460
   tgggcccagg gcactgacac cattctagct gatgagatgg ggctaggcaa gaccatacaa 2520
   accatcgtct tcctctactc actctacaag gagggccaca caaaaggtcc cttcctggtg 2580
   agtgccccac tctctaccat cattaactgg gagcgggagt tccagatgtg ggcacccaaa 2640
   ttctatgtgg tgacatacac gggtgacaag gacagccggg ccatcattcg tgagaatgaa 2700
   ttctcctttg aggacaatgc catcaaaggg ggcaagaaag cttttaagat gaaaagggag 2760
   gcacaggtga agttccatgt tctcctgaca tcgtatgagc tgatcaccat tgatcaggca 2820
   gcacttggtt ccatccgctg ggcctgtctt gtggtagatg aggcccatcg actcaagaac 2880
   aaccagtcca agtttttcag ggttctcaat ggttacaaga tagatcataa gttgctgctg 2940
   acaggaaccc cattgcagaa taatctggag gagctcttcc atctcctgaa cttcctcacc 3000
   ccagagagat ttaacaactt ggagggcttc ctggaggagt ttgctgacat atccaaagag 3060
   gaccagatca agaaactgca tgatttgctg gggccacaca tgctgcggag actcaaggca 3120
   gatgtcttta agaacatgcc agccaagaca gagctcatcg ttcgggtgga gctaagcccc 3180
   atgcagaaga aatactacaa atacatcctg actcgaaatt ttgaggcctt gaattcacga 3240
   ggtggtggga accaggtgtc gctgcttaat atcatgatgg atcttaagaa gtgctgcaac 3300
   catccatacc tttttcccgt ggctgctatg gagtccccca aactccccag tggggcttat 3360
   gagggtgggg cacttattaa gtcgtctggg aagctcatgc tgctccagaa gatgctgcga 3420
   aagctgaagg agcaaggaca ccgagtgctc atcttctcgc agatgaccaa aatgttagac 3480
   ttgcttgagg acttcttaga ctatgaaggc tacaagtatg agcgcatcga tggtggtatc 3540
   acgggtgccc tgaggcagga ggccatcgat cggtttaatg ctcctggggc ccaacaattc 3600
   tgcttcctcc tgtccacccg agctgggggc ctgggcatca atctggccac tgctgacact 3660
   gtcatcatct ttgattctga ctggaacccc cataatgaca tccaggcctt tagccgggct 3720
   catcggattg gccaggccaa caaagtgatg atttaccggt ttgtgactcg cgcgtcagtg 3780
   gaagagcgaa tcacacaagt ggccaagaga aagatgatgc tgacacacct ggttgtgcgg 3840
   cctgggctgg gctccaaggc aggctccatg tccaagcagg agcttgacga cattctcaaa 3900
   tttggcactg aagagctatt caaggatgaa aacgaggggg agaacaagga ggaggacagc 3960
   agtgtgattc attatgacaa tgaggccatc gctcggctgt tggaccggaa ccaggatgca 4020
   actgaggaca ctgacgtgca gaacatgaat gagtatctca gctccttcaa ggtggcacag 4080
   tacgtcgtgc gggaagaaga caagattgag gaaattgagc gagagatcat caagcaggag 4140
   gagaatgtgg accctgacta ctgggagaag ctgctgaggc atcactatga gcaacagcag 4200
   gaagacctag cccggaatct aggcaagggc aagcgggttc gcaagcaagt taactacaat 4260
   gatgctgctc aggaagacca agacaaccag tcagagtact cggtgggttc agaggaggag 4320
   gatgaagact tcgatgaacg tcctgaaggg cgtagacagt caaagaggca gctccggaat 4380
   gagaaagata agccactgcc tccactgctg gcccgagtcg ggggcaacat tgaggtgctg 4440
   ggcttcaaca cccgtcagcg gaaggctttc ctcaatgctg tgatgcgctg ggggatgcca 4500
   ccacaggatg ccttcaccac acagtggctg gtgcgggacc tgaggggcaa gactgagaag 4560
   gagtttaagg cctatgtgtc tttgttcatg cgccatctgt gtgagcctgg ggcagacggc 4620
   tctgaaacct ttgccgatgg ggtccctcgg gagggactga gtcgccagca ggtgttgacc 4680
   cgcattggag tcatgtctct cgtcaaaaag aaggtgcagg agtttgagca catcaatggg 4740
   cgttggtcaa tgccggaact gatgcctgac cccagcgccg attctaagcg ctcctccaga 4800
   gcctcctctc ctaccaaaac gtctcccacc actcctgagg cttctgctac caacagtccc 4860
   tgcacctcta aacctgctac tccagctcca agtgagaaag gagaaggcat aaggacacct 4920
   cttgagaagg aggaagctga aaaccaggag gaaaagccag agaagaacag cagaattggg 4980
   gagaagatgg agacagaggc tgatgccccc agcccagccc catcacttgg ggagcggctg 5040
   gagccaagga agattcctct agaggatgag gtgccagggg tgcctggaga gatggagcct 5100
   gaacctgggt accgtgggga cagagagaag tcagaagatg taaaaggtga ccgggagctt 5160
   cgaccagggc ctcgagatga gccacggtcc aatgggcgac gagaggaaaa gacagagaag 5220
   ccccggttca tgttcaatat cgccgatggt ggcttcacag agcttcacac actgtggcag 5280
   aatgaggaac gggcagctat ttcctcgggg aaactcaatg agatctggca cagaagacat 5340
   gactattggc ttctggctgg gattgtcctc catggctatg cacggtggca ggacatccag 5400
   aatgatgctc aatttgccat tatcaacgag ccatttaaaa ctgaagccaa taaggggaac 5460
   tttctggaga tgaaaaataa gttcctggcc cggaggttca agctcctgga gcaggcgctg 5520
   gtgattgagg agcagctgcg gcgggcggcc tacctgaacc tgtcgcagga gccggcgcac 5580
   cccgccatgg ccctccacgc ccgcttcgcc gaggccgagt gcctggccga gagccaccag   5640
   cacctctcca aggagtcgct ggcggggaac aagccggcca acgccgtcct gcacaaggtt   5700
   ctgaaccagc tggaggagtt gctgagcgac atgaaggcgg acgtgacccg cctgccagcc   5760
   acgctgtccc gaataccccc catcgcagcc cgccttcaga tgtccgagcg cagcatcctc   5820
   agccggctgg ccagcaaggg cacggagcct caccccacac cggcctaccc gccgggtccc   5880
   tacgctacac ctccggggta cggggcggcc ttcagcgccg cacccgtagg ggccctggcc   5940
   gccgcaggcg ccaattacag ccagatgcct gcagggtcct tcatcacagc cgccaccaac   6000
   ggcccaattc accgattttt taaaaaagtt ccagaaatcc agtgacgaat gtggtataca   6060
   aaaaaatata taaattcttt caacttagaa taattaagtc ataaaataca tagggtacaa   6120
   ataccacatt ccgttctaaa atgatatctt aggatcatca aaagaaaaag aggatttgga   6180
   ttatgcaaaa aatgattcct atatatataa tcaattatct aactgacatt tttgcaaatc   6240
   taccacaact tcgcctttta ttgcatatgc taaacaagca gatgctaagt ctgtaaactg   6300
   tgaattaacc tcctttttaa ttaattgttc g                                 6331
<210> 30
   <211> 6331
   <212> DNA
   <213> Homo sapiens
<300>
   <308> GenBank/NM_001272
   <309> 2001-02-03
   <313> (1)..(6331)
<400> 30
   atctttgttt gggtctccca tactgcgtat agatgaatgg gtcaggatat ctggaacaaa     60
   atatggaggt gaagggtgag atcgggaaac aaagggtatg gccccctagt tcccaaaggg    120
   agcagggaga tgggaataga attgaaggta ggttttaggc tacttgggag gaggaatatt    180
   taggtaattg tggagacttt ctcctgtgtg atgaaggcgg cagacactgt gatcctgtgg    240
   gcaagaagta aaaatgacca gctgaggatt tcttttcctc caggactgtg ttggggtgac    300
   aggatgcctg ataaggatga cattcggctg ctgccgtcag cattgggtgt gaagaagaga    360
   aaacgaggac ccaagaagca gaaggagaac aagccaggaa aaccccgaaa acgcaagaag    420
   cgtgacagtg aggaggaatt tggttctgag cgagatgagt accgggagaa gtcagagagt    480
   gggggcagtg aatatggaac cggaccgggt cggaaacgaa gaaggaagca ccgagaaaaa    540
   aaggagaaga agacaaagcg gcggaaaaag ggggagggag atggggggca aaagcaagtg      600
   gaacagaagt catcagcaac tctgcttctg acctggggcc tggaggatgt ggagcatgtg    660
   ttctctgagg aggattacca cacgctcacc aactacaaag ccttcagcca gttcatgagg    720
   cccctaattg ctaagaagaa tcctaagatc ccaatgtcta agatgatgac catccttggg    780
   gccaaatgga gagagttcag tgccaacaac cccttcaagg ggtcagcagc tgctgtggcg    840
   gcggcagcgg cagcagcagc agcagctgta gctgagcagg tgtcagctgc tgtctcgtcg    900
   gccaccccca tagcaccctc cggacccccc gcccttccac caccccctgc tgctgatatc     960
   cagcccccac ccatccgaag agccaaaacc aaagagggca aaggtccagg ccataagagg   1020
   cggagtaaga gcccccgagt gcctgatgga cgcaagaagc ttcggggaaa gaaaatggca   1080
   ccactcaaaa taaaactagg gcttctgggt ggcaagagga agaaaggagg ctcgtatgtt   1140
   tttcagagcg acgaaggtcc tgaaccagag gctgaggaat cagacctgga cagtggcagt   1200
   gtccacagtg cctcaggccg gcctgatggc cctgtccgca ccaagaaact aaagagaggc   1260
   cggccaggaa ggaagaagaa gaaggtcctg ggctgtcctg cagtggccgg ggaggaggag   1320
   gttgatggct acgagacgga tcaccaggat tactgtgagg tgtgccagca gggtggggaa   1380
   attattctgt gtgacacctg ccctcgtgcc taccacctcg tctgccttga tcctgagctt   1440
   gaccgggctc cagagggcaa atggagctgc cctcactgtg agaaggaggg ggtccagtgg   1500
   gaggccaagg aggaagaaga agaatacgaa gaggagggag aggaagaagg ggagaaggag   1560
   gaggaggatg atcacatgga gtactgccgc gtatgcaagg acggcgggga gctcctgtgc   1620
   tgtgacgcgt gcatctcctc ctaccacatt cattgtctaa accctcccct gcctgacatt   1680
   cccaatggtg aatggctgtg tccccgatgc acatgccccg tgctgaaggg tcgagtgcag   1740
   aagatcctac attggcggtg gggggagcca cctgtagcag tgccagcccc tcaacaggca   1800
   gatggaaatc cagatgtccc acccccccgt cctcttcaag gcagatcaga gcgagagttc   1860
   tttgtcaagt gggtaggact atcctactgg cactgctcct gggccaagga gcttcagctg   1920
   gaaatcttcc atttggttat gtatcgaaac taccagcgga agaatgacat ggatgagccc   1980
   ccacccctgg actatggctc cggcgaggat gatgggaaga gcgacaagcg taaagtgaaa   2040
   gacccgcact atgctgagat ggaggagaag tactatcgtt ttggcatcaa gccagagtgg   2100
   atgaccgtcc accgcatcat caaccacagt gtggataaaa aggggaatta ccactatcta   2160
   gtaaaatgga gggacttacc atatgaccag tccacgtggg aggaagatga aatgaatatc   2220
   cctgaatacg aagaacataa gcaaagctac tggagacacc gagaactaat tatgggggaa   2280
   gaccctgccc agccccgcaa gtataagaag aagaagaagg agctacaggg tgatgggcct   2340
   cccagttctc ccactaatga tcctaccgtg aaatatgaga ctcagccacg gtttatcaca   2400
   gccactggag gcaccctgca catgtatcag ttggaagggc tgaactggct acgcttctcc   2460
   tgggcccagg gcactgacac cattctagct gatgagatgg ggctaggcaa gaccatacaa   2520
   accatcgtct tcctctactc actctacaag gagggccaca caaaaggtcc cttcctggtg   2580
   agtgccccac tctctaccat cattaactgg gagcgggagt tccagatgtg ggcacccaaa   2640
   ttctatgtgg tgacatacac gggtgacaag gacagccggg ccatcattcg tgagaatgaa 2700
   ttctcctttg aggacaatgc catcaaaggg ggcaagaaag cttttaagat gaaaagggag 2760
   gcacaggtga agttccatgt tctcctgaca tcgtatgagc tgatcaccat tgatcaggca 2820
   gcacttggtt ccatccgctg ggcctgtctt gtggtagatg aggcccatcg actcaagaac 2880
   aaccagtcca agtttttcag ggttctcaat ggttacaaga tagatcataa gttgctgctg 2940
   acaggaaccc cattgcagaa taatctggag gagctcttcc atctcctgaa cttcctcacc 3000
   ccagagagat ttaacaactt ggagggcttc ctggaggagt ttgctgacat atccaaagag 3060
   gaccagatca agaaactgca tgatttgctg ggccacacaa tgctgcggag actcaaggca 3120
   gatgtcttta agaacatgcc agccaagaca gagctcatcg ttcgggtgga gctaagcccc 3180
   atgcagaaga aatactacaa atacatcctg actcgaaatt ttgaggcctt gaattcacga 3240
   ggtggtggga accaggtgtc gctgcttaat atcatgatgg atcttaagaa gtgctgcaac 3300
   catccatacc tttttcccgt ggctgctatg gagtccccca aactccccag tggggcttat 3360
   gagggtgggg cacttattaa gtcgtctggg aagctcatgc tgctccagaa gatgctgcga 3420
   aagctgaagg agcaaggaca ccgagtgctc atcttctcgc agatgaccaa aatgttagac 3480
   ttgcttgagg acttcttaga ctatgaaggc tacaagtatg agcgcatcga tggtggtatc 3540
   acgggtgccc tgaggcagga ggccatcgat cggtttaatg ctcctggggc ccaacaattc 3600
   tgcttcctcc tgtccacccg agctgggggc ctgggcatca atctggccac tgctgacact 3660
   gtcatcatct ttgattctga ctggaacccc cataatgaca tccaggcctt tagccgggct 3720
   catcggattg gccaggccaa caaagtgatg atttaccggt ttgtgactcg cgcgtcagtg 3780
   gaagagcgaa tcacacaagt ggccaagaga aagatgatgc tgacacacct ggttgtgcgg 3840
   cctgggctgg gctccaaggc aggctccatg tccaagcagg agcttgacga cattctcaaa 3900
   tttggcactg aagagctatt caaggatgaa aacgaggggg agaacaagga ggaggacagc 3960
   agtgtgattc attatgacaa tgaggccatc gctcggctgt tggaccggaa ccaggatgca 4020
   actgaggaca ctgacgtgca gaacatgaat gagtatctca gctccttcaa ggtggcacag 4080
   tacgtcgtgc gggaagaaga caagattgag gaaattgagc gagagatcat caagcaggag 4140
   gagaatgtgg accctgacta ctgggagaag ctgctgaggc atcactatga gcaacagcag 4200
   gaagacctag cccggaatct aggcaagggc aagcgggttc gcaagcaagt taactacaat 4260
   gatgctgctc aggaagacca agacaaccag tcagagtact cggtgggttc agaggaggag 4320
   gatgaagact tcgatgaacg tcctgaaggg cgtagacagt caaagaggca gctccggaat 4380
   gagaaagata agccactgcc tccactgctg gcccgagtcg ggggcaacat tgaggtgctg 4440
   ggcttcaaca cccgtcagcg gaaggctttc ctcaatgctg tgatgcgctg ggggatgcca 4500
   ccacaggatg ccttcaccac acagtggctg gtgcgggacc tgaggggcaa gactgagaag 4560
   gagtttaagg cctatgtgtc tttgttcatg cgccatctgt gtgagcctgg ggcagacggc 4620
   tctgaaacct ttgccgatgg ggtccctcgg gagggactga gtcgccagca ggtgttgacc 4680
   cgcattggag tcatgtctct cgtcaaaaag aaggtgcagg agtttgagca catcaatggg 4740
   cgttggtcaa tgccggaact gatgcctgac cccagcgccg attctaagcg ctcctccaga 4800
   gcctcctctc ctaccaaaac gtctcccacc actcctgagg cttctgctac caacagtccc 4860
   tgcacctcta aacctgctac tccagctcca agtgagaaag gagaaggcat aaggacacct 4920
   cttgagaagg aggaagctga aaaccaggag gaaaagccag agaagaacag cagaattggg 4980
   gagaagatgg agacagaggc tgatgccccc agcccagccc catcacttgg ggagcggctg 5040
   gagccaagga agattcctct agaggatgag gtgccagggg tgcctggaga gatggagcct 5100
   gaacctgggt accgtgggga cagagagaag tcagaagatg taaaaggtga ccgggagctt 5160
   cgaccagggc ctcgagatga gccacggtcc aatgggcgac gagaggaaaa gacagagaag 5220
   ccccggttca tgttcaatat cgccgatggt ggcttcacag agcttcacac actgtggcag 5280
   aatgaggaac gggcagctat ttcctcgggg aaactcaatg agatctggca cagaagacat 5340
   gactattggc ttctggctgg gattgtcctc catggctatg cacggtggca ggacatccag 5400
   aatgatgctc aatttgccat tatcaacgag ccatttaaaa ctgaagccaa taaggggaac 5460
   tttctggaga tgaaaaataa gttcctggcc cggaggttca agctcctgga gcaggcgctg 5520
   gtgattgagg agcagctgcg gcgggcggcc tacctgaacc tgtcgcagga gccggcgcac 5580
   cccgccatgg ccctccacgc ccgcttcgcc gaggccgagt gcctggccga gagccaccag 5640
   cacctctcca aggagtcgct ggcggggaac aagccggcca acgccgtcct gcacaaggtt 5700
   ctgaaccagc tggaggagtt gctgagcgac atgaaggcgg acgtgacccg cctgccagcc 5760
   acgctgtccc gaataccccc catcgcagcc cgccttcaga tgtccgagcg cagcatcctc 5820
   agccggctgg ccagcaaggg cacggagcct caccccacac cggcctaccc gccgggtccc 5880
   tacgctacac ctccggggta cggggcggcc ttcagcgccg cacccgtagg ggccctggcc 5940
   gccgcaggcg ccaattacag ccagatgcct gcagggtcct tcatcacagc cgccaccaac 6000
   ggcccaattc accgattttt taaaaaagtt ccagaaatcc agtgacgaat gtggtataca 6060
   aaaaaatata taaattcttt caacttagaa taattaagtc ataaaataca tagggtacaa 6120
   ataccacatt ccgttctaaa atgatatctt aggatcatca aaagaaaaag aggatttgga 6180
   ttatgcaaaa aatgattcct atatatataa tcaattatct aactgacatt tttgcaaatc 6240
   taccacaact tcgcctttta ttgcatatgc taaacaagca gatgctaagt ctgtaaactg 6300
   tgaattaacc tcctttttaa ttaattgttc g 6331
<210> 31
   <211> 560
   <212> PRT
   <213> Homo sapiens
<220>
   <221> misc_feature
   <223> CDS de la seq ID 27, n° accession GenBank/XM_040709:910..2592
<220>
   <221> misc_feature
   <223> nom: régulateur négatif du recepteur de la prostaglandine F2 (PTG FRN)
<400> 31
<210> 32
   <211> 186
   <212> PRT
   <213> Homo sapiens
<220>
   <221> misc_feature
   <223> CDS partielle de la seq ID 28, n° accession GenBank/AB014734: <1.
   .561
<220>
   <221> misc_feature
   <223> nom: protéine 6 associée à la cellule du muscle lisse humaine (SM AP-6)
<400> 32
   Val His Ser Asp Thr Pro Ser Val Ile Arg Gly Asp Leu Ile Lys Leu
   1               5                   10                  15
   Phe Cys Ile Ile Thr Val Glu Gly Ala Ala Leu Asp Pro Asp Asp Met
               20                  25                  30
   Ala Phe Asp Val Ser Trp Phe Ala Val His Ser Phe Gly Leu Asp Lys
           35                  40                  45
   Ala Pro Val Leu Leu Ser Ser Leu Asp Arg Lys Gly Ile Val Thr Thr
       50                  55                  60
   Ser Arg Arg Asp Trp Lys Ser Asp Leu Ser Leu Glu Arg Val Ser Val
   65                  70                  75                  80
   Leu Glu Phe Leu Leu Gln Val His Gly Ser Glu Asp Gln Asp Phe Gly
                   85                  90                  95
   Asn Tyr Tyr Cys Ser Val Thr Pro Trp Val Lys Ser Pro Thr Gly Ser
               100                 105                 110
   Trp Gln Lys Glu Ala Glu Ile His Ser Lys Pro Val Phe Ile Thr Val
           115                 120                 125
   Lys Met Asp Val Leu Asn Ala Phe Lys Tyr Pro Leu Leu Ile Gly Val
       130                 135                 140
   Gly Leu Ser Thr Val Ile Gly Leu Leu Ser Cys Leu Ile Gly Tyr Cys
   145                 150                 155                 160
   Ser Ser His Trp Cys Cys Lys Lys Glu Val Gln Glu Thr Arg Arg Glu
                   165                 170                 175
   Arg Arg Arg Leu Met Ser Met Glu Met Asp
               180                 185
<210> 33
   <211> 1944
   <212> PRT
   <213> Homo sapiens
<220>
   <221> misc_feature
   <223> CDS de la seq ID 29, n° accession GenBank/AF006515: 211..6045
<220>
   <221> misc_feature
   <223> nom: protéine CHD3
<400> 33
   Met Lys Ala Ala Asp Thr Val Ile Leu Trp Ala Arg Ser Lys Asn Asp
   1               5                   10                  15
   Gln Leu Arg Ile Ser Phe Pro Pro Gly Leu Cys Trp Gly Asp Arg Met
               20                  25                  30
   Pro Asp Lys Asp Asp Ile Arg Leu Leu Pro Ser Ala Leu Gly Val Lys
           35                  40                  45
   Lys Arg Lys Arg Gly Pro Lys Lys Gln Lys Glu Asn Lys Pro Gly Lys
       50                  55                  60
   Pro Arg Lys Arg Lys Lys Arg Asp Ser Glu Glu Glu Phe Gly Ser Glu
   65                  70                  75                  80
   Arg Asp Glu Tyr Arg Glu Lys Ser Glu Ser Gly Gly Ser Glu Tyr Gly
                   85                  90                  95
   Thr Gly Pro Gly Arg Lys Arg Arg Arg Lys His Arg Glu Lys Lys Glu
               100                 105                 110
   Lys Lys Thr Lys Arg Arg Lys Lys Gly Glu Gly Asp Gly Gly Gln Lys
           115                 120                 125
   Gln Val Glu Gln Lys Ser Ser Ala Thr Leu Leu Leu Thr Trp Gly Leu
       130                 135                 140
   Glu Asp Val Glu His Val Phe Ser Glu Glu Asp Tyr His Thr Leu Thr
   145                 150                 155                 160
   Asn Tyr Lys Ala Phe Ser Gln Phe Met Arg Pro Leu Ile Ala Lys Lys
                   165                 170                 175
   Asn Pro Lys Ile Pro Met Ser Lys Met Met Thr Ile Leu Gly Ala Lys
               180                 185                 190
   Trp Arg Glu Phe Ser Ala Asn Asn Pro Phe Lys Gly Ser Ala Ala Ala
           195                 200                 205
   Val Ala Ala Ala Ala Ala Ala Ala Ala Ala Ala Val Ala Glu Gln Val
       210                 215                 220
   Ser Ala Ala Val Ser Ser Ala Thr Pro Ile Ala Pro Ser Gly Pro Pro
   225                 230                 235                 240
   Ala Leu Pro Pro Pro Pro Ala Ala Asp Ile Gln Pro Pro Pro Ile Arg
                   245                 250                 255
   Arg Ala Lys Thr Lys Glu Gly Lys Gly Pro Gly His Lys Arg Arg Ser
               260                 265                 270
   Lys Ser Pro Arg Val Pro Asp Gly Arg Lys Lys Leu Arg Gly Lys Lys
           275                 280                 285
   Met Ala Pro Leu Lys Ile Lys Leu Gly Leu Leu Gly Gly Lys Arg Lys
       290                 295                 300
   Lys Gly Gly Ser Tyr Val Phe Gln Ser Asp Glu Gly Pro Glu Pro Glu
   305                 310                 315                 320
   Ala Glu Glu Ser Asp Leu Asp Ser Gly Ser Val His Ser Ala Ser Gly
                   325                 330                 335
   Arg Pro Asp Gly Pro Val Arg Thr Lys Lys Leu Lys Arg Gly Arg Pro
               340                 345                 350
   Gly Arg Lys Lys Lys Lys Val Leu Gly Cys Pro Ala Val Ala Gly Glu
           355                 360                 365
   Glu Glu Val Asp Gly Tyr Glu Thr Asp His Gln Asp Tyr Cys Glu Val
       370                 375                 380
   Cys Gln Gln Gly Gly Glu Ile Ile Leu Cys Asp Thr Cys Pro Arg Ala
   385                 390                 395                 400
   Tyr His Leu Val Cys Leu Asp Pro Glu Leu Asp Arg Ala Pro Glu Gly
                   405                 410                 415
   Lys Trp Ser Cys Pro His Cys Glu Lys Glu Gly Val Gln Trp Glu Ala
               420                 425                 430
   Lys Glu Glu Glu Glu Glu Tyr Glu Glu Glu Gly Glu Glu Glu Gly Glu
           435                 440                 445
   Lys Glu Glu Glu Asp Asp His Met Glu Tyr Cys Arg Val Cys Lys Asp
       450                 455                 460
   Gly Gly Glu Leu Leu Cys Cys Asp Ala Cys Ile Ser Ser Tyr His Ile
   465 470 475 480
   His Cys Leu Asn Pro Pro Leu Pro Asp Ile Pro Asn Gly Glu Trp Leu
   485 490 495
   Cys Pro Arg Cys Thr Cys Pro Val Leu Lys Gly Arg Val Gln Lys Ile
   500 505 510
   Leu His Trp Arg Trp Gly Glu Pro Pro Val Ala Val Pro Ala Pro Gln
   515 520 525
   Gln Ala Asp Gly Asn Pro Asp Val Pro Pro Pro Arg Pro Leu Gln Gly
   530 535 540
   Arg Ser Glu Arg Glu Phe Phe Val Lys Trp Val Gly Leu Ser Tyr Trp
   545 550 555 560
   His Cys Ser Trp Ala Lys Glu Leu Gln Leu Glu Ile Phe His Leu Val
   565 570 575
   Met Tyr Arg Asn Tyr Gln Arg Lys Asn Asp Met Asp Glu Pro Pro Pro
   580 585 590
   Leu Asp Tyr Gly Ser Gly Glu Asp Asp Gly Lys Ser Asp Lys Arg Lys
   595 600 605
   Val Lys Asp Pro His Tyr Ala Glu Met Glu Glu Lys Tyr Tyr Arg Phe
   610 615 620
   Gly Ile Lys Pro Glu Trp Met Thr Val His Arg Ile Ile Asn His Ser
   625 630 635 640
   Val Asp Lys Lys Gly Asn Tyr His Tyr Leu Val Lys Trp Arg Asp Leu
   645 650 655
   Pro Tyr Asp Gln Ser Thr Trp Glu Glu Asp Glu Met Asn Ile Pro Glu
   660 665 670
   Tyr Glu Glu His Lys Gln Ser Tyr Trp Arg His Arg Glu Leu Ile Met
   675 680 685
   Gly Glu Asp Pro Ala Gln Pro Arg Lys Tyr Lys Lys Lys Lys Lys Glu
   690 695 700
   Leu Gln Gly Asp Gly Pro Pro Ser Ser Pro Thr Asn Asp Pro Thr Val
   705 710 715 720
   Lys Tyr Glu Thr Gln Pro Arg Phe Ile Thr Ala Thr Gly Gly Thr Leu
   725 730 735
   His Met Tyr Gln Leu Glu Gly Leu Asn Trp Leu Arg Phe Ser Trp Ala
   740 745 750
   Gln Gly Thr Asp Thr Ile Leu Ala Asp Glu Met Gly Leu Gly Lys Thr
   755 760 765
   Ile Gln Thr Ile Val Phe Leu Tyr Ser Leu Tyr Lys Glu Gly His Thr
   770 775 780
   Lys Gly Pro Phe Leu Val Ser Ala Pro Leu Ser Thr Ile Ile Asn Trp
   785 790 795 800
   Glu Arg Glu Phe Gln Met Trp Ala Pro Lys Phe Tyr Val Val Thr Tyr
   805 810 815
   Thr Gly Asp Lys Asp Ser Arg Ala Ile Ile Arg Glu Asn Glu Phe Ser
   820 825 830
   Phe Glu Asp Asn Ala Ile Lys Gly Gly Lys Lys Ala Phe Lys Met Lys
   835 840 845
   Arg Glu Ala Gln Val Lys Phe His Val Leu Leu Thr Ser Tyr Glu Leu
   850 855 860
   Ile Thr Ile Asp Gln Ala Ala Leu Gly Ser Ile Arg Trp Ala Cys Leu
   865 870 875 880
   Val Val Asp Glu Ala His Arg Leu Lys Asn Asn Gln Ser Lys Phe Phe
   885 890 895
   Arg Val Leu Asn Gly Tyr Lys Ile Asp His Lys Leu Leu Leu Thr Gly
   900 905 910
   Thr Pro Leu Gln Asn Asn Leu Glu Glu Leu Phe His Leu Leu Asn Phe
   915 920 925
   Leu Thr Pro Glu Arg Phe Asn Asn Leu Glu Gly Phe Leu Glu Glu Phe
   930 935 940
   Ala Asp Ile Ser Lys Glu Asp Gln Ile Lys Lys Leu His Asp Leu Leu
   945 950 955 960
   Gly Pro His Met Leu Arg Arg Leu Lys Ala Asp Val Phe Lys Asn Met
   965 970 975
   Pro Ala Lys Thr Glu Leu Ile Val Arg Val Glu Leu Ser Pro Met Gln
   980 985 990
   Lys Lys Tyr Tyr Lys Tyr Ile Leu Thr Arg Asn Phe Glu Ala Leu Asn
   995 1000 1005
   Ser Arg Gly Gly Gly Asn Gln Val Ser Leu Leu Asn Ile Met Met
   1010 1015 1020
   Asp Leu Lys Lys Cys Cys Asn His Pro Tyr Leu Phe Pro Val Ala
   1025 1030 1035
   Ala Met Glu Ser Pro Lys Leu Pro Ser Gly Ala Tyr Glu Gly Gly
   1040 1045 1050
   Ala Leu Ile Lys Ser Ser Gly Lys Leu Met Leu Leu Gln Lys Met
   1055 1060 1065
   Leu Arg Lys Leu Lys Glu Gln Gly His Arg Val Leu Ile Phe Ser
   1070 1075 1080
   Gln Met Thr Lys Met Leu Asp Leu Leu Glu Asp Phe Leu Asp Tyr
   1085 1090 1095
   Glu Gly Tyr Lys Tyr Glu Arg Ile Asp Gly Gly Ile Thr Gly Ala
   1100 1105 1110
   Leu Arg Gln Glu Ala Ile Asp Arg Phe Asn Ala Pro Gly Ala Gln
   1115 1120 1125
   Gln Phe Cys Phe Leu Leu Ser Thr Arg Ala Gly Gly Leu Gly Ile
   1130 1135 1140
   Asn Leu Ala Thr Ala Asp Thr Val Ile Ile Phe Asp Ser Asp Trp
   1145 1150 1155
   Asn Pro His Asn Asp Ile Gln Ala Phe Ser Arg Ala His Arg Ile
   1160 1165 1170
   Gly Gln Ala Asn Lys Val Met Ile Tyr Arg Phe Val Thr Arg Ala
   1175 1180 1185
   Ser Val Glu Glu Arg Ile Thr Gln Val Ala Lys Arg Lys Met Met
   1190 1195 1200
   Leu Thr His Leu Val Val Arg Pro Gly Leu Gly Ser Lys Ala Gly
   1205 1210 1215
   Ser Met Ser Lys Gln Glu Leu Asp Asp Ile Leu Lys Phe Gly Thr
   1220 1225 1230
   Glu Glu Leu Phe Lys Asp Glu Asn Glu Gly Glu Asn Lys Glu Glu
   1235 1240 1245
   Asp Ser Ser Val Ile His Tyr Asp Asn Glu Ala Ile Ala Arg Leu
   1250 1255 1260
   Leu Asp Arg Asn Gln Asp Ala Thr Glu Asp Thr Asp Val Gln Asn
   1265 1270 1275
   Met Asn Glu Tyr Leu Ser Ser Phe Lys Val Ala Gln Tyr Val Val
   1280 1285 1290
   Arg Glu Glu Asp Lys Ile Glu Glu Ile Glu Arg Glu Ile Ile Lys
   1295 1300 1305
   Gln Glu Glu Asn Val Asp Pro Asp Tyr Trp Glu Lys Leu Leu Arg
   1310 1315 1320
   His His Tyr Glu Gln Gln Gln Glu Asp Leu Ala Arg Asn Leu Gly
   1325 1330 1335
   Lys Gly Lys Arg Val Arg Lys Gln Val Asn Tyr Asn Asp Ala Ala
   1340 1345 1350
   Gln Glu Asp Gln Asp Asn Gln Ser Glu Tyr Ser Val Gly Ser Glu
   1355 1360 1365
   Glu Glu Asp Glu Asp Phe Asp Glu Arg Pro Glu Gly Arg Arg Gln
   1370 1375 1380
   Ser Lys Arg Gln Leu Arg Asn Glu Lys Asp Lys Pro Leu Pro Pro
   1385 1390 1395
   Leu Leu Ala Arg Val Gly Gly Asn Ile Glu Val Leu Gly Phe Asn
   1400 1405 1410
   Thr Arg Gln Arg Lys Ala Phe Leu Asn Ala Val Met Arg Trp Gly
   1415 1420 1425
   Met Pro Pro Gln Asp Ala Phe Thr Thr Gln Trp Leu Val Arg Asp
   1430 1435 1440
   Leu Arg Gly Lys Thr Glu Lys Glu Phe Lys Ala Tyr Val Ser Leu
   1445 1450 1455
   Phe Met Arg His Leu Cys Glu Pro Gly Ala Asp Gly Ser Glu Thr
   1460 1465 1470
   Phe Ala Asp Gly Val Pro Arg Glu Gly Leu Ser Arg Gln Gln Val
   1475 1480 1485
   Leu Thr Arg Ile Gly Val Met Ser Leu Val Lys Lys Lys Val Gln
   1490 1495 1500
   Glu Phe Glu His Ile Asn Gly Arg Trp Ser Met Pro Glu Leu Met
   1505 1510 1515
   Pro Asp Pro Ser Ala Asp Ser Lys Arg Ser Ser Arg Ala Ser Ser
   1520 1525 1530
   Pro Thr Lys Thr Ser Pro Thr Thr Pro Glu Ala Ser Ala Thr Asn
   1535 1540 1545
   Ser Pro Cys Thr Ser Lys Pro Ala Thr Pro Ala Pro Ser Glu Lys
   1550 1555 1560
   Gly Glu Gly Ile Arg Thr Pro Leu Glu Lys Glu Glu Ala Glu Asn
   1565 1570 1575
   Gln Glu Glu Lys Pro Glu Lys Asn Ser Arg Ile Gly Glu Lys Met
   1580 1585 1590
   Glu Thr Glu Ala Asp Ala Pro Ser Pro Ala Pro Ser Leu Gly Glu
   1595 1600 1605
   Arg Leu Glu Pro Arg Lys Ile Pro Leu Glu Asp Glu Val Pro Gly
   1610 1615 1620
   Val Pro Gly Glu Met Glu Pro Glu Pro Gly Tyr Arg Gly Asp Arg
   1625 1630 1635
   Glu Lys Ser Glu Asp Val Lys Gly Asp Arg Glu Leu Arg Pro Gly
   1640 1645 1650
   Pro Arg Asp Glu Pro Arg Ser Asn Gly Arg Arg Glu Glu Lys Thr
   1655 1660 1665
   Glu Lys Pro Arg Phe Met Phe Asn Ile Ala Asp Gly Gly Phe Thr
   1670 1675 1680
   Glu Leu His Thr Leu Trp Gln Asn Glu Glu Arg Ala Ala Ile Ser
   1685 1690 1695
   Ser Gly Lys Leu Asn Glu Ile Trp His Arg Arg His Asp Tyr Trp
   1700 1705 1710
   Leu Leu Ala Gly Ile Val Leu His Gly Tyr Ala Arg Trp Gln Asp
   1715 1720 1725
   Ile Gln Asn Asp Ala Gln Phe Ala Ile Ile Asn Glu Pro Phe Lys
   1730 1735   1740
   Thr Glu Ala Asn Lys Gly Asn Phe Leu Glu Met Lys Asn Lys Phe
   1745 1750 1755
   Leu Ala Arg Arg Phe Lys Leu Leu Glu Gln Ala Leu Val Ile Glu
   1760 1765 1770
   Glu Gln Leu Arg Arg Ala Ala Tyr Leu Asn Leu Ser Gln Glu Pro
   1775 1780 1785
   Ala His Pro Ala Met Ala Leu His Ala Arg Phe Ala Glu Ala Glu
   1790 1795 1800
   Cys Leu Ala Glu Ser His Gln His Leu Ser Lys Glu Ser Leu Ala
   1805 1810 1815
   Gly Asn Lys Pro Ala Asn Ala Val Leu His Lys Val Leu Asn Gln
   1820 1825 1830
   Leu Glu Glu Leu Leu Ser Asp Met Lys Ala Asp Val Thr Arg Leu
   1835 1840 1845
   Pro Ala  Thr Leu Ser Arg Ile  Pro Pro Ile Ala Ala  Arg Leu Gln
       1850                 1855                 1860
   Met Ser  Glu Arg Ser Ile Leu  Ser Arg Leu Ala Ser  Lys Gly Thr
       1865                 1870                 1875
   Glu Pro  His Pro Thr Pro Ala  Tyr Pro Pro Gly Pro  Tyr Ala Thr
       1880                 1885                 1890
   Pro Pro  Gly Tyr Gly Ala Ala  Phe Ser Ala Ala Pro  Val Gly Ala
       1895                 1900                 1905
   Leu Ala  Ala Ala Gly Ala Asn  Tyr Ser Gln Met Pro  Ala Gly Ser
       1910                 1915                 1920
   Phe Ile  Thr Ala Ala Thr Asn  Gly Pro Ile His Arg  Phe Phe Lys
       1925                 1930                 1935
   Lys Val  Pro Glu Ile Gln
       1940
<210> 34
   <211> 1944
   <212> PRT
   <213> Homo sapiens
<220>
   <221> misc_feature
   <223> CDS de la seq ID 30, n° accession GenBank/NM_001272: 211..6045
<220>
   <221> misc_feature
   <223> nom: protéine 3 liant l'hélicase d'ADN à chromodomaine CHD3
<400> 34
   Met Lys Ala Ala Asp Thr Val Ile Leu Trp Ala Arg Ser Lys Asn Asp
   1               5                   10                  15
   Gln Leu Arg Ile Ser Phe Pro Pro Gly Leu Cys Trp Gly Asp Arg Met
               20                  25                  30
   Pro Asp Lys Asp Asp Ile Arg Leu Leu Pro Ser Ala Leu Gly Val Lys
           35                  40                  45
   Lys Arg Lys Arg Gly Pro Lys Lys Gln Lys Glu Asn Lys Pro Gly Lys
       50                  55                  60
   Pro Arg Lys Arg Lys Lys Arg Asp Ser Glu Glu Glu Phe Gly Ser Glu
   65                  70                  75                  80
   Arg Asp Glu Tyr Arg Glu Lys Ser Glu Ser Gly Gly Ser Glu Tyr Gly
                   85                  90                  95
   Thr Gly Pro Gly Arg Lys Arg Arg Arg Lys His Arg Glu Lys Lys Glu
               100                 105                 110
   Lys Lys Thr Lys Arg Arg Lys Lys Gly Glu Gly Asp Gly Gly Gln Lys
           115                 120                 125
   Gln Val Glu Gln Lys Ser Ser Ala Thr Leu Leu Leu Thr Trp Gly Leu
       130                 135                 140
   Glu Asp Val Glu His Val Phe Ser Glu Glu Asp Tyr His Thr Leu Thr
   145                 150                 155                 160
   Asn Tyr Lys Ala Phe Ser Gln Phe Met Arg Pro Leu Ile Ala Lys Lys
                   165                 170                 175
   Asn Pro Lys Ile Pro Met Ser Lys Met Met Thr Ile Leu Gly Ala Lys
                   180                 185                 190
   Trp Arg Glu Phe Ser Ala Asn Asn Pro Phe Lys Gly Ser Ala Ala Ala
               195                 200                 205
   Val Ala Ala Ala Ala Ala Ala Ala Ala Ala Ala Val Ala Glu Gln Val
       210                 215                 220
   Ser Ala Ala Val Ser Ser Ala Thr Pro Ile Ala Pro Ser Gly Pro Pro
   225                 230                 235                 240
   Ala Leu Pro Pro Pro Pro Ala Ala Asp Ile Gln Pro Pro Pro Ile Arg
                   245                 250                 255
   Arg Ala Lys Thr Lys Glu Gly Lys Gly Pro Gly His Lys Arg Arg Ser
               260                 265                 270
   Lys Ser Pro Arg Val Pro Asp Gly Arg Lys Lys Leu Arg Gly Lys Lys
           275                 280                 285
   Met Ala Pro Leu Lys Ile Lys Leu Gly Leu Leu Gly Gly Lys Arg Lys
       290                 295                 300
   Lys Gly Gly Ser Tyr Val Phe Gln Ser Asp Glu Gly Pro Glu Pro Glu
   305                 310                 315                 320
   Ala Glu Glu Ser Asp Leu Asp Ser Gly Ser Val His Ser Ala Ser Gly
                   325                 330                 335
   Arg Pro Asp Gly Pro Val Arg Thr Lys Lys Leu Lys Arg Gly Arg Pro
               340                 345                 350
   Gly Arg Lys Lys Lys Lys Val Leu Gly Cys Pro Ala Val Ala Gly Glu
           355                 360                 365
   Glu Glu Val Asp Gly Tyr Glu Thr Asp His Gln Asp Tyr Cys Glu Val
       370                 375                 380
   Cys Gln Gln Gly Gly Glu Ile Ile Leu Cys Asp Thr Cys Pro Arg Ala
   385                 390                 395                 400
   Tyr His Leu Val Cys Leu Asp Pro Glu Leu Asp Arg Ala Pro Glu Gly
                   405                 410                 415
   Lys Trp Ser Cys Pro His Cys Glu Lys Glu Gly Val Gln Trp Glu Ala
               420                 425                 430
   Lys Glu Glu Glu Glu Glu Tyr Glu Glu Glu Gly Glu Glu Glu Gly Glu
           435                 440                 445
   Lys Glu Glu Glu Asp Asp His Met Glu Tyr Cys Arg Val Cys Lys Asp
       450                 455                 460
   Gly Gly Glu Leu Leu Cys Cys Asp Ala Cys Ile Ser Ser Tyr His Ile
   465                 470                 475                 480
   His Cys Leu Asn Pro Pro Leu Pro Asp Ile Pro Asn Gly Glu Trp Leu
                   485                 490                 495
   Cys Pro Arg Cys Thr Cys Pro Val Leu Lys Gly Arg Val Gln Lys Ile
               500                 505                 510
   Leu His Trp Arg Trp Gly Glu Pro Pro Val Ala Val Pro Ala Pro Gln
           515                 520                 525
   Gln Ala Asp Gly Asn Pro Asp Val Pro Pro Pro Arg Pro Leu Gln Gly
       530                 535                 540
   Arg Ser Glu Arg Glu Phe Phe Val Lys Trp Val Gly Leu Ser Tyr Trp
   545                 550                 555                 560
   His Cys Ser Trp Ala Lys Glu Leu Gln Leu Glu Ile Phe His Leu Val
                   565                 570                 575
   Met Tyr Arg Asn Tyr Gln Arg Lys Asn Asp Met Asp Glu Pro Pro Pro
               580                 585                 590
   Leu Asp Tyr Gly Ser Gly Glu Asp Asp Gly Lys Ser Asp Lys Arg Lys
           595                 600                 605
   Val Lys Asp Pro His Tyr Ala Glu Met Glu Glu Lys Tyr Tyr Arg Phe
       610                 615                 620
   Gly Ile Lys Pro Glu Trp Met Thr Val His Arg Ile Ile Asn His Ser
   625                 630                 635                 640
   Val Asp Lys Lys Gly Asn Tyr His Tyr Leu Val Lys Trp Arg Asp Leu
                   645                 650                 655
   Pro Tyr Asp Gln Ser Thr Trp Glu Glu Asp Glu Met Asn Ile Pro Glu
               660                 665                 670
   Tyr Glu Glu His Lys Gln Ser Tyr Trp Arg His Arg Glu Leu Ile Met
           675                 680                 685
   Gly Glu Asp Pro Ala Gln Pro Arg Lys Tyr Lys Lys Lys Lys Lys Glu
       690                 695                 700
   Leu Gln Gly Asp Gly Pro Pro Ser Ser Pro Thr Asn Asp Pro Thr Val
   705                 710                 715                 720
   Lys Tyr Glu Thr Gln Pro Arg Phe Ile Thr Ala Thr Gly Gly Thr Leu
                   725                 730                 735
   His Met Tyr Gln Leu Glu Gly Leu Asn Trp Leu Arg Phe Ser Trp Ala
               740                 745                 750
   Gln Gly Thr Asp Thr Ile Leu Ala Asp Glu Met Gly Leu Gly Lys Thr
           755                 760                 765
   Ile Gln Thr Ile Val Phe Leu Tyr Ser Leu Tyr Lys Glu Gly His Thr
       770                 775                 780
   Lys Gly Pro Phe Leu Val Ser Ala Pro Leu Ser Thr Ile Ile Asn Trp
   785                 790                 795                 800
   Glu Arg Glu Phe Gln Met Trp Ala Pro Lys Phe Tyr Val Val Thr Tyr
                   805                 810                 815
   Thr Gly Asp Lys Asp Ser Arg Ala Ile Ile Arg Glu Asn Glu Phe Ser
               820                 825                 830
   Phe Glu Asp Asn Ala Ile Lys Gly Gly Lys Lys Ala Phe Lys Met Lys
           835                 840                 845
   Arg Glu Ala Gln Val Lys Phe His Val Leu Leu Thr Ser Tyr Glu Leu
       850                 855                 860
   Ile Thr Ile Asp Gln Ala Ala Leu Gly Ser Ile Arg Trp Ala Cys Leu
   865                 870                 875                 880
   Val Val Asp Glu Ala His Arg Leu Lys Asn Asn Gln Ser Lys Phe Phe
               885                 890                 895
   Arg Val Leu Asn Gly Tyr Lys Ile Asp His Lys Leu Leu Leu Thr Gly
           900                 905                 910
   Thr Pro Leu Gln Asn Asn Leu Glu Glu Leu Phe His Leu Leu Asn Phe
       915                 920                 925
   Leu Thr Pro Glu Arg Phe Asn Asn Leu Glu Gly Phe Leu Glu Glu Phe
       930                 935                 940
   Ala Asp Ile Ser Lys Glu Asp Gln Ile Lys Lys Leu His Asp Leu Leu
   945                 950                 955                 960
   Gly Pro His Met Leu Arg Arg Leu Lys Ala Asp Val Phe Lys Asn Met
               965                 970                 975
   Pro Ala Lys Thr Glu Leu Ile Val Arg Val Glu Leu Ser Pro Met Gln
           980                 985                 990
   Lys Lys Tyr Tyr Lys Tyr Ile Leu  Thr Arg Asn Phe Glu  Ala Leu Asn
       995                 1000                1005
   Ser Arg  Gly Gly Gly Asn Gln  Val Ser Leu Leu Asn  Ile Met Met
       1010                1015                1020
   Asp Leu  Lys Lys Cys Cys Asn  His Pro Tyr Leu Phe  Pro Val Ala
       1025                1030                1035
   Ala Met  Glu Ser Pro Lys Leu  Pro Ser Gly Ala Tyr  Glu Gly Gly
       1040                1045                1050
   Ala Leu  Ile Lys Ser Ser Gly  Lys Leu Met Leu Leu  Gln Lys Met
       1055                1060                1065
   Leu Arg  Lys Leu Lys Glu Gln  Gly His Arg Val Leu  Ile Phe Ser
       1070                1075                1080
   Gln Met  Thr Lys Met Leu Asp  Leu Leu Glu Asp Phe  Leu Asp Tyr
       1085                1090                1095
   Glu Gly  Tyr Lys Tyr Glu Arg  Ile Asp Gly Gly Ile  Thr Gly Ala
       1100                1105                1110
   Leu Arg  Gln Glu Ala Ile Asp  Arg Phe Asn Ala Pro  Gly Ala Gln
       1115                1120                1125
   Gln Phe  Cys Phe Leu Leu Ser  Thr Arg Ala Gly Gly  Leu Gly Ile
       1130                1135                1140
   Asn Leu  Ala Thr Ala Asp Thr  Val Ile Ile Phe Asp  Ser Asp Trp
       1145                1150                1155
   Asn Pro  His Asn Asp Ile Gln  Ala Phe Ser Arg Ala  His Arg Ile
       1160                1165                1170
   Gly Gln  Ala Asn Lys Val Met  Ile Tyr Arg Phe Val  Thr Arg Ala
       1175                1180                1185
   Ser Val  Glu Glu Arg Ile Thr  Gln Val Ala Lys Arg  Lys Met Met
       1190                1195                1200
   Leu Thr  His Leu Val Val Arg  Pro Gly Leu Gly Ser  Lys Ala Gly
       1205                1210                1215
   Ser Met  Ser Lys Gln Glu Leu  Asp Asp Ile Leu Lys  Phe Gly Thr
       1220                1225                1230
   Glu Glu  Leu Phe Lys Asp Glu  Asn Glu Gly Glu Asn  Lys Glu Glu
       1235                 1240                 1245
   Asp Ser  Ser Val Ile His Tyr  Asp Asn Glu Ala Ile  Ala Arg Leu
       1250                 1255                 1260
   Leu Asp  Arg Asn Gln Asp Ala  Thr Glu Asp Thr Asp  Val Gln Asn
       1265                 1270                 1275
   Met Asn  Glu Tyr Leu Ser Ser  Phe Lys Val Ala Gln  Tyr Val Val
       1280                 1285                 1290
   Arg Glu  Glu Asp Lys Ile Glu  Glu Ile Glu Arg Glu  Ile Ile Lys
       1295                 1300                 1305
   Gln Glu  Glu Asn Val Asp Pro  Asp Tyr Trp Glu Lys  Leu Leu Arg
       1310                 1315                 1320
   His His  Tyr Glu Gln Gln Gln  Glu Asp Leu Ala Arg  Asn Leu Gly
       1325                 1330                 1335
   Lys Gly  Lys Arg Val Arg Lys  Gln Val Asn Tyr Asn  Asp Ala Ala
       1340                 1345                 1350
   Gln Glu  Asp Gln Asp Asn Gln  Ser Glu Tyr Ser Val  Gly Ser Glu
       1355                 1360                 1365
   Glu Glu  Asp Glu Asp Phe Asp  Glu Arg Pro Glu Gly  Arg Arg Gln
       1370                 1375                 1380
   Ser Lys  Arg Gln Leu Arg Asn  Glu Lys Asp Lys Pro  Leu Pro Pro
       1385                 1390                 1395
   Leu Leu  Ala Arg Val Gly Gly  Asn Ile Glu Val Leu  Gly Phe Asn
       1400                 1405                 1410
   Thr Arg  Gln Arg Lys Ala Phe  Leu Asn Ala Val Met  Arg Trp Gly
       1415                 1420                 1425
   Met Pro  Pro Gln Asp Ala Phe  Thr Thr Gln Trp Leu  Val Arg Asp
       1430                 1435                 1440
   Leu Arg  Gly Lys Thr Glu Lys  Glu Phe Lys Ala Tyr  Val Ser Leu
       1445                 1450                 1455
   Phe Met  Arg His Leu Cys Glu  Pro Gly Ala Asp Gly  Ser Glu Thr
       1460                 1465                 1470
   Phe Ala  Asp Gly Val Pro Arg  Glu Gly Leu Ser Arg  Gln Gln Val
       1475                 1480                 1485
   Leu Thr  Arg Ile Gly Val Met  Ser Leu Val Lys Lys  Lys Val Gln
       1490                 1495                 1500
   Glu Phe  Glu His Ile Asn Gly  Arg Trp Ser Met Pro  Glu Leu Met
       1505                 1510                 1515
   Pro Asp  Pro Ser Ala Asp Ser  Lys Arg Ser Ser Arg  Ala Ser Ser
       1520                 1525                 1530
   Pro Thr  Lys Thr Ser Pro Thr  Thr Pro Glu Ala Ser  Ala Thr Asn
       1535                 1540                 1545
   Ser Pro  Cys Thr Ser Lys Pro  Ala Thr Pro Ala Pro  Ser Glu Lys
       1550                 1555                 1560
   Gly Glu Gly Ile Arg Thr Pro Leu Glu Lys Glu Glu Ala Glu Asn
       1565                1570                1575
   Gln Glu Glu Lys Pro Glu Lys Asn Ser Arg Ile Gly Glu Lys Met
       1580                1585                1590
   Glu Thr Glu Ala Asp Ala Pro Ser Pro Ala Pro Ser Leu Gly Glu
       1595                1600                1605
   Arg Leu Glu Pro Arg Lys Ile Pro Leu Glu Asp Glu Val Pro Gly
       1610                1615                1620
   Val Pro Gly Glu Met Glu Pro Glu Pro Gly Tyr Arg Gly Asp Arg
       1625                1630                1635
   Glu Lys Ser Glu Asp Val Lys Gly Asp Arg Glu Leu Arg Pro Gly
       1640                1645                1650
   Pro Arg Asp Glu Pro Arg Ser Asn Gly Arg Arg Glu Glu Lys Thr
       1655                1660                1665
   Glu Lys Pro Arg Phe Met Phe Asn Ile Ala Asp Gly Gly Phe Thr
       1670                1675                1680
   Glu Leu His Thr Leu Trp Gln Asn Glu Glu Arg Ala Ala Ile Ser
       1685                1690                1695
   Ser Gly Lys Leu Asn Glu Ile Trp His Arg Arg His Asp Tyr Trp
       1700                1705                1710
   Leu Leu Ala Gly Ile Val Leu His Gly Tyr Ala Arg Trp Gln Asp
       1715                1720                1725
   Ile Gln Asn Asp Ala Gln Phe Ala Ile Ile Asn Glu Pro Phe Lys
       1730                1735                1740
   Thr Glu Ala Asn Lys Gly Asn Phe Leu Glu Met Lys Asn Lys Phe
       1745                1750                1755
   Leu Ala Arg Arg Phe Lys Leu Leu Glu Gln Ala Leu Val Ile Glu
       1760                1765                1770
   Glu Gln Leu Arg Arg Ala Ala Tyr Leu Asn Leu Ser Gln Glu Pro
       1775                1780                1785
   Ala His Pro Ala Met Ala Leu His Ala Arg Phe Ala Glu Ala Glu
       1790                1795                1800
   Cys Leu Ala Glu Ser His Gln His Leu Ser Lys Glu Ser Leu Ala
       1805                1810                1815
   Gly Asn Lys Pro Ala Asn Ala Val Leu His Lys Val Leu Asn Gln
       1820                1825                1830
   Leu Glu Glu Leu Leu Ser Asp Met Lys Ala Asp Val Thr Arg Leu
       1835                1840                1845
   Pro Ala Thr Leu Ser Arg Ile Pro Pro Ile Ala Ala Arg Leu Gln
       1850                1855                1860
   Met Ser Glu Arg Ser Ile Leu Ser Arg Leu Ala Ser Lys Gly Thr
       1865                1870                1875
   Glu Pro His Pro Thr Pro Ala Tyr Pro Pro Gly Pro Tyr Ala Thr
       1880                1885                1890
   Pro Pro Gly Tyr Gly Ala Ala Phe Ser Ala Ala Pro Val Gly Ala
           1895                      1900                     1905
   Leu Ala  Ala Ala Gly Ala Asn  Tyr Ser Gln Met Pro  Ala Gly Ser
       1910                      1915                     1920
   Phe Ile  Thr Ala Ala Thr Asn  Gly Pro Ile His Arg  Phe Phe Lys
       1925                      1930                     1935
   Lys Val  Pro Glu Ile Gln
       1940

## Description

La présente invention appartient au domaine des compositions pharmaceutiques utiles pour le traitement de pathologies résultant d'une dérégulation du mécanisme de l'angiogénèse.

Elle concerne des compositions comprenant d'une part des séquences de nouveaux gènes, dont la fonction n'était pas identifiée à ce jour et dont l'implication dans le mécanisme de l'angiogénèse a été mis en évidence pour la première fois par la demanderesse et d'autre part des séquences de gènes, dont au moins une de leurs fonctions a été préalablement identifiée, mais dont leur implication, en tant que gènes codant pour les constituants cellulaires de la cellule endothéliale, dans le mécanisme de l'angiogenèse a été mise en évidence pour la première fois lors des travaux réalisés par la demanderesse dans le cadre de la présente invention. Ces gènes sont identifiés par leurs séquences nucléotidiques dans le listage de séquences en annexe. La présente invention concerne également les séquences polypeptidiques des facteurs codés par lesdits gènes qui trouvent leur application dans l'étude clinique du processus de l'angiogénèse, le pronostic, le diagnostic et le traitement de pathologies liées à ce processus ainsi que dans la mise en oeuvre des essais pharmacologiques, pharmacogénomiques, ou pharmacosignalitiques.

L'angiogenèse est un processus fondamental par lequel de nouveaux vaisseaux sanguins se forment. Ce processus est essentiel dans plusieurs phénomènes physiologiques normaux tels que la reproduction, le développement ou encore la cicatrisation. Dans ces phénomènes biologiques normaux, l'angiogenèse est sous contrôle strict, c'est-à-dire qu'elle est déclenchée pendant une période courte, quelques jours, puis complètement inhibée. Cependant, plusieurs pathologies sont liées à une angiogénèse invasive et incontrôlée. L'arthrite, par exemple, est une pathologie due à l'endommagement des cartilages par les néovaisseaux invasifs. Dans la rétinopathie diabétique, l'invasion de la rétine par les néovaisseaux conduit à l'aveuglement des malades; la néovascularisation de l'appareil oculaire présente la cause majeure de l'aveuglement et cette néovascularisation domine une vingtaine de maladies de l'oeil. Enfin, la croissance et la métastase des tumeurs sont directement liées à la néovascularisation et sont dépendantes de l'angiogenèse. La tumeur stimule la croissance des néovaisseaux pour sa croissance elle-même. De plus, ces néovaisseaux présentent les voies d'échappement des tumeurs pour joindre la circulation sanguine et provoquer les métastases dans des sites à distance comme le foie, le poumon ou l'os.

Dans d'autres pathologies telles que les maladies cardio-vasculaires, les maladies d'artères périphériques, les lésions vasculaires ou cérébrales, l'angiogenèse peut présenter une base thérapeutique importante. En effet, la promotion de l'angiogenèse dans les endroits endommagés peut conduire à la formation de néovaisseaux sanguins latéraux et alternatifs aux vaisseaux endommagés fournissant ainsi le sang et par conséquence l'oxygène et d'autres facteurs nutritifs et biologiques nécessaires à la survie des tissus concernés.

La formation de néovaisseaux par les cellules endothéliales implique la migration, la croissance, et la différentiation des cellules endothéliales. La régulation de ces phénomènes biologiques est directement liée à l'expression génétique. En matière d'angiogénèse, un nombre sans cesse grandissant d'études montre que la régulation de l'angiogenèse se fait à travers un équilibre entre des facteurs agissant directement sur la cellule endothéliale. Ces facteurs peuvent être stimulants, d'une part, tels que, entre autres, le VEGF, le FGFs, l'IL-8, le HGF/SF, le PDGF. Ils peuvent aussi être inhibants, comme, entre autres, l'IL-10, l'IL-12, le gro-_ __et gro-_, le facteur plaquettaire 4, l'angiostatine, l'inhibiteur dérivé de chondrocyte humaine, la thrombospondine, le facteur inhibiteur de la leucémie. (Jensen, 1998, Surg. Neural., 49, 189-195 ; Tamatani et al., 1999, Carcinogenesis, 20, 957-962 ; Tanaka et al., 1998, Cancer Res., 58, 3362-3369 ; Ghe et al., 1997, Cancer Res., 57, 3733-3740 ; Kawahara et al., 1998, Hepatology, 28, 1512-1517 ; Chandhuni et al., 1997, Cancer Res., 57, 1814-1819 ; Jendraschak et Sage, 1996, Semin. Cancer Biol., 7, 139-146 ; Majewski et al., 1996, J. Invest. Dermatol., 106, 1114-1119).

La régulation de l'angiogénèse telle qu'elle est décrite de nos jours se fait à travers un équilibre de deux types de facteurs :
- les facteurs angiogéniques (polypeptides extracellulaires, pour la plupart mitogèniques) agissant directement sur les cellules endothéliales induisant l'angiogènese.
- les facteurs angiostatiques (polypeptides extracellulaires, également pour la plupart, mitogéniques ou agissant sur la mitogénèse) agissant également directement sur les cellules endothéliales pour inhiber l'angiogénèse.

L'équilibre entre ces deux types de facteurs extracellulaires régule l'angiogénèse. On note à ce stade là, que le contrôle de l'angiogénèse se fait par la production de facteurs angiogéniques et facteurs angiostatiques. Par exemple, il a déjà été montré que la stimulation de la cellule endothéliale par un facteur angiogénique induit l'expression 1) de l'activateur du plasminogène de type urokinase (uPA) et de son inhibiteur PAI-I (Pepper et al, 1990, J Cell Biol 111(2),743-55 ; Pepper et al, 1996, Enzyme Protein,49(1-3),138-62), 2) des métalloprotéinases de la matrice (MMPs) et d'inhibiteurs physiologiques de l'activité de ces MMPs (TIMPs) (Cornelius et al, 1995, J Invest Dermatol,105(2),170-6; Jackson et Nguyen, 1997, Int J Biochem Cell Biol, 29(10),1167-77), 3) des inhibiteurs tels que l'Angiopoïètine-2(Ang-2) ou la Thrombospondine-1 (TSP-1) (Mandriota et Pepper, 1998, Circ. Res. 83, 852-859; Oh et al, 1999, J Biol Chem, 274(22), 15732-9 ; Suzuma et al, 1999, American Journal of Pathology, 154, 343-354).Donc, il apparaît normal qu'une cellule endothéliale à l'état angiogénique produisent des facteurs angiogéniques mais également des facteurs angiostatiques. La production de ces derniers permet de contrôler l'angiogénèse.

En parallèle à cet état de fait, les cellules endothéliales stimulées par un facteur angiostatique produisent des facteurs angiostatiques mais également des facteurs angiogéniques pour contrôler l'état angiostatique. Ce phénomène a déjà été décrit chez d'autres types cellulaires producteurs de facteurs impliqués dans l'angiogenèse, qui lorsqu'ils sont stimulés par un facteur angiostatique tel que l'interféron-gamma produisent des facteurs angiogéniques et des facteurs angiostatiques (Kobayashi et al, 1995, Immunopharmacology,31(1), 93-101; Arkins et al, 1995, Mol Endocrinol, 9(3), 350-60 ; Kodelja et al, 1997, Immunobiology,197(5), 478-93).

Dans la présente invention, la demanderesse a démontré que la stimulation des cellules endothéliales par un facteur angiostatique conduit à l'expression de gènes codant pour des constituants cellulaires impliqués dans la promotion de l'angiogénèse.

Ainsi, la stimulation des cellules endothéliales par un facteur angiogénique ou un facteur angiostatique peut l'une et l'autre induire l'expression de régulateurs positifs et négatifs de l'angiogenèse

Le contrôle de l'angiogénèse représente donc un axe stratégique, à la fois de recherche fondamentale, afin d'améliorer la compréhension de nombreux phénomènes pathologiques liés à l'angiogénèse, mais aussi une base pour l'élaboration des nouvelles thérapies destinées à traiter les pathologies liées à l'angiogénèse.

Afin de contrôler l'angiogenèse, plusieurs groupes pharmaceutiques ont développé des stratégies thérapeutiques basées directement sur l'utilisation de signaux paracrines, les facteurs stimulateurs et inhibiteurs, comme agents pour promouvoir ou inhiber l'angiogenèse. Ces stratégies sont basées essentiellement sur l'utilisation de ces facteurs sous leur forme polypeptidiques comme agents stimulateurs ou inhibiteurs de l'angiogenèse, ou encore plus récemment sous la forme de vecteurs d'expression codant pour les facteurs choisis.

Un procédé permettant l'identification de nouveaux gènes impliqués dans la régulation de l'angiogenèse a été mis au point. Il a fait l'objet d'un demande de brevet français publiée sous le n° FR 2798674 et d'une demande de brevet internationale PCT publiée sous le n° WO 01/21831. Cette méthode a la particularité de traduire fidèlement le mécanisme intime régulant l'angiogenèse en prenant en compte tous les facteurs extracellulaires décrits comme agents régulateurs de l'angiogenèse, c'est-à-dire les facteurs angiogéniques, les facteurs angiostatiques, ainsi que les différents composants de la matrice extracellulaire. Cette méthodologie consiste à mettre en oeuvre ces différents facteurs extracellulaires à travers quatre conditions expérimentales bien définies. Les cellules endothéliales sont cultivées sur une composante et/ou un mélange bien défini de plusieurs composantes de la matrice extracellulaire et placées sous les quatre conditions expérimentales à savoir :
Une condition contrôle où les cellules endothéliales ne sont pas stimulées.
Une condition angiogénique où les cellules endothéliales sont stimulées par un ou plusieurs facteurs angiogéniques.
Une condition d'inhibition de l'angiogenèse où les cellules endothéliales sont stimulées par un ou plusieurs facteurs angiogéniques et mises en présence d'un ou plusieurs facteurs angiostatiques.
Une autre condition de contrôle où les cellules endothéliales sont stimulées par un ou plusieurs facteurs angiostatiques.

Ces quatre conditions permettent d'obtenir des préparations d'ARNm spécifiques de l'angiogenèse à savoir de l'état angiogénique et/ou l'inhibition de l'angiogenèse et permettent de déceler les gènes codant pour les constituants cellulaires impliqués dans la régulation de l'angiogénèse, incluant des régulateurs positifs et des régulateurs négatifs. Donc, le procédé ci-dessus décrit permet le criblage systématique de tous les facteurs angiogéniques et angiostatiques ainsi que des différentes composantes de la matrice extracellulaire dans le but de mettre en évidence et identifier les gènes codant pour les constituants cellulaires impliqués dans la régulation de l'angiogenèse. De plus, étant donné que l'expression génique peut être analysée tout au long de la cinétique de la formation des néovaisseaux par les cellules endothéliales, cette approche constitue une méthodologie in *vitro* permettant de relier l'expression génique aux paramètres fonctionnels biologiques de l'angiogenèse.

L'identification des cinq gènes rapportés ci-dessous a été effectuée selon la méthodologie décrite ci-dessus, en utilisant les facteurs angiogéniques et angiostatiques, ainsi que le collagène type I comme composant de la matrice extracellulaire pour reproduire les quatre conditions expérimentales.

La Demanderesse a prouvé l'implication de ces cinq nouveaux gènes identifiés par les séquences SEQ ID No. : 1 à SEQ ID No. : 5 dans la liste de séquences en annexe, dans le mécanisme de régulation de l'angiogènese.

Selon un mode particulier de mise en oeuvre, la composition pharmaceutique de l'invention comprend à titre de composé actif au moins une séquence nucléotidique telle que décrite dans la revendication 1.

Au sens de la présente invention, doivent être considérées comme des séquences équivalentes, des séquences nucléotidiques présentant des modifications structurelles, mineures, ne modifiant pas leur fonction, telles que délétions, mutations ou additions de bases, dont l'identité est d'au moins de 90% avec les séquences nucléotidiques identifiées sous les numéros SEQ ID No. : 1 à SEQ ID No. : 5, et SEQ ID No. 27 à SEQ ID No. 30 dans la liste de séquences en annexe.

Selon un autre mode particulier de mise en oeuvre, la composition pharmaceutique, régulatrice de l'angiogénèse, comprend au moins une séquence inhibitrice de l'angiogènese.

Selon un mode particulier de mise en oeuvre, la composition pharmaceutique de l'invention comprend une ou plusieurs séquences inhibitrices de l'angiogènese telle que décrite dans la revendication 2.

De préférence, la composition pharmaceutique de l'invention comprend une séquence antisens choisies parmi la SEQ ID N°11, dans la liste de séquences en annexe.

L'invention a également pour objet une composition pharmaceutique destinée au diagnostic, pronostic et/ou au traitement de pathologies liées à l'angiogenèse caractérisée en ce qu'elle comprend au moins une séquence polypeptidique, telle que décrite dans la revendication 1.

Au sens de la présente invention, doivent être considérées comme des séquences équivalentes, des séquences polypeptidiques présentant des modifications structurelles, mineures, ne modifiant pas leur fonction, telles que délétions, mutations ou additions de résidus d'acides aminés, dont l'identité est d'au moins de 85%, de préférence d'au moins 90% avec les séquences polypeptidiques identifiées sous les numéros SEQ ID No. : 6 à SEQ ID N. : 10 ou avec les séquences polypeptidiques identifiées sous les numéros SEQ ID No. : 31 à SEQ ID No. : 34 dans la liste de séquences en annexe.

Un autre objet de l'invention concerne les séquences nucléotidiques antisens des séquences nucléotidiques identifiées sous les numéros SEQ ID No : 1, SEQ ID No. 27 et SEQ ID No. 25 dans la liste de séquences en annexe.

Dans le cadre de la présente invention on comprend par séquence antisens: Toute séquence d'ADN, d'au moins 10 mers, complémentaire d'un ARNm qui en s'hybridant avec une portion de la séquence d'ARNm initial inhibe son expression, c'est-à-dire sa traduction en une protéine.

Tout préférentiellement l'invention a pour objet les séquences antisens ayant une identité d'au moins 85%, de préférence d'au moins 95% avec une séquence choisie parmi les séquences identifiées sous les numéros SEQ ID N° 11 à SEQ ID N° 14 dans la liste de séquences en annexe.

L'invention a également pour objet un vecteur d'expression de mammifère comprenant au moins une séquences antisens définie ci-dessus.

Selon des modes de réalisation préférés, ledit vecteur d'expression de mammifère comprend au moins une séquence antisens d'au moins une des séquences identifiées sous les numéros SEQ ID No. : 1, SEQ ID No. 27 et SEQ ID No. 28 dans la liste de séquences en annexe, ainsi qu'un promoteur qui permet l'expression dudit ADN antisens.

Plus particulièrement ledit vecteur est choisi parmi le groupe de vecteurs GS-V1, identifiés par leur séquence portant le numéro SEQ ID N° 15 dans la liste de séquences en annexe.

De plus, l'introduction de ladite séquence antisens identifiée sous le numéro SEQ ID N° 11 dans la liste de séquences en annexe, ou de l'un de leurs homologues dans des vecteurs d'expression de mammifère, et l'insertion ultérieure desdits vecteurs dans des cellules de mammifère permet l'obtention de lignées cellulaires sous-exprimant les gènes intervenant dans le mécanisme de l'angiogènese.

Aussi, pour la construction de ces vecteurs, des amorces spécifiques pour chacune des séquences identifiées, sont dessinées. Elles comprennent dans leurs extrémités les sites de restriction non contenus dans le fragment cloné et présents dans la région multisite du vecteur d'expression.

Ces amorces particulièrement préférées sont indiquées sur le Tableau **I** (voir matériels et méthode) et identifiées avec les numéros de séquences SEQ ID No. 19 à SEQ ID No. : 26 dans la liste de séquences en annexe.

L'invention a également pour objet un vecteur d'expression de mammifère comprenant au moins une séquence nucléotidique choisie parmi l'ensemble de séquences identifiées sous les numéros SEQ ID No. : 1, SEQ ID No. 27 et SEQ ID No. 28 dans la liste de séquences en annexe ou un de leurs fragments.

Ces constructions sont utiles d'une part pour préparer des compositions thérapeutiques destinées au traitement, par thérapie cellulaire, de désordres angiogéniques et d'autre part pour vérifier l'efficacité d'un traitement d'un désordre angiogénique chez un mammifère, notamment chez un être humain, ou encore pour vérifier la fonctionnalité des gènes éventuellement impliquées dans le mécanisme de l'angiogénèse, dans ledit mammifère.

Un autre objet de l'invention est une méthode de préparation d'une protéine codée par au moins un des gènes dont les séquences sont identifiées sous les numéros SEQ ID No. : 1, SEQ ID No. 27 et SEQ ID No. 28 dans la liste de séquences en annexe ou d'un de leurs fragments.

Ces protéines identifiées par les séquences SEQ ID No. 6, SEQ ID No. 31 et SEQ ID No. 32 dans la liste de séquences en annexe, ou leurs fragments peuvent être produites, sous forme de protéines recombinantes in *vitro,* en introduisant dans un hôte adéquat un vecteur d'expression correspondant adéquat, puis purifiées et utilisées ensuite comme agent thérapeutique.

Une telle méthode de préparation d'une protéine recombinante comprend les étapes de :
a) la construction d'un vecteur d'expression comprenant au moins une séquence parmi celles identifiées sous les numéros SEQ ID No. : 1 à SEQ ID No. 5, et SEQ ID No. 27 à SEQ ID No. 30 dans la liste de séquences en annexe ou d'un de leurs fragments.
b) l'introduction dudit vecteur dans un hôte cellulaire,
c) la culture desdites cellules dans un milieu adéquat,
d) la purification des protéines exprimées ou d'un de leurs fragments.

L'invention concerne également une protéine recombinante obtenue par la méthode ci-dessus.

L'invention a également pour objet une composition thérapeutique comprenant une telle protéine recombinante.

A titre d'exemple, des systèmes d'expression de protéines recombinantes chez les bactéries telles que E. Coli peuvent être utilisés pour exprimer de protéines ou polypeptides non glycosylés.

La séquence codante ou une séquence partielle du gène d'intérêt est amplifiée par PCR en utilisant des amorces spécifiques de ce gène avec aux extrémités des sites d'enzymes de restriction de préférence différents pour permettre l'orientation du gène dans le vecteur d'expression. L'ADN amplifié est purifié puis digéré par les enzymes de restriction ensuite insérée par ligation au vecteur d'expression préalablement digéré par ces mêmes enzymes de restriction. Un grand nombre de vecteurs peut-être utilisé comme le vecteur pBR322 (Bolivar et al., Gene 2 (1977) 95-113) ou ses dérivés, contenant par exemple le promoteur de l'ARN polymérase du bactériophage T7 pour un haut niveau d'expression, comme le plasmide pET3a (Studier et Moffatt,1986, J Mol Biol, 189(1):113-30), contenant de préférence les séquences qui codent pour des marqueurs de sélection (résistance à des antibiotiques), un site multiple de clonages contenant les sites d'enzymes de restrictions adéquats pour l'insertion de l'ADN, le système cellule/hôte est de préférence un système inductible comme celui utilisé pour le radiomarquage in vivo du facteur de croissance FGF2 (Colin et al, 1997, Eur.J.Biochem., 249, 473-480) et déjà décrit par Patry et al (1994, FEBS Lett, 349(1):23-8), il peut également contenir une région codant pour une queue poly histidine en extrémité du polypeptide d'intérêt pour faciliter la purification.

L'ADN amplifié est ligué dans le plasmide qui est transformé dans la bactérie selon la méthode décrite par Sambrook et al. (1989, Molecular Cloning: A Laboratory Manual, 2nd ed., Cold Spring Harbor Laboratory Press, Cold Spring Harbor, NY.). Les cellules transformées sont étalées sur milieu LB agar contenant les antibiotiques, les colonies résistantes aux antibiotiques sont alors contrôlées par PCR puis analyse sur gel. L'ADN plasmidique peut être isolé puis séquencé pour confirmer la construction. La production et la purification de la protéine recombinante peuvent être réalisées comme décrit (Patry et al, 1994, FEBS Lett, 349(1):23-8),473-480). Brièvement, une colonie isolée est inoculée dans le milieu de culture liquide tel que le milieu LB broth additionné des antibiotiques. Après une nuit d'incubation, la pré-culture peut être utilisée pour ensemencer une culture d'un plus grand volume. L'expression du polypeptide est alors induite, les cellules se développent pendant quelques heures puis sont récoltées par centrifugation. Le culot cellulaire peut être lysé par agents chimiques connus dans l'art ou mécaniquement par sonication par exemple. La protéine peut-être purifiée grâce à ses propriétés physicochimiques comme décrit pour la purification du FGF2 recombinant (Colin et al, 1997, Eur.J.Biochem., 249, 473-480) ou si la protéine est étiquetée avec une queue poly histidine, elle peut être purifiée via cette queue par immobilisation sur un support chélateur d'ions métalliques comme décrit (Tang et al, Protein Expr Purif 1997 Dec. 11(3):279-83).

A titre d'exemple, des systèmes d'expression de protéines recombinantes pour exprimer des polypeptides ayant des modifications post traductionnelles comme la glycosylation, tels que des systèmes eucaryotes (levures, plantes, insectes) peuvent être utilisés.

Ainsi, la protéine recombinante peut être produite par exemple dans la levure *Pichia Pastoris* comme décrit par Sreekrishna et al (1988, J Basic Microbiol,28(4):265-78). L'ADN amplifié sera introduit de la même façon après digestion et ligation dans un vecteur d'expression de *Pichia Pastoris,* contenant de préférence une séquence codant pour un marqueur de sélection (Scorer et al, Biotechnology (N Y), 1994 Feb;12(2):181-4). La protéine peut être soit intracellulaire soit sécrétée si le vecteur contient à l'extrémité du gène introduit une séquence codant pour une séquence signal de sécrétion comme par exemple le facteur prepropeptide de Saccharomyces cerevisiae (Cregg et al., 1993; Scorer et al., 1993). Une queue Histidine peut également être ajoutée à l'une des extrémités de la protéine recombinante pour faciliter la purification (Mozley et al, 1997, Photochem Photobiol, 66(5):710-5).

De préférence, ledit hôte est choisi parmi : une bactérie, une levure, une cellule d'insecte et une cellule d'un mammifère, une cellules végétale.

L'administration de compositions thérapeutiques comprenant de telles protéines peut se faire par exemple par voie topique, orale, intradermique, transdermique, Intraveineuse.

Les fragments desdites protéines peuvent être utilisés en tant qu'antagonistes de la protéine dont ils sont issus. Ainsi l'administration adéquate à un animal d'une composition thérapeutique comprenant de tels fragments est préconisée pour induire une diminution de l'activité de ladite protéine dans le mécanisme de l'angiogenèse dans une pathologie donnée.

La présente invention concerne également une méthode de diagnostic et/ou pronostic d'une pathologie angiogénique chez un mammifère, notamment chez un être humain, consistant à détecter dans les cellules dudit mammifère la surexpression ou la sous-expression d'une ou plusieurs séquences nucléotidiques identifiées par les numéros SEQ ID No. : 1, SEQ ID No. 27et No. : SEQ ID No. 25 dans la liste de séquences en annexe.

Une telle méthode de diagnostic et /ou pronostic comprend les étapes suivantes :
- la détection de l'expression d'une ou plusieurs desdites séquences nucléotidiques SEQ ID No. : 1 , SEQ ID No. 27 et SEQ ID No. 28 par une population cellulaire d'un mammifère,
- la détection de l'expression de celle(s) même(s) séquence(s) par une population cellulaire de référence dont l'état angiogénique est connu,
- l'identification des différences éventuelles du niveau d'expression de celle(s) même(s) séquence(s) par les deux populations cellulaires.

La présente invention concerne également une méthode de diagnostic et de pronostic d'une pathologie angiogénique chez un mammifère, notamment chez un être humain, consistant à détecter dans les cellules dudit mammifère la surexpression ou la sous-expression d'une ou plusieurs séquences polypeptidiques identifiées par les numéros SEQ ID No. : 6 ou avec les séquences polypeptidiques identifiées sous les numéros SEQ ID No. : 31 et SEQ ID No. : 32 dans la liste de séquences en annexe.

Selon un mode de mise en oeuvre préféré, ladite méthode comporte les étapes suivantes :
a) la détection de l'expression d'une ou plusieurs desdites séquences polypeptidiques SEQ ID No. : 6 ou avec les séquences polypeptidiques identifiées sous les numéros SEQ ID No. : 31 et SEQ ID No. : 32 par une population cellulaire d'un mammifère,
b) la détection de l'expression de celle(s) même(s) séquence(s) polypeptidique(s) par une population cellulaire de référence dont l'état angiogénique est connu,
c) l'identification des différences éventuelles du niveau d'expression de celle(s) même(s) séquence(s) polypeptidique(s) par les deux populations cellulaires.

Selon un mode particulier de mise en oeuvre, dans la méthode de diagnostic et pronostic de l'invention la détection de l'expression des séquences est effectuée après avoir mis les cellules endothéliales en présence d'un fluide biologique issu d'un patient.

La présente invention concerne également une méthode de vérification de l'efficacité thérapeutique d'un traitement angiogénique chez un mammifère, notamment chez un être humain, par l'identification d'une population cellulaire chez ledit mammifère susceptible de surexprimer ou sous-exprimer une ou plusieurs séquences nucléotidiques identifiées par les numéros SEQ ID No. : 1, SEQ ID No. 27 et SEQ ID No. 28 dans la liste de séquences en annexe.

Une telle méthode de vérification de l'efficacité thérapeutique comprend les étapes suivantes :
- la détection de l'expression d'une ou plusieurs desdites séquences nucléotidiques SEQ ID No. : 1, SEQ ID No. 27 et SEQ ID No. 28 par une population cellulaire d'un mammifère auquel est administrée une composition thérapeutique destinée à traiter un désordre angiogénique,
- la détection de l'expression de celle(s) même(s) séquence(s) nucléotidiques par une population cellulaire de référence dont l'état angiogénique est connu,
- l'identification des différences éventuelles du niveau d'expression de celle(s) même(s) séquence(s) nucléotidique(s) par les deux populations cellulaires.

Selon des modes de réalisation préférés, la méthode de vérification est effectuée sur une population cellulaire d'un mammifère *in vivo, ex-vivo,* ou bien sur une population cellulaire isolée dudit mammifère *in vitro*

Selon un mode particulier de mise en oeuvre, dans la méthode de vérification de l'invention la détection de l'expression des séquences est effectuée après avoir mis les cellules endothéliales en présence d'un fluide biologique issu d'un patient.

La présente invention concerne également une méthode de criblage de composés utiles pour le traitement angiogénique d'un mammifère, notamment d'un être humain.

Selon un mode de mise en oeuvre préféré, une telle méthode de criblage comprend les étapes suivantes :
- la détection de l'expression d'une ou plusieurs desdites séquences nucléotidiques SEQ ID No. : 1, SEQ ID No. 27 et SEQ ID No. 28 par une population cellulaire mise en présence d'un composé susceptible d'avoir un effet thérapeutique sur un désordre angiogénique,
- la détection de l'expression de celle(s) même(s) séquence(s) nucléotidique(s) par une population cellulaire de référence dont l'état angiogénique est connu,
- l'identification des différences éventuelles du niveau d'expression de celle(s) même(s) séquence(s) nucléotidique(s) par les deux populations cellulaires.

Selon un autre mode de mise en oeuvre préféré, une telle méthode de criblage comprend également les étapes suivantes :
- la détection de l'expression d'une ou plusieurs desdites séquences polypeptidiques identifiées par les numéros SEQ ID No. : 6 ou avec les séquences polypeptidiques identifiées sous les numéros SEQ ID No. : 31 et SEQ ID No. : 32 dans la liste de séquences en annexe par une population cellulaire mise en présence d'un composé susceptible d'avoir un effet thérapeutique sur un désordre angiogénique,
- la détection de l'expression de celle ou celles mêmes séquences polypeptidiques par une population cellulaire de référence dont l'état angiogénique est connu,
- l'identification des différences éventuelles du niveau d'expression de celle(s) même(s) séquence(s) polypeptidique(s) par les deux populations cellulaires.

Selon un mode particulier de mise en oeuvre, dans la méthode de criblage de l'invention la détection de l'expression des séquences est effectuée après avoir mis les cellules endothéliales en présence d'un fluide biologique issu d'un patient.

Parmi les désordres angiogéniques, susceptibles d'être diagnostiqués ou traités avec les compositions pharmaceutiques de l'invention on peut citer : la vascularisation de tumeurs, les rétinopathies, l'arthrite rhumatoïde, la maladie de Crohn, l'athérosclérose, l'hyperstimulation de l'ovaire, le psoriasis, l'endométrie associée à la néovascularisation, la resténose due à l'angioplastie du ballon, la superproduction tissulaire due à la cicatrisation, la maladie vasculaire périphérique, l'hypertension, l'inflammation vasculaire, la maladie et les phénomènes de Raynaud, l'anévrisme, la resténose artérielle, la thrombophlébite, la lymphagyte, le lymphodème, la cicatrisation et la réparation tissulaire, l'ischémie, l'angine, l'infarctus de myocarde, la maladie chronique du coeur, les insuffisances cardiaques telles que l'insuffisance cardiaque congestive, la dégénération maculaire liée à l'âge et l'ostéoporose.

Dans le cadre de la présente invention on entend par sonde tout fragment d'ADN simple brin dont la séquence est complémentaire à une séquence recherchée : celle-ci pourra ainsi être décelée par hybridation avec la sonde marquée (par incorporation d'atomes radioactifs ou de groupements fluorescents), qui joue le rôle d'un "hameçon" Moléculaire.

Selon des modes de mise en oeuvre préférés, le support dudit dispositif est choisi parmi une membrane de verre, une membrane métallique, une membrane polymère, une membrane de silice.

De tels dispositifs sont par exemple des puces à ADN comprenant une ou plusieurs séquences nucléotidiques identifiées sous les numéros SEQ ID No. : 1 à SEQ ID No. : 5, et SEQ ID No. 27 à SEQ ID No. 30, dans la liste de séquences en annexe.

La vérification de l'implication des cinq gènes identifiés et de leurs homologues dans le mécanisme de l'angiogènese a été effectuée selon la méthodologie décrite dans la section Matériel et méthodes.

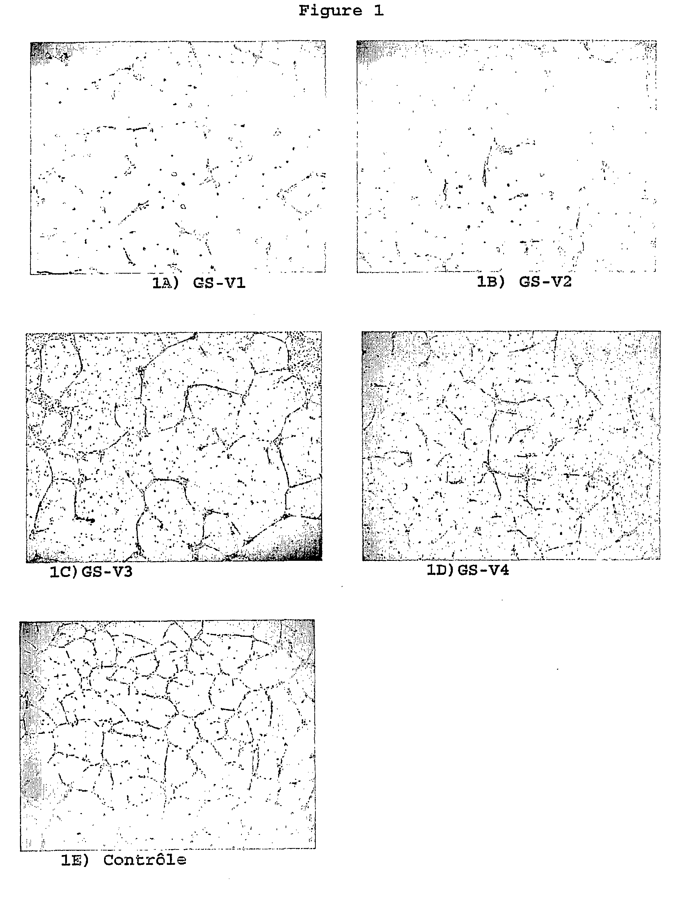

Cette implication est illustrée à l'aide de la figure 1 en annexe, dans laquelle :
La figure 1 montre les résultats obtenus sur la formation de tubes capillaires sur des cellules endothéliales humaines sous l'effet de l'expression des vecteurs GS-V1, GS-V2, GS-V3, GS-V4, dans laquelle :
   - la figure 1A montre que la formation des tubes capillaires est inhibée dans des cellules endothéliales transfectées avec le vecteur GS-V1 codant pour le transcrit antisens spécifique de GS-N1,
   la figure 1B montre que la formation des tubes capillaires est inhibée dans des cellules endothéliales transfectées avec le vecteur GS-V2 codant pour le transcrit antisens spécifique de GS-N2,
   - la figure 1C montre que la formation des tubes capillaires est inhibée dans des cellules endothéliales transfectées avec le vecteur GS-V3 codant pour le transcrit antisens spécifique de GS-N3,
   - la figure 1D montre que la formation des tubes capillaires est inhibée dans des cellules endothéliales transfectées avec le vecteur GS-V4 codant pour le transcrit antisens spécifique de GS-N4 et de son homologue GS-N5 et
   - la figure 1F montre que la formation des tubes capillaires n'est pas modifiée dans des cellules endothéliales transfectées avec le vecteur le vecteur vide (Contrôle).

### MATÉRIEL ET METHODES

### 1.Culture des cellules et test d'angiogenèse

Des cellules endothéliales de veines ombilicales (HUVEC) sous lesdites quatre conditions de culture sont alors utilisées pour identifier les gènes codant pour les constituants cellulaires impliqués dans la régulation de l'angiogenèse.

Les cellules endothéliales sont maintenues en milieu complet (EGM-2-MV de Clonetics).

Pour l'identification des gènes impliqués dans l'angiogenèse dans les test *in vitro* de l'angiogenèse selon le modèle de Montesano et al.(1986, Proc Natl Acad Sci U S A, 83(19),7297-301). Les cellules sont tout d'abord ensemencées sur un gel de Collagène type I en milieu complet jusqu'à confluence. Puis, les cellules HUVEC de référence sont cultivées en milieu appauvri en sérum et sans facteurs de croissance : EBM-2-MV + 2% de sérum et différents facteurs sont ajoutés dans les conditions test.

Le FGF2 : à des concentrations comprises entre 5 ng/ml et 60 ng/ml, de préférence entre 10 et 40 ng/ml ; du VEGF : à des concentrations comprises entre 10 ng/ml et 60 ng/ml, de préférence comprises entre 30 ng/ml et 50 ng/ml ; du PF4 : à de concentrations comprises entre 0,1 et 5 *µ*g/ml, de préférence comprises entre 0,5 *µ*g/ml et 1 *µ*g/ml ; du TNF-alpha à des concentrations comprises entre 20 ng/ml et 100 ng/ml, de préférence comprises entre 30 ng/ml et 60 ng/ml ; du IFN-gamma :à des concentrations comprises entre 50 ng/ml et 200 ng/ml, de préférence comprises entre 80 ng/ml et 120 ng/ml.

Les cellules endothéliales humaines placées sous les quatre conditions de culture précitées sont alors utilisées pour identifier des gènes codant pour les constituants cellulaires impliqués dans la régulation de l'angiogenèse.

### 2. Facteurs angiogéniques et angiostatiques.

Des facteurs angiogéniques et angiostatiques, ayant un effet sur l'expression des gènes identifiés en corrélation avec la formation de néovaisseaux ou l'inhibition de néovaisseaux respectivement, utilisés, à titre d'exemple, dans le cadre de la présente invention, sont illustrés ci-dessous.
VEGF= Facteur de croissance endothélial vasculaire.
PF4= Facteur plaquettaire 4.
TNF-_____Facteur de nécrose tumorale alpha

Le TNF-alpha qui est un régulateur de l'angiogénèse, peut induire l'angiogénèse *in vivo* mais également inhiber la formation des vaisseaux *in vitro* (Frater-Schroder et al, 1987, Proc Natl Acad Sci U S A,84(15),5277-81; Sato et al, 1987, J Natl Cancer Inst 79(6),1383-91; Fajardo et al, 1992, Am J Pathol Mar,140(3),539-44; Niida et al, 1995, Neurol Med Chir (Tokyo),35(4),209-14. Dans notre modèle d'angiogénèse *in vitro,* le TNF-alpha est utilisé dans des conditions d'inhibition de l'angiogenèse.

### 3. Comparaison des expressions géniques.

Les expressions génique peuvent être alors comparées en utilisant les puces d'ADN, le SAGE, une réaction d'amplification par PCR quantitative, les vecteurs viraux pour construire des banques soustractives, ou encore l'analyse par affichage différentiel.

Dans le cadre des travaux expérimentaux ayant conduit à la présente invention, la Demanderesse a utilisé préférentiellement la technique d'affichage différentiel pour l'identification desdits gènes.

### Affichage différentiel

Les ARN totaux sont préparés à partir des cellules HUVEC cultivées sur un gel de collagène en présence des différents facteurs utilisés, selon la méthode RNeasy Mini kit (Qiagen) en intégrant une étape de digestion à la DNase I selon le protocole préconisé par le fabricant.

L'affichage différentiel à partir des ARNs totaux est réalisé selon la méthode décrite par Liang et Pardee (1992, Science, 14;257(5072),967-7)en utilisant l'alpha_P33-ATP en dilution isotopique au cours de l'amplification par PCR pour la visualisation des bandes par autoradiographie des gels d'électrophorèse.

Ainsi, les fragments d'ADN différentiellement présents sur le gel en fonction des conditions de culture analysées sont découpés, réamplifiés, clonés dans un plasmide PGEM easy vector, (Promega), séquencés et identifiés par questionnement de la banque BLAST.

### 4. Vérification de l'implication des gènes identifiés dans le mécanisme de l'angiogènese.

### Test de fonctionnalité des gènes

Dans une deuxième étape, la fonctionnalité de chaque séquence identifiée est testée sur le modèle d'angiogenèse *in vitro* avec les cellules endothéliales transfectées avec un vecteur d'expression comprenant un oligonucléotide antisens de ladite séquence.

Pour la construction de ces vecteurs, des amorces spécifiques pour chacun des séquences identifiées, sont dessinées. Ces amorces sont indiquées sur le Tableau I ci-dessous et identifiées avec les numéros de séquences SEQ ID No. 19 à SEQ ID No. : 26 dans la liste de séquences en annexe.

**Tableau I**

| **SÉQ.ID du gène identifié** | **Nom amorce** |
|---|---|
| SEQ ID No 1 (GS-N1) | GV1-1 |
| | GV1-2 |
| SEQ ID No 2 (GS-N2) | GV2-1 |
| | GV2-2 |
| SEQ ID No 3 (GS-N3) | GV3-1 |
| | GV3-2 |
| SEQ ID No 4 (GS-N4) | GV4-1 |
| | GV4-2 |
| SEQ ID No 5 (GS-N5) | GV4-1 |
| | GV4-2 |

Ces amorces contiennent à chacune de leurs extrémités, un site d'une enzyme de restriction différente (SalI : GTCGAC ou MluI : ACGCGT).

Des fragments amplifiés de chaque gène sont obtenus par PCR à partir des plasmides bactériens contenant le fragment du gène identifié en utilisant lesdites amorces.

Ces fragments sont purifiés, digérés par les enzymes de restriction SalI et MluI et insérés dans un vecteur d'expression chez les mammifères du type pCi-neo vector, (Promega), lui-même digéré par ces deux enzymes de restriction.

Chaque fragment est introduit dans l'orientation antisens.

D'une façon générale, les vecteurs susceptibles d'être utilisés, pour la mise en évidence de la fonctionnalité des gènes identifiés dans la présente invention dans le mécanisme de l'angiogénèse comprennent tout système de vecteurs d'expression chez les mammifères comprenant un promoteur qui permet l'expression d'un gène cloné, à titre d'exemple on peut citer le promoteur « fort » du cytomégalovirus humain (CMV).

Les vecteurs d'expression constitutifs ou inductibles susceptibles d'être utilisés, sont indiqués dans la liste non-exhaustive ci-après indiquée :

Les vecteurs pCI Mammalian Expression vector, dont le numéro d'accession est U47120 dans la base de données EMBL. Expression Vector System cloning vector pALTER(R)*-MAX (Promega), vecteurs pcDNA3 .1, -/hygro, -/Zeo, pcDNA4/HisMAx, -E, pBudCE4, pRcRSV, pRcCMV2, pSecTag2,-/hygro secretion vectors, les vecteurs pEBVHis A, B et C) (Invitrogen), des vecteurs d'expression chez le mammifère pIRES, pIRES-EYFP pIRES2-EGFP, pCMV-Myc et pCMV-HA. La séquence de CMV dont le numéro d'accession est ABV26916 ou AI953818 est décrite dans la demande de brevet WO01/60860.Epitope-Tagged pTRE, les vecteurs VP16 Minimal Domain (ptTA 2, ptTA 3 et ptTA 4), les vecteurs d'expression Tet bidirectionnels (pBI, pBI-EGFP, pBI-G, pBI-L), pRevTRE, pTRE2, pLEGFP-N1 Vecteur Retroviral pLEGFP-C1, les systèmes d'expression adenoviraux Adeno-X , pCMS-EGFP, pd1EGFP-N1, pd2ECFP-N1, pd2EYFP-N1, pEGFP(-C1,-C2, -C3, -N1, -N2, -N3), pEYFP-C1, -N1 (Clontech).

Chaque vecteur comprenant ledit fragment anti-sens est alors produit chez *E.Coli* extrait, purifié et quantifié. Un *µ*g de chaque vecteur est incubé en présence d'un agent transfectant (effectene, Qiagen) en suivant le protocole préconisé par le fabricant avec les cellules endothéliales. Vingt-quatre heures après la transfection, les cellules endothéliales sont trypsinées et étalées sur la matrice extracellulaire contenant les facteurs angiogéniques en l'occurrence le matrigel selon le modèle décrit par Grant et al, (1989,Cell,58(5), 933-43). Après 24h d'incubation, la formation de vaisseaux est observée et comparée aux cellules témoins transfectées avec le vecteur d'expression de mammifère vide.

### 5. Etablissement de la banque de lignées stables exprimant les vecteurs d'expression contenant les séquences des gènes ou leurs fragments ou les séquences antisens.

Les systèmes d'expression peuvent comprendre un marqueur de sélection à un antibiotique (un gène de résistance à un antibiotique), pour sélectionner les cellules transfectées exprimant de façon stable le vecteur comprenant l'acide nucléique cloné dans ledit vecteur et ce, soit dans le même vecteur, soit dans un 2^{ème} vecteur co-transfecté.

Ce vecteur d'expression peut être un système d'expression constitutif ou inductible.

Dans l'exemple particulier ci-dessous décrit, les lignées stables pour l'expression de l'oligonucléotide antisens correspondant à chaque gène identifié ont été obtenues avec un vecteur d'expression constitutif et après sélection en présence d'antibiotique.

Pour ce faire, 24h après la transfection effectuée dans les conditions décrites ci-dessus, les cellules endothéliales BAEC sont trypsinées et ensemencées à raison de 80 000 cellules/puits en plaque de six puits en présence de 700 *µ*g/ml de l'antibiotique G418 (Promega). Un puit contrôle est ensemencé avec des cellules non transfectées. Le milieu est changé tous les trois jours avec une recharge de l'antibiotique. Les cellules contrôles sont éliminées après 8 à 10 jours, les cellules résistantes à l'antibiotique sont récoltées à confluence (après 2 à trois semaines) puis transférées en flacons de culture toujours en présence de l'antibiotique. Les lignées stables sont ensuite testées pour leur capacité à former ou non des vaisseaux dans le test d'angiogenèse *in vitro.*

### 6. Résultats

### 6.1 Identification des gènes.

La séquence d'acide nucléiques nommés GS-N1, et la protéine codée par ladite séquence d'acides nucléiques GS-N1 identifiées sous le numéro SEQ ID No. : 6 nommée : angiopartnerine, n'a pas été identifiée auparavant comme ayant un rôle biologique quelconque, et encore moins un dans le processus de l'angiogenèse ou la différentiation des cellules endothéliales en tubes capillaires. Les séquences d'acide nucléiques nommés GS-N2 à GS-N5 identifiées par les numéros SEQ ID No : 2 à SEQ ID No : 5 dans la liste de séquences en annexe et respectivement les protéines codées par lesdits acides nucléiques, identifiées sous les numéros SEQ ID No. : 7 à SEQ ID No. : 10 dans la liste de séquences en annexe n'ont pas été identifiées auparavant comme ayant un rôle biologique dans le processus de l'angiogènese ou la différentiation des cellules endothéliales en tubes capillaires. Ces séquences sont décrites ci-dessous.

La méthode d'affichage différentielle ci-dessus décrite a permis l'identification des ARNm suivants :
- GS-N1 : un ARNm de 6160pb identifié par la séquence SEQ ID No : 1 dans la liste de séquences en annexe. Une recherche BLAST sur la base de séquences GENBANK permet de l'identifier sous le numéro d'accession AB037857.

La séquence de cet ARNm a une séquence codante partielle du nucléotide 1 au nucléotide 2777. Il a été donc identifié une protéine GS-P1, résultante de la traduction de cet ARNm. Cette protéine est composée de 924aa, identifiée sous le numéro SEQ ID No 6 dans la liste de séquences en annexe, appelée angiopartnerine.

L'angiopartnerine est homologue au régulateur négatif du récepteur de la prostaglandine F2 (PTGFRN), (n°accession XM_040709,séquence nucléique : 5975 pb, séquence protéique : 560 aa) PTGFRN dont le numéro d'accession est aussi BC022235 dans la base de données EMBL, est décrite dans le brevet US 7,747,660, à la protéine 6 associée à la cellule du muscle lisse humaine (SMAP6) (n°accession AB014734,séquence nucléique : 2197 pb, séquence protéique partielle: 186 aa), identifiées sous les numéros SEQ ID No. : 27 et SEQ ID No. : 28 respectivement dans la liste de séquences en annexe, elle-même similaire à la protéine régulatrice du récepteur de la prostaglandine F2 alpha(FPRP)appelée indifféremment CD9P-1 et renommée EWI-F par Stipp et al (2001, J. Biol. Chem., 276, 44, 40545-40554). La séquence GS-N1 comprend les séquences SEQ ID No. : 27 et SEQ ID No. : 28, présentant 99% d'homologie avec celles-ci.

La FPRP est une glycoprotéine transmembranaire de type 1 contenant 6 domaines immunoglobulines extracellulaires. Elle a été identifiée et caractérisée à l'origine du fait de sa capacité à s'associer avec la prostaglandine F2 alpha et inhiber la liaison de cette dernière avec son récepteur. Elle pourrait également s'associer à d'autres récepteurs couplés à la protéine G, contenant 7 domaines transmembranaires et réduire l'interaction ligand-récepteur (Orlicky et Nordeen, 1996, Prostaglandins Leukot. Essent. Fatty Acids. 55:261-268; Orlicky et al, 1998 J. Lip. Res., Vol. 39, 1152-1161).L'augmentation de l'expression de la FPLP a déjà été associée à la différentiation cellulaire notamment celle des adipocytes (Orlicky et al, 1998, J. Lip. Res., Vol. 39, 1152-1161).Des travaux distincts ont démontré que CD9P-1 ou FPLP est un partenaire majeur de deux membres de la famille des tetraspanines (également appelée TM4SF), CD9 et CD81, dans des complexes protéiques, s'associant spécifiquement soit avec CD81 soit avec CD9 et CD81(Charrin S et al, 2001, J Biol Chem; Stipp et al (2001, J. Biol. Chem., 276, 7, 4853-4862).Les tétrapanines ont été impliquées dans beaucoup de fonctions cellulaires telles que l'adhésion, la migration, la co-stimulation, la transduction du signal et la différentiation, les diverses fonctions attribuées aux tétraspanines pouvant être liées à leur association spécifique avec des molécules partenaires spécifiques; (Le Naour et al, 2000, Science, 287, 319-321).

la protéine CD9 a une large distribution tissulaire, elle a été notamment trouvée dans plusieurs types de tumeurs (Si et Hersey, 1993, Int J Cancer,54:37-43; Miyake et al, 1996, Cancer Res,56:1244-1249) ainsi que dans les vaisseaux formés par les cellules endothéliales (Zola et al, 1989, Immunol Cell Biol.;67:63-70). Elle a été impliquée dans des fonctions telles que la transduction du signal, l'adhésion cellulaire, la motilité, la progression de tumeurs (Ozaki et al, 1995, J Biol Chem,270:15119-15124; Forsyth , 1991, Immunology.,72:292-296; Anton et al, 1995, J Neurosci.,15:584-595; Shaw et al, 1995, J Biol Chem., 270:24092-24099; Ikeyama et al, 1993, J Exp Med., 177:1231-1237) et notamment l'adhésion et la migration des cellules endothéliales au cours de l'angiogenèse (Klein-Soyer et al, 2000, Arterioscler Thromb Vasc Biol., 20:360-9). La surexpression de CD9 dans les cellules d'adénocarcinome supprime la motilité et le potentiel métastasique (Ikeyama et al, 1993, J Exp Med., 177:1231-1237), son expression est inversement corrélée avec les tumeurs primaires et l'apparition des métastases dans les mélanomes, les cancers du poumon, du colon et du sein (Si et Hersey, 1993, Int. J. Cancer, 54, 37-43; Miyake et al, 1995, Cancer Res.,55:4127-4131; Adachi et al, 1998, J. Clin. Oncol.,16, 1397-1406; Mori el al, 1998, Clin. Cancer Ress., 4, 1507-1510).

La protéine CD9P-1 (ou FPLP) a été identifiée comme étant la molécule partenaire majeure de CD9 dans des lignées cancéreuses (Serru et al, 1999, Biochem J, 340, 103-111)

Il a également été décrit que la protéine CD81 est impliquée dans diverses fonctions telles que la signalisation cellulaire et l'activation des lymphocytes B (Fearon et Carter,1995),la régulation de la prolifération des lymphocyte T (Miyazaki et al, 1997, EMBO J, 16, 4217-4225), elle peut également jouer un rôle dans le cancer car CD81 est un récepteur possible pour le virus de l'hépatite C, une cause majeure du carcinome hépatique (Pileri et al, 1998, Science, 282, 938-941).

Bien que le rôle exacte de CD9P-1 ou FPRP n'est pas encore défini, son association avec CD9 ou CD81 peut suggérer un rôle dans des fonctions de régulation des récepteurs CD9 ou CD81. Cependant, à ce jour aucun rôle dans la régulation de l'angiogenèse n'a été décrite ni pour la protéine appelée Angiopartnerine, identifiée sous le numéro SEQ ID No 6 dans la liste de séquences en annexe, ni pour les protéines PTGFRN, CD9P-1/FPRP.
- GS-N2 ; un ARNm de 3266 pb, identifié par la séquence SEQ ID No : 2 dans la liste de séquences en annexe. Une recherche BLAST sur la base de séquences GENBANK permet de l'identifier sous le numéro d'accession AF247704.

La séquence de cet ARNm a une séquence codante du nucléotide 49 au nucléotide 753. Il a donc été identifié une protéine GS-P2, résultante de la traduction de cet ARNm. Cette protéine est composée de 234 aa, identifiée sous le numéro SEQ ID No 7 dans la liste de séquences en annexe, elle est appelée protéine homeobox NKX3.1

La protéine NKX3.1 est un membre de la classe NK des protéines Homeobox, étroitement liée à la protéine NK-3 de la Drosophile (Kim, Y et Nirenberg, 1989, Proc. Natl Acad. Sci. USA, 86, 7716-7720 ; He et al., 1997, Genomics, 43, 69-77). Des travaux chez le murin, ont permis de montrer l'expression du gène *Nkx3.1* chez le foetus et l'embryon en développement dans une variété de types de tissus tels que le mésoderme, le muscle lisse vasculaire, l'épithélium et des régions du système nerveux central (Kos et al,1998, Mech. Dev., 70, 25-34; Tanaka et al, 1999, Mech. Dev., 85, 179-182; Bhatia-Gaur et al, 1999, Genes Dev., 13, 966-977). Chez l'adulte, la protéine NKX3.1 est localisée de façon prédominante dans la prostate, plus particulièrement dans les cellules épithéliales et son expression est régulée par les androgènes (He et al, 1997, Genomics, 43, 69-77 ; Prescott et al, 1998, Prostate, 35, 71-80 ; Sciavolino et al, 1997, Dev. Dyn., 209, 127-138 ; Ornstein et al, 2001, J Urol, 165(4):1329-34). Elle semble jouer un rôle essentiel dans la fonction de la prostate et régule la prolifération des cellules épithéliales de la prostate ; le gène NKX3.1 a été proposé être un gène supresseur des tumeurs spécifiques de la prostate (Bhatia-Gaur et al, 1999, Genes Dev, 13(8):966-977). La perte de l'expression de NKX3.1 dans les cancers humains de la prostate a été corrélée récemment avec la progression de tumeurs (Bowen et al, 2000, Cancer Res, 60(21):6111-5). Par ailleurs, il a déjà été rapporté que des protéines homeobox sont impliquées dans la régulation de l'angiogénèse (revue: Gorski et Walsh, 2000, Circulation Research, 87:865-872).

Cependant, aucun rôle pour la protéine homeobox NKX3.1 n'a été décrit, à ce jour, dans la régulation de l'angiogénèse.
- GS-N3 : un ARNm de 6771 pb, identifié par la séquence SEQ ID No : 3 dans la liste de séquences en annexe. Une recherche BLAST sur la base de séquences GENBANK permet de l'identifier sous le numéro d'accession U91543.

La séquence de cet ARNm a une séquence codante du nucléotide 151 au nucléotide 6153. Il a donc identifié une protéine GS-P3, résultante de la traduction de cet ARNm. Cette protéine est composée de 2000 aa, identifiée sous le numéro SEQ ID No 8 dans la liste de séquences en annexe, appelée hélicase doigt de Zinc.

La protéine hZFH (human zinc-finger helicase) appartient à la famille des hélicases type Snf2, connues pour agir comme régulateurs transcriptionnels pour des multiples gènes (Aubry et al, 1998, Eur J Biochem, 254(3):558-64). Elle contient également un chromodomaine et est homologue à la protéine CHD3 (« chromodomain helicase DNA binding protein 3 ») identifiée par la séquence SEQ ID No : 29 et SEQ ID No : 30 dans la liste de séquences en annexe. Les protéines CHD3 sont décrites comme pouvant réguler l'expression des gènes en réprimant la transcription via une altération de la structure de la chromatine (Zhang et al,1998, Cell, 95, 279-289 ; Kehle et al, 1998, Science, 282, 1897-1900). A ce jour, aucun rôle dans la régulation de l'expression des gènes impliqués dans l'angiogénèse n'a été décrite ni pour la protéine hZFH, ni pour la protéine CHD3.
- GS-N4 : un ARNm de 3041pb identifié par la séquence SEQ ID No : 4 dans la liste de séquences en annexe. Une recherche BLAST sur la base de séquences GENBANK permet de l'identifier sous le numéro d'accession BC001571.

La séquence de cet ARNm a une séquence codante du nucléotide 67 au nucléotide 2808. Il a donc identifié une protéine GS-P4, résultante de la traduction de cet ARNm. Cette protéine est composée de 913 aa, identifiée sous le numéro SEQ ID No 9 dans la liste de séquences en annexe, appelée facteur d'initiation à la traduction eucaryote, sous unité 8 (110kD) (EIF3S8)

Cette séquence GS-N4 présente une homologie avec la séquence suivante :
- GS-N5 : un ARNm de 1507pb identifié par la séquence SEQ ID No : 5 dans la liste de séquences en annexe. Une recherche BLAST sur la base de séquences GENBANK permet de l'identifier sous le numéro d'accession BC000533.

La séquence de cet ARNm a une séquence codante du nucléotide 407 au nucléotide 1384. Il a donc été identifié une protéine GS-P5, résultante de la traduction de cet ARNm. Cette protéine est composée de 325 aa, identifiée sous le numéro SEQ ID No 10 dans la liste de séquences en annexe, appelée protéine similaire au facteur d'initiation à la traduction eucaryote, sous unité 8 (110kD).

Le facteur 3 d'initiation de la traduction eucaryote (EIF3), le plus gros facteur d'initiation de la synthèse protéique avec une taille de 650kDa est composé d'au moins neuf sous unités peptidiques (Hershey et al,1996, Biochimie, 78, 903-907), dont la sous unité 8 (p110) (Asano et al,. 1997, J. Biol. Chem., 272, 1101-1109). L'EIF3 joue un rôle central dans le processus d'initiation de la biosynthèse protéique notamment dans la liaison de l'initiateur méthionyl- ARNt et l'ARNm à la sous unité ribosomale 40S pour former un complexe d'initiation 40S (Merrick et Hershey, 1996, The pathway and mechanism of eukaryotic protein synthesis. In: Hershey JWB,Mathews MB,Sonenberg N, eds. Translational Control. Cold Spring Harbor, NY: Cold Spring Harbor Press; 1996:31-67). L' EIF3 semble jouer un rôle central dans l'initiation par interaction avec de nombreux autres composants traductionnels (Vornlocher et al, 1999, J Biol Chem, Vol. 274, Issue 24, 16802-16812). Les fonctions de chaque sous unité sont encore mal connues. Un niveau d'expression élevé de certaines sous unités sont détectées dans des tumeurs comme la p150, la p170, la p170 est proposée jouer un autre rôle en plus de ses fonctions dans l'initiation de la traduction (Lin et al , 2001, J Cell Biochem,80(4):483-90 ; Pincheira et al, 2000, Eur J Cell Biol,80(6):410-8). La surexpression de la sous unité 8 (p110) a également été mise en évidence dans une tumeur des cellules germinales par Roche et al (2000, American Journal of Pathology, 157:1597-1604) qui suggèrent un rôle de cette sous unité dans le développement de la tumeur en augmentant la traduction en général conduisant à une croissance et une division cellulaires augmentées.

A ce jour, aucune implication, ni de la sous unité 8 de l'EIF3, ni de sa protéine similaire n'a jamais été décrite dans la régulation de l'angiogénèse.

L'expression des ARNms ci-dessus identifiés est observée dans des cellules endothéliales humaines lorsque la formation de tubes capillaires, synonyme de l'angiogenèse, est inhibée suite à l'action d'un facteur angiostatique.

La Demanderesse a mis en évidence que l'augmentation de l'expression du gène correspondant à chacun de ces ARNms accompagne l'inhibition de la formation de néovaisseaux par les cellules endothéliales.

En effet, les cellules endothéliales humaines formant des néovaisseaux suite à la stimulation par un facteur angiogénique montrent une expression très faible de cet ARNm, tandis que les mêmes cellules endothéliales humaines stimulées par le même facteur angiogénique et mises en présence d'un facteur angiostatique (où l'angiogenèse est inhibée) et/ou encore les mêmes cellules endothéliales stimulées par le facteur angiostatique seul, montrent une expression de ce gène élevée(comme indiqué tableau II).

Ces résultats prouvent l'existence d'une corrélation directe entre l'expression de chacun de ces gènes et l'état angiostatique (i.e. l'inhibition de l'angiogenèse) des cellules endothéliales humaines.

**Tableau II**

| **SÉQ.ID** | **Inducteurs de l'expression** |
|---|---|
| SEQ ID No 1 (GS-N1) | TNF-alpha |
| SEQ ID No 2 (GS-N2) | TNF-alpha |
| SEQ ID No 3 (GS-N3) | TNF-alpha |
| SEQ ID No 4 (GS-N4) | PF4 |
| SEQ ID No 5 (GS-N5) | PF4 |

### 6.2 Vérification du rôle des gènes identifiés dans la régulation de l'angiogénèse.

De plus, il a été également montré le rôle fonctionnel de ces gènes dans la formation de néovaisseaux par les cellules endothéliales humaines.

En effet, une séquence nucléotidique spécifique de chacun des gènes identifiés, choisi parmi les séquences nucléotidiques identifiés par les séquences SEQ ID No. : 11 à SEQ ID. : 14 dans la liste de séquences en annexe a été introduite dans le vecteur d'expression pCI-neo Vector dans l'orientation antisens.

Les vecteurs résultants appelés GS-V1 à GS-V4 identifiés par leurs séquence SEQ ID No : 15 à SEQ ID No : 18 dans la liste de séquences en annexe ont été utilisés pour réprimer l'expression du gène codant pour cet ARNm chez les cellules endothéliales humaines suite à la transfection de ces dernières par ce vecteur.

Les cellules endothéliales humaines ont été stimulées alors par des facteurs angiogéniques. Les résultats obtenus, pour chacune des séquences GS-N1 à GS-N5 en utilisant les séquences antisens et les vecteurs correspondants montrent que :
la répression de l'expression des gènes identifiés sous les numéros de séquences SEQ ID No. 1 à SEQ ID No. 5, inhibe la formation de néovaisseaux par les cellules endothéliales humaines.

Les résultats obtenus, pour chacune des séquences, en utilisant les séquences antisens et les vecteurs correspondants, indiqués dans le tableau III ci-dessous sont illustrés dans la figure 1 (Figures 1A à lE) en annexe.

**TABLEAU III**

| NOM | Gènes SÉQ.ID | **Protéine SEQ. ID** | **Séquences antisens** | **Vecteur avec l'antisens inséré** | **Fig** | **Fig contrôle** |
|---|---|---|---|---|---|---|
| 1 | SEQ ID No 1 (GS-N1) | SEQ ID No 6 (GS-P1) angiopartnerine | SEQ ID No 11 (392pb) | SEQ ID No 15 (GS-V1) | 1A | 1F |
| 2 | SEQ ID No 2 (GS-N2) | SEQ ID No 7 (GS-P2) homeobox NKX3.1 | SEQ ID No 12 (250pb) | SEQ ID No 16 (GS-V2) | 1B | 1F |
| 3 | SEQ ID No 3 (GS-N3) | SEQ ID No 8 (GS-P3) hélicase doigt de Zinc | SEQ ID No 13 (pb) | SEQ ID No 17 (GS-V3) | 1C | 2C |
| 4 | SEQ ID No 4 (GS-N4) | SEQ ID No 9 (GS-P4) facteur d'initiation à la traduction eucaryote, sous unité 8 e | SEQ ID No 14 (167pb) | SEQ ID No 18 (GS-V4) | 1D | 1F |
| 5 | SEQ ID No 5 (GS-N5) | SEQ ID No 10 (GS-P5) facteur d'initiation à la traduction eucaryote, sous unité 8 | SEQ ID No 14 (167pb) | SEQ ID No 18 (GS-V4) | | |

## Revendications

1. Composition pharmaceutique comprenant à titre d'agent actif au moins une substance choisie parmi :
(i) une molécule d'acide nucléique d'un gène d'une cellule endothéliale, de séquence identifiée sous le numéro SEQ ID N°1, SEQ ID N°27 ou SEQ ID N°28 dans la liste de séquences en annexe,
(ii) une molécule polypeptidique codée par ladite molécule d'acide nucléique identifiée sous le numéro SEQ ID N°6, SEQ ID N°31 ou SEQ ID N°32 dans la liste de séquences en annexe,
pour le diagnostic in vivo, pronostic in vivo et/ou le traitement d'une pathologie angiogénique.

2. Composition comprenant à titre d'agent actif au moins une molécule choisie parmi une molécule d'acides nucléiques antisens d'au moins une molécule choisie parmi les SEQ ID N°1, SEQ ID N°27 et SEQ ID N°28 dans la liste de séquences en annexe pour le diagnostic in vivo, pronostic in vivo et/ou le traitement d'une pathologie angiogénique.

3. Composition pharmaceutique pour le diagnostic in vivo, pronostic in vivo et/ou le traitement d'une pathologie angiogénique selon la revendication **2,** ladite pathologie angiogénique comprenant la maladie vasculaire périphérique, la cicatrisation et la réparation tissulaire, l'ostéoporose, la vascularisation de tumeurs, les rétinopathies, l'arthrite rhumatoïde, la maladie de Crohn, l'athérosclérose, l'hyperstimulation de l'ovaire, le psoriasis, l'endométrie associée à la néovascularisation, la resténose due à l'angioplastie du ballon, la superproduction tissulaire due à la cicatrisation, l'hypertension, l'inflammation vasculaire, la maladie et les phénomènes de Raynaud, l'anévrisme, la resténose artérielle, la thrombophlébite, la lymphagyte, le lymphodème, l'ischémie, l'angine, l'infarctus de myocarde, la maladie chronique du coeur, les insuffisances cardiaques telles que l'insuffisance cardiaque congestive, la dégénération maculaire liée à l'âge.

4. Composition pour le diagnostic in vivo, pronostic in vivo et/ou le traitement d'une pathologie angiogénique selon la revendication **2** ou **3 caractérisée en ce qu'**elle comprend la molécule d'acides nucléiques antisens identifiée sous le numéro SEQ ID N°11 dans la liste de séquences en annexe.

5. Molécule d'acides nucléiques antisens identifiée par le numéro SEQ ID N°11 dans la liste de séquences en annexe ou une molécule d'acides nucléiques antisens ayant une identité d'au moins 85%, de préférence d'au moins 95% avec la séquence identifiée sous le numéro SEQ ID N°11 dans la liste de séquences en annexe.

6. Vecteur d'expression de mammifère comprenant au moins une séquence antisens définie à la revendication **5.**

7. Vecteur selon la revendication **6 caractérisé en ce qu'**il est le vecteur GS-V1, identifié par la séquence SEQ ID N°15 dans la liste de séquences en annexe.

8. Méthode de diagnostic et/ou de pronostic d'une pathologie angiogénique chez un mammifère, notamment chez un être humain, **caractérisée en ce que** l'on détecte in vitro dans les cellules dudit mammifère la surexpression ou la sous-expression d'une ou plusieurs séquences nucléotidiques identifiées par les numéros SEQ ID N°1, SEQ ID N°27 et SEQ ID N°28 dans la liste de séquences en annexe ou la surexpression ou la sous-expression d'une ou plusieurs séquences polypeptidiques identifiées par les numéros SEQ ID N°6, SEQ ID N°31 et SEQ ID N°32 dans la liste de séquences en annexe,
ladite pathologie angiogénique comprenant la vascularisation de tumeurs, les rétinopathies, l'arthrite rhumatoïde, la maladie de Crohn, l'athérosclérose, l'hyperstimulation de l'ovaire, le psoriasis, l'endométrie associée à la néovascularisation, la resténose due à l'angioplastie du ballon, la superproduction tissulaire due à la cicatrisation, la maladie vasculaire périphérique, l'hypertension, l'inflammation vasculaire, la maladie et les phénomènes de Raynaud, l'anévrisme, la resténose artérielle, la thrombophlébite, la lymphagyte, le lymphodème, la cicatrisation et la réparation tissulaire, l'ischémie, l'angine, l'infarctus de myocarde, la maladie chronique du coeur, les insuffisances cardiaques telles que l'insuffisance cardiaque congestive, la dégénération maculaire liée à l'âge et l'ostéoporose.

9. Méthode selon la revendication **8 caractérisée en ce qu'**elle comporte les étapes suivantes :
- la détection de l'expression d'une ou plusieurs desdites séquences nucléotidiques SEQ ID N°1, SEQ ID N°27 et SEQ ID N°28 ou d'une ou plusieurs desdites séquences polypeptidiques SEQ ID N°6, SEQ ID N°31 et SEQ ID N°32 par une population cellulaire d'un mammifère,
- la détection de l'expression d'une ou plusieurs de ces mêmes séquences nucléotidiques ou polypeptidiques par une population cellulaire de référence dont l'état angiogénique est connu,
- l'identification des différences éventuelles du niveau d'expression de ces mêmes séquences par les deux populations cellulaires.

10. Méthode de diagnostic et/ou de pronostic selon une quelconque des revendications **8** à **9, caractérisée en ce que** la détection de l'expression des séquences est effectuée après avoir mis les cellules endothéliales en présence d'un fluide biologique issu d'un patient.

11. Méthode de vérification de l'efficacité thérapeutique d'un traitement angiogénique chez un mammifère, notamment chez un être humain, **caractérisée en ce qu'**elle comporte l'identification in vitro dans une population cellulaire dudit mammifère de la surexpression ou de la sous-expression d'au moins un gène impliqué dans un désordre angiogénique identifié par une des séquences nucléotidiques identifiées par les numéros SEQ ID N°1, SEQ ID N°27 et SEQ ID N°28 dans la liste de séquences en annexe.

12. Méthode de vérification de l'efficacité thérapeutique selon la revendication **11 caractérisée en ce qu'**elle comporte les étapes suivantes :
- la détection de l'expression d'une ou plusieurs desdites séquences nucléotidiques SEQ ID N°1, SEQ ID N°27 et SEQ ID N°28 par une population cellulaire isolée d'un mammifère auquel est administrée une composition thérapeutique destinée à traiter un désordre angiogénique comprenant la vascularisation de tumeurs, les rétinopathies, l'arthrite rhumatoïde, la maladie de Crohn, l'athérosclérose, l'hyperstimulation de l'ovaire, le psoriasis, l'endométrie associée à la néovascularisation, la resténose due à l'angioplastie du ballon, la superproduction tissulaire due à la cicatrisation, la maladie vasculaire périphérique, l'hypertension, l'inflammation vasculaire, la maladie et les phénomènes de Raynaud, l'anévrisme, la resténose artérielle, la thrombophlébite, la lymphagyte, le lymphodème, la cicatrisation et la réparation tissulaire, l'ischémie, l'angine, l'infarctus de myocarde, la maladie chronique du coeur, les insuffisances cardiaques telles que l'insuffisance cardiaque congestive, la dégénération maculaire liée à l'âge et l'ostéoporose,
- la détection de l'expression de ces mêmes séquences nucléotidiques par une population cellulaire de référence dont l'état angiogénique est connu,
- l'identification des différences éventuelles du niveau d'expression de ces mêmes séquences par les deux populations cellulaires.

13. Méthode de vérification de l'efficacité thérapeutique selon la revendication 12 **caractérisée en ce que** la détection de l'expression d'une ou plusieurs séquences nucléotidiques est effectuée sur une population cellulaire isolée dudit mammifère.

14. Méthode de vérification selon une quelconque des revendications **11** à **13, caractérisée en ce que** la détection de l'expression des séquences est effectuée après avoir mis les cellules endothéliales en présence d'un fluide biologique issu d'un patient.

15. Méthode de criblage in vitro de composés utiles pour le traitement d'un désordre angiogénique d'un mammifère, notamment d'un être humain, **caractérisée en ce qu'**elle comporte les étapes suivantes :
a) la détection de l'expression d'une ou plusieurs desdites séquences nucléotidiques SEQ ID N°1, SEQ ID N°27 et SEQ ID N°28 ou desdites séquences polypeptidiques SEQ ID N°6, SEQ ID N°31 et SEQ ID N°32 par une population cellulaire isolée d'un mammifère mise en présence d'un composé susceptible d'avoir un effet thérapeutique sur un désordre angiogénique comprenant la vascularisation de tumeurs, les rétinopathies, l'arthrite rhumatoïde, la maladie de Crohn, l'athérosclérose, l'hyperstimulation de l'ovaire, le psoriasis, l'endométrie associée à la néovascularisation, la resténose due à l'angioplastie du ballon, la superproduction tissulaire due à la cicatrisation, la maladie vasculaire périphérique, l'hypertension, l'inflammation vasculaire, la maladie et les phénomènes de Raynaud, l'anévrisme, la resténose artérielle, la thrombophlébite, la lymphagyte, le lymphodème, la cicatrisation et la réparation tissulaire, l'ischémie, l'angine, l'infarctus de myocarde, la maladie chronique du coeur, les insuffisances cardiaques telles que l'insuffisance cardiaque congestive, la dégénération maculaire liée à l'âge et l'ostéoporose,
b) la détection de l'expression de ces mêmes séquences nucléotidiques ou polypeptidiques par une population cellulaire de référence dont l'état angiogénique est connu,
c) l'identification des différences éventuelles du niveau d'expression de ces mêmes séquences nucléotidiques par les deux populations cellulaires.

16. Méthode de criblage selon la revendication **15, caractérisée en ce que** la détection de l'expression des séquences est effectuée après avoir mis les cellules endothéliales en présence d'un fluide biologique issu d'un patient.

## Patentansprüche

1. Pharmazeutische Zusammensetzung, umfassend als Wirkstoff mindestens eine Substanz, die aus Folgendem ausgewählt ist:
i. einem Nukleinsäuremolekül eines Gens einer Endothelialzelle mit einer Sequenz, die unter der Nummer SEQ ID N° 1, SEQ ID N° 27 oder SEQ ID N° 28 in der Liste der Sequenzen im Anhang identifiziert ist,
ii. einem Polypeptidmolekül, das von dem Nukleinsäuremolekül codiert ist, das unter der Nummer SEQ ID N° 6, SEQ ID N° 31 oder SEQ ID N° 32 in der Liste der Sequenzen im Anhang identifiziert ist,
zur in vivo-Diagnose, in vivo-Prognose und/oder zur Behandlung einer angiogene Krankheit.

2. Zusammensetzung, umfassend als Wirkstoff mindestens ein Molekül, das aus Folgendem ausgewählt ist: einem Molekül von Antisense-Nukleinsäuren von mindestens einem Molekül, ausgewählt aus SEQ ID N° 1, SEQ ID N° 27 und SEQ ID N° 28 aus der Liste von Sequenzen im Anhang zur in vivo-Diagnose, in vivo-Prognose und/oder zur Behandlung einer angiogene Krankheit.

3. Pharmazeutische Zusammensetzung zur in vivo-Diagnose, in vivo-Prognose und/oder zur Behandlung einer angiogenen Krankheit nach Anspruch 2, wobei die angiogene Krankheit die periphere Gefäßerkrankung, die Gewebeheilung und Reparatur, die Osteoporose, die Vaskularisierung von Tumoren, die Rhetinopathien, die rheumatoide Arthritis, den Morbus Crohn, die Atherosklerose, die ovarielle Hyperstimulation, die Psoriasis, die Endometritis in Verbindung mit der Neovaskularisation, die Restenose aufgrund der Ballon-Angioplastie, die Gewebeüberproduktion aufgrund der Heilung, die Hypertension, die vaskuläre Entzündung, die Raynaud'sche Krankheit und die Raynaud'schen Phänomene, das Aneurysma, die arterielle Restenose, die Thrombophlebitis, die Lymphangitis, das Lymphödem, die Ischämie, die Angina, den Myocardinfarkt, die chronische Herzkrankheit, die Herzinsuffizienzen wie z.B. die kongestive Herzinsuffizienz, die Makuladegeneration in Verbindung mit dem Alter umfasst.

4. Zusammensetzung zur in vivo-Diagnose, in vivo-Prognose und/oder zur Behandlung einer angiogene Krankheit nach Anspruch **2** oder **3, dadurch gekennzeichnet, dass** sie das Molekül von Antisense-Nukleinsäuren umfasst, das unter der Nummer SEQ ID N° 11 in der Liste der Sequenzen im Anhang identifiziert ist.

5. Molekül von Antisense-Nukleinsäuren, das unter der Nummer SEQ ID N° 11 in der Liste der Sequenzen im Anhang identifiziert ist, oder ein Molekül von Antisense-Nukleinsäuren, das eine Identität von mindestens 85 %, vorzugsweise mindestens 95 %, mit der Sequenz aufweist, die unter der Nummer SEQ ID N° 11 in der Liste der Sequenzen im Anhang identifiziert ist.

6. Säugetier-Expressionsvektor, umfassend mindestens eine Antisense-Sequenz, die in Anspruch **5** definiert ist.

7. Vektor nach Anspruch **6, dadurch gekennzeichnet, dass** es sich um den Vektor GS-V1 handelt, der von der Sequenz SEQ ID N° 15 in der Liste der Sequenzen im Anhang identifiziert ist.

8. Verfahren zur Diagnose und/oder Prognose einer angiogenen Krankheit bei einem Säugetier, insbesondere bei einem Menschen, **dadurch gekennzeichnet, dass** in vitro in den Zellen des Säugetiers die Überexpression oder die Unterexpression von einer oder von mehreren Nukleotidsequenzen nachgewiesen wird, die unter den Nummern SEQ ID N° 1, SEQ ID N° 27 und SEQ ID N° 28 in der Liste der Sequenzen im Anhang identifiziert sind, oder die Überexpression oder die Unterexpression von einer oder von mehreren Polypeptidsequenzen, die unter den Nummern SEQ ID N° 6, SEQ ID N° 31 oder SEQ ID N° 32 in der Liste der Sequenzen im Anhang identifiziert sind,
wobei die angiogene Krankheit die Vaskularisierung von Tumoren, die Rhetinopathien, die rheumatoide Arthritis, den Morbus Crohn, die Atherosklerose, die ovarielle Hyperstimulation, die Psoriasis, die Endometritis in Verbindung mit der Neovaskularisation, die Restenose aufgrund der Ballon-Angioplastie, die Gewebeüberproduktion aufgrund der Heilung, die periphere vaskuläre Erkrankung, die Hypertension, die vaskuläre Entzündung, die Raynaud'sche Krankheit und die Raynaud'schen Phänomene, das Aneurysma, die arterielle Restenose, die Thrombophlebitis, die Lymphangitis, das Lymphödem, die Gewebeheilung und -reparatur, die Ischämie, die Angina, den Myocardinfarkt, die chronische Herzkrankheit, die Herzinsuffizienzen wie z.B. die kongestive Herzinsuffizienz, die Makuladegeneration in Verbindung mit dem Alter und der Osteoporose umfasst.

9. Verfahren nach Anspruch **8, dadurch gekennzeichnet, dass** es die folgenden Schritte aufweist:
- den Nachweis der Expression von einer oder von mehreren der Nukleotidsequenzen SEQ ID N° 1, SEQ ID N° 27 und SEQ ID N° 28 oder von einer oder von mehreren der Polypeptidsequenzen SEQ ID N° 6, SEQ ID N° 31 und SEQ ID N° 32 durch eine Zellpopulation eines Säugetiers,
- den Nachweis der Expression von einer oder von mehreren dieser gleichen Nukleotid- oder Polypeptidsequenzen durch eine Referenz-Zellpopulation, von der der angiogene Zustand bekannt ist,
- die Identifizierung der eventuellen Differenzen des Expressionsniveaus dieser gleichen Sequenzen durch die zwei Zellpopulationen.

10. Verfahren zur Diagnose und/oder Prognose nach einem der Ansprüche **8** bis **9, dadurch gekennzeichnet, dass** der Nachweis der Expression der Sequenzen durchgeführt wird, nachdem die Endothelialzellen mit einem biologischen Fluid, das von einem Patienten stammt, zusammengebracht wurden.

11. Verfahren zur Verifizierung der therapeutischen Wirksamkeit einer angiogenen Behandlung bei einem Säugetier, insbesondere bei einem Menschen, **dadurch gekennzeichnet, dass** es die in vitro-Identifizierung in einer Zellpopulation des Säugetiers der Überexpression oder der Unterexpression mindestens eines Gens umfasst, der an einer angiogenen Störung beteiligt ist, die von einer der Nukleotidsequenzen identifiziert ist, die unter den Nummern SEQ ID N° 1, SEQ ID N° 27 oder SEQ ID N° 28 in der Liste der Sequenzen im Anhang identifiziert sind.

12. Verfahren zur Verifizierung der therapeutischen Wirksamkeit nach Anspruch **11, dadurch gekennzeichnet, dass** es die folgenden Schritte aufweist:
- den Nachweis der Expression von einer oder von mehreren der Nukleotidsequenzen SEQ ID N° 1, SEQ ID N° 27 und SEQ ID N° 28 durch eine Zellpopulation, die von einem Säugetier isoliert wird, dem eine therapeutische Zusammensetzung verabreicht wird, die dazu ausgelegt ist, eine angiogene Störung zu behandeln, umfassend die Vaskularisierung von Tumoren, die Rhetinopathien, die rheumatoide Arthritis, den Morbus Crohn, die Atherosklerose, die ovarielle Hyperstimulation, die Psoriasis, die Endometritis in Verbindung mit der Neovaskularisation, die Restenose aufgrund der Ballon-Angioplastie, die Gewebeüberproduktion aufgrund der Heilung, die periphere vaskuläre Erkrankung, die Hypertension, die vaskuläre Entzündung, die Raynaud'sche Krankheit und die Raynaud'schen Phänomene, das Aneurysma, die arterielle Restenose, die Thrombophlebitis, die Lymphangitis, das Lymphödem, die Gewebeheilung und Reparatur, die Ischämie, die Angina, den Myocardinfarkt, die chronische Herzkrankheit, die Herzinsuffizienzen wie z.B. die kongestive Herzinsuffizienz, die Makuladegeneration in Verbindung mit dem Alter und der Osteoporose,
- den Nachweis dieser gleichen Nukleotidsequenzen durch eine Referenz-Zellpopulation, deren angiogener Zustand bekannt ist,
- die Identifizierung der eventuellen Differenzen des Expressionsniveaus dieser gleichen Sequenzen durch die zwei Zellpopulationen.

13. Verfahren zur Verifizierung der therapeutischen Wirksamkeit nach Anspruch **12, dadurch gekennzeichnet, dass** der Nachweis der Expression von einer oder von mehreren Nukleotidsequenzen auf einer isolierten Zellpopulation des Säugetiers erfolgt.

14. Verfahren zur Verifizierung nach einem der Ansprüche **11** bis **13, dadurch gekennzeichnet, dass** der Nachweis der Expression der Sequenzen durchgeführt wird, nachdem die Endothelialzellen mit einem biologischen Fluid, das von einem Patienten stammt, zusammengebracht wurden.

15. Verfahren zum in vitro-Sieben von Zusammensetzungen, die für die Behandlung einer angiogenen Störung eines Säugtiers, insbesondere eines Menschen, verwendet werden, **dadurch gekennzeichnet, dass** es die folgenden Schritte aufweist:
(a) den Nachweis der Expression von einer oder von mehreren der Nukleotidsequenzen SEQ ID N° 1, SEQ ID N° 27 und SEQ ID N° 28 oder der Polypeptidsequenzen SEQ ID N° 6, SEQ ID N° 31 und SEQ ID N° 32 durch eine isolierte Zellpopulation eines Säugetiers, die mit einer Zusammensetzung zusammengebracht wurde, die einen therapeutischen Effekt auf eine angiogene Störung haben kann, umfassend die Vaskularisierung von Tumoren, die Rhetinopathien, die rheumatoide Arthritis, den Morbus Crohn, die Atherosklerose, die ovarielle Hyperstimulation, die Psoriasis, die Endometritis in Verbindung mit der Neovaskularisation, die Restenose aufgrund der Ballon-Angioplastie, die Gewebeüberproduktion aufgrund der Heilung, die periphere vaskuläre Erkrankung, die Hypertension, die vaskuläre Entzündung, die Raynaud'sche Krankheit und die Raynaud'schen Phänomene, das Aneurysma, die arterielle Restenose, die Thrombophlebitis, die Lymphangitis, das Lymphödem, die Gewebeheilung und Reparatur, die Ischämie, die Angina, der Myocardinfarkt, die chronische Herzkrankheit, die Herzinsuffizienzen wie z.B. die kongestive Herzinsuffizienz, die Makuladegeneration in Verbindung mit dem Alter und der Osteoporose,
(b) den Nachweis der Expression dieser gleichen Nukleotid- oder Polypeptidsequenzen durch eine Referenz-Zellpopulation, deren angiogener Zustand bekannt ist,
(c) die Identifizierung der eventuellen Differenzen des Expressionsniveaus dieser gleichen Nukleotidsequenzen durch die zwei Zellpopulationen.

16. Verfahren zum Sieben nach Anspruch **15, dadurch gekennzeichnet, dass** der Nachweis der Expression der Sequenzen durchgeführt wird, nachdem die Endothelialzellen mit einem biologischen Fluid, das von einem Patienten stammt, zusammengebracht wurden.

## Claims

1. A pharmaceutical composition comprising as an active agent at least one substance selected from:
i. a nucleic acid molecule of a gene of an endothelial cell, of a sequence identified under the number SEQ ID NO: 1, SEQ ID NO: 27 or SEQ ID NO: 28 in the sequences listed in the annex,
ii. a polypeptide molecule encoded by said nucleic acid molecule of a sequence identified under the number SEQ ID NO: 6, SEQ ID NO: 31 or SEQ ID NO: 32 in the sequences listed in the annex,
for the in vivo diagnostic, the in vivo prognostic and/or the treatment of an angiogenic pathology.

2. A composition comprising as an active agent at least one molecule selected from an antisens nucleic acid molecule of at least one molecule selected from SEQ ID NO: 1, SEQ ID NO: 27 and SEQ ID NO: 28 in the sequences listed in the annex for the in vivo diagnostic, in vivo prognostic and/or the treatment of an angiogenic pathology.

3. The pharmaceutical composition for the in vivo diagnostic, the in vivo prognostic and/or the treatment of an angiogenic pathology according to claim **2,** wherein said angiogenic pathology comprises peripheral vascular disease, the healing and repair of tissues, osteoporosis, tumors vascularization, retinopathies, rheumatoid arthritis, Crohn disease, atherosclerosis, ovarian hyperstimulation, psoriasis, endometriosis associated with neovascularization, restenosis due to balloon angioplasty, tissue overproduction due to healing, hypertension, vascular inflammation, Raynaud disease and phenomena, aneurism, arterial restenosis, thrombophlebitis, lymphagitis, lymphedema, ischemia, angina, myocardial infarction, chronic heart disease, heart failure such as congestive heart failure, age-related macular degeneration.

4. A composition for the in vivo diagnostic, the in vivo prognostic and/or the treatment of an angiogenic pathology according to claim **2** or **3, characterized in that** it comprises the antisens nucleic acid molecule identified under the number SEQ ID NO: 11 in the sequences listed in the annex.

5. An antisens nucleic acid molecule identified under the number SEQ ID NO: 11 in the sequences listed in the annex or an antisens nucleic acid molecule having an identity of at least 85%, preferably of at least 95% with the sequence identified under the number SEQ ID NO: 11 in the sequences listed in the annex.

6. A mammal expression vector comprising at least one antisens sequence defined in claim **5.**

7. The vector according to claim **6 characterized in that** it is the vector GS-V1, identified by the sequence SEQ ID NO: 15 in the sequences listed in the annex.

8. A method of diagnostic and/or prognostic of an angiogenic pathology in a mammal, in particular in human, **characterized in that** an overexpression or sub-expression of one or more nucleotidic sequences identified by the numbers SEQ ID NO: 1, SEQ ID NO: 27, and SEQ ID NO: 28 in the sequences listed in the annex or overexpression or sub-expression of one or more polypeptidic sequence identified by the numbers SEQ ID NO: 6, SEQ ID NO: 31 and SEQ ID NO: 32 in the sequences listed in the annex is detected in vitro in the cells of said mammal, wherein said angiogenic pathology comprises tumors vascularization, retinopathies, rheumatoid arthritis, Crohn disease, atherosclerosis, ovarian hyperstimulation, psoriasis, endometriosis associated with neovascularization, restenosis due to balloon angioplasty, tissue overproduction due to healing, peripheral vascular disease, hypertension, vascular inflammation, Raynaud disease and phenomena, aneurism, arterial restenosis, thrombophlebitis, lymphagitis, lymphedema, the healing and repair of tissue, ischemia, angina, myocardial infarction, chronic heart disease, heart failure such as congestive heart failure, age-related macular degeneration and osteoporosis.

9. The method according to claim **8, characterized in that** it comprises the following steps:
- detecting the expression of one or more of said nucleotidic sequences SEQ ID NO: 1, SEQ ID NO: 27, and SEQ ID NO: 28 or one or more of said polypeptidic sequences SEQ ID NO: 6, SEQ ID NO: 31 and SEQ ID NO: 32 by a cellular population of a mammal,
- detecting the expression of one or more of said nucleotidic or polypeptidic sequences by a cellular population of reference wherein the angiogenic state is known,
- identifying potential differences of expression level of said sequences by both cellular populations.

10. The method of diagnostic and/or prognostic according to anyone of claims **8** to **9, characterized in that** the detection of the expression of sequences is performed after contacting the endothelial cells with biological fluid from a patient.

11. A method for verifying the therapeutic efficacy of an angiogenic treatment in a mammal, in particular in human, **characterized in that** it comprises the in vitro identification in a cellular population of said mammal of overexpression or subexpression of at least one gene involved in an angiogenic disorder identified by a nucleotidic sequence identified by the numbers SEQ ID NO: 1, SEQ ID NO: 27, and SEQ ID NO: 28 in the sequences listed in the annex.

12. The method for verifying the therapeutic efficacy according to claim **11 characterized in that** it comprises the following steps:
- detecting the expression of one or more of said nucleotidic sequences SEQ ID NO: 1, SEQ ID NO: 27, and SEQ ID NO: 28 by a cellular population isolated from a mammal wherein is administered a therapeutic composition addressed to treat an angiogenic disorder comprising tumors vascularization, retinopathies, rheumatoid arthritis, Crohn disease, atherosclerosis, ovarian hyperstimulation, psoriasis, endometriosis associated with neovascularization, restenosis due to balloon angioplasty, tissue overproduction due to healing, peripheral vascular disease, hypertension, vascular inflammation, Raynaud disease and phenomena, aneurism, arterial restenosis, thrombophlebitis, lymphagitis, lymphedema, the healing and repair of tissue, ischemia, angina, myocardial infarction, chronic heart disease, heart failure such as congestive heart failure, age-related macular degeneration and osteoporosis,
- detecting the expression of said nucleotidic sequences by a cellular population of reference wherein the angiogenic state is known,
- identifying the potential differences of the expression level of said sequences by both cellular populations.

13. The method for verifying the therapeutic efficacy according to claim **12, characterized in that** the detection of the expression of one or more nucleotidic sequences is performed on a cellular population isolated from said mammal.

14. The method for verifying according to anyone of claims **11** to **13, characterized in that** the detection of the expression of said sequences is performed after contacting the endothelial cells with a biological fluid from a patient.

15. A method for in vitro screening of compounds useful for the treatment of an angiogenic disorder of a mammal, in particular human, **characterized in that** it comprises the following steps:
a) detecting the expression of one or more of said nucleotidic sequences SEQ ID NO: 1, SEQ ID NO: 27, and SEQ ID NO: 28 or said polypeptidic sequences SEQ ID NO: 6, SEQ ID NO: 31 and SEQ ID NO: 32 by a cellular population isolated from a mammal contacted with a compound liable to have a therapeutic effect on an angiogenic disorder comprising tumors vascularization, retinopathies, rheumatoid arthritis, Crohn disease, atherosclerosis, ovarian hyperstimulation, psoriasis, endometriosis associated with neovascularization, restenosis due to balloon angioplasty, tissue overproduction due to healing, peripheral vascular disease, hypertension, vascular inflammation, Raynaud disease and phenomena, aneurism, arterial restenosis, thrombophlebitis, lymphagitis, lymphedema, the healing and repair of tissue, ischemia, angina, myocardial infarction, chronic heart disease, heart failure such as congestive heart failure, age-related macular degeneration and osteoporosis,
b) detecting the expression of said nucleotidic or polypeptidic sequences by a cellular population of reference wherein the angiogenic state is known,
c) identifying the potential differences of the expression level of said sequences by both cellular populations.

16. The method for screening according to claim **15, characterized in that** the detection of the expression of sequences is performed after contacting the endothelial cells with a biological fluid from a patient.
